(19) Europäisches Patentamt / European Patent Office / Office européen des brevets

(11) EP 4 461 295 A2

(12) # EUROPEAN PATENT APPLICATION

(43) Date of publication:
**13.11.2024 Bulletin 2024/46**

(21) Application number: **24183848.1**

(22) Date of filing: **08.02.2018**

(51) International Patent Classification (IPC):
***A61K 31/40*** *(2006.01)*

(52) Cooperative Patent Classification (CPC):
(C-Sets available)
**A61P 3/06; A61K 31/194; A61K 31/397; A61K 31/40; A61P 9/10** (Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.02.2017 US 201762456571 P**

(62) Document number(s) of the earlier application(s) in accordance with Art. 76 EPC:
**18751030.0 / 3 579 827**

(71) Applicant: **Esperion Therapeutics, Inc.**
**Ann Arbor, MI 48108 (US)**

(72) Inventors:
- **LALWANI, Narendra**
  **Ann Arbor, 48108 (US)**
- **MACDOUGALL, Diane**
  **Ann Arbor, 48108 (US)**

(74) Representative: **Mewburn Ellis LLP**
**Aurora Building**
**Counterslip**
**Bristol BS1 6BX (GB)**

Remarks:
•This application was filed on 21-06-2024 as a divisional application to the application mentioned under INID code 62.
•Claims filed after the date of filing of the application (Rule 68(4) EPC).

(54) **TRIPLET COMBINATION FORMULATIONS AND METHODS OF TREATING OR REDUCING THE RISK OF CARDIOVASCULAR DISEASE**

(57) Disclosed herein are compositions comprising ETC-1002; Ezetimibe; a statin; and one or more pharmaceutical excipients or carriers. Also disclosed herein are methods for using said composition. Uses include methods of treating cardiovascular disease or reducing the risk of cardiovascular disease in a subject. Uses also include methods of treating hypercholesterolemia and/or dyslipidemias in a subject.

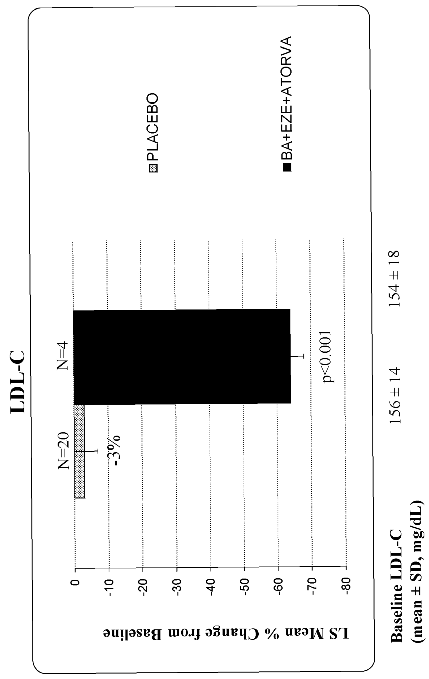


FIG. 1

EP 4 461 295 A2

(52) Cooperative Patent Classification (CPC): (Cont.)

C-Sets
**A61K 31/194, A61K 2300/00;**
**A61K 31/397, A61K 2300/00;**
**A61K 31/40, A61K 2300/00**

## Description

## BACKGROUND

*Field of the invention*

This application relates to methods and compositions of useful for treating or reducing the risk of cardiovascular disease. This application discloses methods and compositions comprising ETC-1002, a statin, and Ezetimibe for lowering low-density lipoprotein cholesterol (LDL-C) and concomitantly treating or reducing the risk of cardiovascular disease.

*Description of the Related Art*

Statins are the cornerstone of prevention and treatment of cardiovascular disease, but can produce statin-associated muscle symptoms in 5% to 29% of patients. Muscle symptoms including pain, stiffness, cramping, or weakness, usually without serum creatine kinase (CK) elevations, is the primary manifestation of statin intolerance.

Despite aggressive interventional and pharmacologic therapies, CVD is the number 1 cause of death globally (WHO 2015). The Global Burden of Disease study estimated that 29.6% of all deaths worldwide (approximately 15.6 million deaths) were caused by CVD in 2010, more than all communicable, maternal, neonatal and nutritional disorders combined, and double the number of deaths caused by cancers (Nichols 2014). In the United States (US), based on 2011 death rate data, more than 2150 Americans die from CVD daily, an average of 1 death every 40 seconds (Mozffarian 2015). Of great concern, approximately 155,000 Americans dying from CVD are less than 65 years of age (Mozffarian 2015). In Europe, CVD remains the most common cause of deaths, resulting in almost 2 times as many deaths as cancer (Townsend 2015).

Elevated LDL-C is a major modifiable risk factor for the development of atherosclerosis and CVD (Sharrett 2001). Evidence supporting LDL-C as a therapeutic target and surrogate for cardiovascular (CV) outcomes comes from interventional studies with LDL-C-lowering therapies, epidemiological studies, and genetic variants (both gain of function and loss of function). Large randomized clinical studies aimed at lowering LDL-C show a consistent, log-linear relationship between LDL-C reduction and CV risk reduction, independent of the mechanism for LDL-C lowering (Kathiresan 2008; Baigent 2010; Robinson 2005; Stamler 1986, Silverman 2016). A published patient-level meta-analysis including 26 statin trials and more than 160,000 participants, showed a consistent relationship between LDL-C reduction and CV outcomes (Baigent 2010). This analysis showed that a 1 mmol/L (~39 mg/dL) reduction in LDL-C was associated with a 22% reduction in the 5-year incidence of major coronary events, revascularizations, and ischemic strokes. More recently, a meta-analysis in 312,175 patients from 49 trials assessed the relationship between LDL-C reduction and CV outcomes when LDL-C reduction was due to statin therapy versus established non-statin interventions that work primarily by upregulation of the LDL receptor such as diet, bile acid sequestrants, ileal bypass, and Ezetimibe (Silverman 2016). This meta-analysis reported that the relationship between LDL-C reduction and CV outcomes was similar regardless of whether LDL-C reduction was derived from statin therapy or non-statin interventions that upregulate the LDL receptor (Silverman 2016). Thus, LDL-C lowering via upregulation the LDL receptor is largely accepted as a valid surrogate endpoint of CV risk reduction by clinicians and regulatory authorities (Stone 2013).

Statins are central to the LDL-C-lowering strategy and are supported by a large body of data demonstrating robust effectiveness in lowering LDL-C and reducing the risk of CVD (Waters 2006, Grundy 2004). However, there is increasing awareness of the limitations and risks of statin use. Many individuals at risk for CVD fail to achieve LDL-C goals (Martin 2013, Virani 2011). In 2011, the Food and Drug Administration (FDA) mandated safety-labeling changes limiting the use of high dose (80 mg) simvastatin due to safety concerns of muscle injury or myopathy (Egan 2011). Although myopathy events are rare, a more widespread problem is various muscle side effects such as pain and weakness, particularly at high doses, leading to poor tolerability and lack of persistence on statin therapy (Cohen 2012).

Hypercholesterolemic patients have several therapeutic options to lower LDL-C. Statin and non-statin therapies that lower LDL-C via upregulation of LDL receptors are associated with reduced cardiovascular disease (CVD) risk.

Accordingly, there is a need to develop effective therapies to treat CVD and CV risk while minimizing the number of adverse side effects.

## SUMMARY

Generally, this application relates to methods and compositions comprising Bempedoic acid (ETC-1002), Ezetimibe and statin for the treating or reducing the risk of cardiovascular disease. Herein disclosed is a triplet combination comprising: ETC-1002; Ezetimibe; a statin; and one or more pharmaceutical excipients or carriers. Also disclosed herein are kits comprising one or more compositions which themselves comprise one or more components of the triplet combination. The triplet combination is useful for treating or reducing the risk of cardiovascular disease. Disclosed are

methods of treating or reducing the risk of cardiovascular disease, atherosclerotic cardiovascular disease, hyperlipidemia, dyslipidemia or mixed dyslipidemia using the triplet combination, optionally adding a PCSK9 inhibitor antibody therapy or an approved lipid-modifying therapy to the combination, and optionally in patients having hypercholesterolemia or suffering from muscle-related adverse effects associated with statin use. Also disclosed are methods of treating or reducing the risk of cardiovascular disease that affect the: level of low-density lipoprotein cholesterol (LDL-C), level of very low density lipoprotein (VLDL), number of VLDL particles, size of VLDL particles, level of apolipoprotein A-1 (ApoA1), level of apolipoprotein B (ApoB), ratio of apolipoprotein B (ApoB) to apolipoprotein A-1 (ApoA1), level of triglycerides (TG), level of total cholesterol (TC), level of high-sensitivity C-reactive protein (hs-CRP), level of non-HDL-C, or any combination thereof in a subject in comparison to that in a matched control subject receiving the same dose of the statin and the Ezetimibe but lacking ETC-1002.

Bempedoic acid, statin, and Ezetimibe work by divergent mechanisms to lower LDL-C via up-regulation of LDL receptors. The present invention provides methods, kits and compositions useful for treating patients with elevated LDL-C to achieve lowered LDL-C using triplet therapy.

The inventors have found that triplet combination therapy comprising Bempedoic acid (ETC-1002), Ezetimibe and statin has synergistic effects and provides for outcomes when treating or reducing the risk of cardiovascular disease that are additive and in some aspects are more than sum of the predicted benefits from any one of or the dual combination of Bempedoic acid (ETC-1002), Ezetimibe and statin. Moreover, the inventors find that the triplet combination provides less frequent and less severe side effects and/or adverse events occurring for patients suffering from cardiovascular disease, or a risk thereof, or otherwise in need of therapy for cardiovascular disease.

As is described herein, these triplet compositions provide significant reductions in total cholesterol, and specifically LDL-C, in patients in need of therapy for cardiovascular disease.

Accordingly, the present application discloses kits, compositions and methods using ETC-1002, Ezetimibe, and a statin, based on results from clinical studies, disclosed herein, that demonstrate the triplet combination is superior when compared the efficacy and safety of ETC-1002 monotherapy, Ezetimibe monotherapy, therapy with ETC-1002 combined with Ezetimibe (EZE), and each one which with or without statin therapy. This outcome is especially relevant among hypercholesterolemic patients with or without a history of statin-related muscle symptoms.

## BRIEF DESCRIPTION OF THE SEVERAL VIEWS OF THE DRAWINGS

These and other features, aspects, and advantages of the present invention will become better understood with regard to the following description, and accompanying drawings.

**Figure 1** is a bar graph showing the percent change in LDL-C from baseline after 6 weeks of dosing of Bempedoic acid 180 mg, Ezetimibe 10 mg, and Atorvastatin 20 mg.
**Figure 2** is a graph displaying the percentage in LDL-C change over time (LDL-C time course).
**Figure 3** is a bar graph showing the percent change in hsCRP from baseline after 6 weeks of dosing of Bempedoic acid 180 mg, Ezetimibe 10 mg, and Atorvastatin 20 mg.
**Figure 4** is a bar graph showing the percent change of patients with LDL-C reduction ≥50% after 6 weeks of dosing of Bempedoic acid 180 mg, Ezetimibe 10 mg, and Atorvastatin 20 mg.
**Figure 5** is a bar graph showing the percent change of LDL-C from baseline to Week 6 in patients receiving Bempedoic acid, Ezetimibe and Atorvastatin.
**Figure 6** is a bar graph showing the percent change of patients achieving LDL-C <70 mg/dL after 6 weeks of dosing of Bempedoic acid 180 mg, Ezetimibe 10 mg, and Atorvastatin 20 mg.
**Figure 7** is a bar graph displaying the percent change of atherogenic lipids from baseline after 6 weeks of dosing of Bempedoic acid 180 mg, Ezetimibe 10 mg, and Atorvastatin 20 mg.
**Figure 8** is a bar graph showing the percent change of LDL-C from baseline to Week 6.

## DETAILED DESCRIPTION

*Advantages and utility*

Bempedoic acid (ETC-1002) is an oral, first-in class, small molecule designed to lower low-density lipoprotein cholesterol (LDL-C). ETC-1002 is an agent that has been shown to lower low-density lipoprotein cholesterol (LDL-C) by direct inhibition of hepatic adenosine triphosphate citrate lyase, leading to reduced de novo cholesterol synthesis and increased LDL receptor expression.

The general class of "statins" are compounds which lower cholesterol levels in the body by inhibiting the enzyme 3-hydroxy-3-methylglutaryl-coenzyme A (HMG-CoA) reductase and concomitantly, the pathway for synthesizing cholesterol in the liver. Examples of compounds which are part of the "stain" class include, but are not limited to, atorvastatin,

simvastatin, rosuvastatin, and pravastatin. Treatments usually administer from about 1 mg to 80 mg of a statin compound.

As described above, Bempedoic acid (ETC-1002) is a small molecule that inhibits adenosine triphosphate-citrate lyase (ACL), an enzyme upstream of 3-hydroxy-3-methylglutaryl coenzyme A (HMG-CoA) reductase in the cholesterol biosynthesis pathway. Bempedoic acid, like both statins and Ezetimibe, up-regulates LDL-C receptors. However, unlike statins, Bempedoic acid does not inhibit cholesterol synthesis in muscle tissue, therefore it is anticipated that negative muscle-related adverse effects associated with statin use may be avoided by use of Bempedoic acid.

Briefly, and as described in more detail below, described herein are compositions, methods of making the said compositions and methods for treating cardiovascular disease or reducing the risk of cardiovascular disease using a triplet combination of Ezetimibe, a statin and ETC-1002. The advantages for this approach are numerous and include, but are not limited to, increased reduction of total cholesterol and low density lipoprotein cholesterol levels in patients. Those patients treated with the triplet combination experience outcomes that are at least as good as, if not more than simply the additive beneficial effects of these three components. In some aspects, the triplet combination demonstrates a synergistic effect. In other words, the triplet combination is more effective than predicted as much of the results significantly exceed those when patients are treated with Ezetimibe monotherapy, ETC-1002 monotherapy, and the combination of Ezetimibe and ETC-1002, each one with or without statin therapy.

As described above, statins are the cornerstone of prevention and treatment of cardiovascular disease, but can produce statin-associated muscle symptoms in 5% to 29% of patients. Statin-associated muscle symptoms are an important clinical problem because statin discontinuation in hypercholesterolemic patients increases cardiovascular risk. Patients who discontinue statin treatment because of intolerance show a trend toward decreased 8-year survival compared with patients who continue statin therapy. Hence, there is a significant need for cardiovascular therapies for patients that exhibit muscle-related statin intolerance.

*Definitions*

Terms used in the claims and specification are defined as set forth below unless otherwise specified.

As used herein and in the appended claims, singular articles such as "a," "an" and "the" and similar referents in the context of describing the elements (especially in the context of the following claims) are to be construed to cover both the singular and the plural, unless otherwise indicated herein or clearly contradicted by context. Recitation of ranges of values herein are merely intended to serve as a shorthand method of referring individually to each separate value falling within the range, including the upper and lower bounds of the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (*e.g.*, "such as") provided herein, is intended merely to better illuminate the embodiments and does not pose a limitation on the scope of the claims unless otherwise stated. No language in the specification should be construed as indicating any non-claimed element as essential.

As used herein, the term "cardiovascular diseases" refers to diseases of the heart and circulatory system. These diseases are often associated with dyslipoproteinemias and/or dyslipidemias. Cardiovascular diseases which the compositions of the present disclosure are useful for preventing or treating include but are not limited to arteriosclerosis; atherosclerosis; stroke; ischemia; endothelium dysfunctions, in particular those dysfunctions affecting blood vessel elasticity; peripheral vascular disease; coronary heart disease; myocardial infarction; cerebral infarction and restenosis.

As used herein, the term "dyslipidemias" refers to disorders that lead to or are manifested by aberrant levels of circulating lipids. To the extent that levels of lipids in the blood are too high, the compositions of the disclosure are administered to a patient to restore normal levels. Normal levels of lipids are reported in medical treatises known to those of skill in the art. For example, recommended blood levels of LDL, HDL, free triglycerides and others parameters relating to lipid metabolism can be found at the web site of the American Heart Association and that of the National Cholesterol Education Program of the National Heart, Lung and Blood Institute (http://www.americanheart.org/cholesterol- /about_level.html and http://www.nhlbi.nih.gov/health/public/heart/chol/hbc_what.html, respectively). At the present time, the recommended level of HDL cholesterol in the blood is above 35 mg/dL; the recommended level of LDL cholesterol in the blood is below 130 mg/dL; the recommended LDL:HDL cholesterol ratio in the blood is below 5: 1, ideally 3.5:1; and the recommended level of free triglycerides in the blood is less than 200 mg/dL. Dyslipidemias include, but are not limited to, hyperlipidemia and low blood levels of high density lipoprotein (HDL) cholesterol. In certain embodiments, the hyperlipidemia for prevention or treatment by the compounds of the present disclosure is familial hypercholesterolemia; familial combined hyperlipidemia; reduced or deficient lipoprotein lipase levels or activity, including reductions or deficiencies resulting from lipoprotein lipase mutations; hypertriglyceridemia; hypercholesterolemia; high blood levels of urea bodies (*e.g.* beta.-OH butyric acid); high blood levels of Lp(a) cholesterol; high blood levels of low density lipoprotein (LDL) cholesterol; high blood levels of very low density lipoprotein (VLDL) cholesterol and high blood levels of non-esterified fatty acids.

Generally, reference to a certain element such as hydrogen or H is meant to include all isotopes of that element.

For example, if an R group is defined to include hydrogen or H, it also includes deuterium and tritium. Compounds comprising radioisotopes such as tritium, $^{14}C$, $^{32}P$ and $^{35}S$ are thus within the scope of the present technology. Procedures for inserting such labels into the compounds of the present technology will be readily apparent to those skilled in the art based on the disclosure herein.

The term "ameliorating" refers to any therapeutically beneficial result in the treatment of a disease state, *e.g.,* an inflammatory disease state, including lessening in the severity or progression, remission, or cure thereof. In some embodiments, "ameliorating" includes prophylaxis of a disease state.

As used herein, the term "IMP" "IMP" means an investigational medicinal product. Such products include a pharmaceutical form of an active substance or placebo being tested or used as a reference in a clinical trial, including products already with a marketing authorization but used or assembled (formulated or packaged) in a way different from the authorized form, or when used for an unauthorized indication, or when used to gain further information about the authorized form.

The term "in vitro" refers to processes that occur in a living cell growing separate from a living organism, *e.g.*, growing in tissue culture.

The term "in vivo" refers to processes that occur in a living organism.

The term "mammal" as used herein includes both humans and non-humans and include but is not limited to humans, non-human primates, canines, felines, murines, bovines, equines, and porcines.

The term "sufficient amount" means an amount sufficient to produce a desired effect, *e.g.,* an amount sufficient to modulate protein aggregation in a cell.

The term "therapeutically effective amount" is an amount that is effective to ameliorate a symptom of a disease. A therapeutically effective amount can, in some embodiments, be a "prophylactically effective amount" as prophylaxis can be considered therapy.

The compounds of the present technology can exist as solvates, especially hydrates. Hydrates may form during manufacture of the compounds or compositions comprising the compounds, or hydrates may form over time due to the hygroscopic nature of the compounds. Compounds of the present technology can exist as organic solvates as well, including DMF, ether, and alcohol solvates among others. The identification and preparation of any particular solvate is within the skill of the ordinary artisan of synthetic organic or medicinal chemistry.

"Subject" refers to a mammalian organism treated using a compound of the present disclosure. The "subject" can be a human or non-human mammalian organism.

"Tautomer" refer to alternate forms of a compound that differ in the position of a proton, such as enol-keto and imine-enamine tautomers, or the tautomeric forms of heteroaryl groups containing a ring atom attached to both a ring NH moiety and a ring =N moiety such as pyrazoles, imidazoles, benzimidazoles, triazoles, and tetrazoles.

"Treating" or "treatment" of a disease or disorder in a subject refers to 1) preventing the disease or disorder from occurring in a subject that is predisposed or does not yet display symptoms of the disease or disorder; 2) inhibiting the disease or disorder or arresting its development; or 3) ameliorating or alleviating the cause of the regression of the disease or disorder.

As used herein, the terms "prevent," "preventing," "prevention," "prophylactic treatment" and the like refer to reducing the probability of developing a disease, disorder, or condition in a subject, who does not have, but is at risk of or susceptible to developing a disease, disorder, or condition. Thus, in some embodiments, an agent can be administered prophylactically to prevent the onset of a disease, disorder, or condition, or to prevent the recurrence of a disease, disorder, or condition.

For the purposes of this specification and appended claims, unless otherwise indicated, all numbers expressing amounts, sizes, dimensions, proportions, shapes, formulations, parameters, percentages, parameters, quantities, characteristics, and other numerical values used in the specification and claims, are to be understood as being modified in all instances by the term "about" even though the term "about" may not expressly appear with the value, amount or range. Accordingly, unless indicated to the contrary, the numerical parameters set forth in the following specification and attached claims are not and need not be exact, but may be approximate and/or larger or smaller as desired, reflecting tolerances, conversion factors, rounding off, measurement error and the like, and other factors known to those of skill in the art depending on the desired properties sought to be obtained by the presently disclosed subject matter. For example, the term "about," when referring to a value can be meant to encompass variations of, in some aspects, $\pm$ 100% in some aspects $\pm$ 50%, in some aspects $\pm$ 20%, in some aspects $\pm$ 10%, in some aspects $\pm$ 5%, in some aspects $\pm$1%, in some aspects $\pm$ 0.5%, and in some aspects $\pm$ 0.1% from the specified amount, as such variations are appropriate to perform the disclosed methods or employ the disclosed compositions.

Unless defined otherwise, all technical and scientific terms used herein have the meaning commonly understood by a person skilled in the art to which this disclosure belongs.

Herein any and all heteroaryl and heterocycloalkyl substituents may contain up to four heteroatoms selected from the group consisting of: O, N, and S.

*Abbreviations:*

AE is an abbreviation for adverse event
CK in an abbreviation for creatine kinase
EZE = is an abbreviation for ezetimibe
HDL-C is an abbreviation for high-density lipoprotein cholesterol
CRP = is an abbreviation for high-sensitivity C-reactive protein
LDL-C is an abbreviation for low-density lipoprotein cholesterol
LS is an abbreviation for least-squares
NCEP ATP-III is an abbreviation for National Cholesterol Education Program Adult Treatment Panel III
non-HDL-C is an abbreviation for non-high-density lipoprotein cholesterol
VLDL is an abbreviation for very-low-density lipoprotein
NCEP ATP-III is an abbreviation for National Cholesterol Education Program Adult Treatment Panel III

*Methods of treatment*

Disclosed herein is a method of treating or reducing the risk of cardiovascular disease in a subject in need thereof, the method comprising administering: ETC-1002; Ezetimibe; a statin; and one or more pharmaceutical excipients or carriers to the subject, wherein the administration treats or reduces the risk of cardiovascular disease in the subject.

Disclosed herein is a method of treating or reducing the risk of atherosclerotic cardiovascular disease in a subject in need thereof, the method comprising administering: ETC-1002; Ezetimibe; a statin; and one or more pharmaceutical excipients or carriers to the subject, wherein the administration treats or reduces the risk of atherosclerotic cardiovascular disease in the subject.

Disclosed herein is a method of treating dyslipidemia in a subject in need thereof, the method comprising administering: ETC-1002; Ezetimibe; a statin; and one or more pharmaceutical excipients or carriers to the subject, wherein the administration treats or alleviates dyslipidemia in the subject.

Disclosed herein is a method of treating hypercholesterolemia in a subject in need thereof, the method comprising administering: ETC-1002; Ezetimibe; a statin; and one or more pharmaceutical excipients or carriers to the subject, wherein the administration treats or alleviates hypercholesterolemia in the subject.

Disclosed herein is a method of lowering cholesterol the method comprising administering: ETC-1002; Ezetimibe; a statin; and one or more pharmaceutical excipients or carriers to the subject, wherein the administration lowers cholesterol in the subject.

Disclosed herein is a method of treating or reducing the risk of cardiovascular disease in a subject in need thereof, the method comprising administering: ETC-1002; Ezetimibe; a statin; a PCSK9 inhibitor antibody therapy; and one or more pharmaceutical excipients or carriers to the subject, wherein the administration treats or reduces the risk of cardiovascular disease in the subject.

Disclosed herein is a method of treating or reducing the risk of cardiovascular disease in a subject in need thereof, the method comprising administering: ETC-1002; Ezetimibe; a statin; another approved lipid-modifying therapy other than a statin and/or Ezetimibe; and one or more pharmaceutical excipients or carriers to the subject, wherein the administration treats or reduces the risk of cardiovascular disease in the subject.

In some aspects, the method treats or reduces the risk of cardiovascular disease in the subject. In some aspects, the method treats or reduces the risk of atherosclerotic cardiovascular disease in the subject.

In one aspect, the triplet combination shows a significantly greater efficacy and safety profile even in acute hypercholesterolemic patients/ subjects or in patients/ subjects suffering dyslipidemia.

In one aspect, methods and compositions of present disclosure even lower cholesterol in patients with persistently elevated LDL-C, despite the subject being on a stable statin therapy at a high dosage of statin, *i.e.* a statin dosage from 40-80 mg.

The formulations of the present disclosure are also useful in methods of treating the following conditions: dyslipoproteinemias and/or dyslipidemias, arteriosclerosis, atherosclerosis, stroke, ischemia, and/or endothelium dysfunction in a subject.

The present disclosure also provides methods for the treatment or prevention of a dyslipidemia comprising administering ETC-1002; a statin; Ezetimibe; and one or more pharmaceutical excipients to a subject in need thereof.

Dyslipidemias which the compositions of the present disclosure are useful for preventing or treating include, but are not limited to, hyperlipidemia and low blood levels of high density lipoprotein (HDL) cholesterol. In certain embodiments, the hyperlipidemia for prevention or treatment by the compounds of the present disclosure is familial hypercholesterolemia; familial combined hyperlipidemia; reduced or deficient lipoprotein lipase levels or activity, including reductions or deficiencies resulting from lipoprotein lipase mutations; hypertriglyceridemia; hypercholesterolemia; high blood levels of urea bodies (*e.g.* beta.-OH butyric acid); high blood levels of Lp(a) cholesterol; high blood levels of low

density lipoprotein (LDL) cholesterol; high blood levels of very low density lipoprotein (VLDL) cholesterol and high blood levels of non-esterified fatty acids.

The present disclosure further provides methods of altering lipid metabolism in a subject, *e.g.*, reducing LDL in the blood of a patient, reducing free triglycerides in the blood of a patient, increasing the ratio of HDL to LDL in the blood of a patient, and inhibiting saponified and/or non-saponified fatty acid synthesis, said methods comprising administering to the patient the compounds or the of the present disclosure.

*Clinical Endpoints*

In some aspects, the administration reduces the level C-reactive protein (CRP) up to 20% or 30% or more relative to baseline.

In some aspects, the administration dose dependently reduces apolipoprotein B by 15% to 17% or more, non-high-density lipoprotein cholesterol by 14% to 17% or more, total cholesterol by 13% to 15% or more, and LDL particle number by 17% to 21% or more.

In some aspects, the administration decreases LDL-C in the subject up to 24% or more relative to baseline. In some aspects, the administration decreases non HDL-C in the subject by at least 30, 35, 37, 40, 42, or 45% or more relative to baseline. In some aspects, the administration decreases hsCRP in the subject by at least 20, 25, 26, 30, 35, 38, or 40% or more relative to baseline.

In some aspects, the administration decreases non-HDL-C in the subject by at least 30, 35, 40, 43, 45, 48, or 50% or more relative to baseline. In other aspects, the administration decreases HDL-C in the subject relative to baseline.

In an embodiment, the administration reduces the: level of low-density lipoprotein cholesterol (LDL-C), level of very low density lipoprotein (VLDL), number of VLDL particles, size of VLDL particles, level of apolipoprotein A-1 (ApoA1), level of apolipoprotein B (ApoB), ratio of apolipoprotein B (ApoB) to apolipoprotein A-1 (ApoA1), level of triglycerides (TG), level of total cholesterol (TC), level of high-sensitivity C-reactive protein (hs-CRP), level of non-HDL-C, or any combination thereof in the subject.

In some aspects, LDL-C is decreased in the subject by at least 30, 35, 40, 43, 45, 48, or 50% relative to baseline. In some aspects, the method decreases LDL-C level in the subject by 40% relative to baseline. In some aspects, the method decreases LDL-C level in the subject by 45% relative to baseline. In some aspects, the method decreases LDL-C level in the subject by 50% relative to baseline. In some aspects, non HDL-C is decreased in the subject by at least 30, 35, 37, 40, 42, or 45% relative to baseline. In some aspects, hsCRP is decreased in the subject by at least 20, 25, 26, 30, 35, 38, or 40% relative to baseline.

In some aspects, the method decreases LDL-C level in the subject by 61% relative to baseline. In some aspects, the method decreases LDL-C level in the subject by 64% relative to baseline LDL-C level in the subject. In some aspects, the method decreases LDL-C level in the subject by 50% relative to baseline six (6) weeks after administration. In some aspects, the method decreases LDL-C level in the subject by 64% relative to baseline LDL-C level in the subject six (6) weeks after administration. In some aspects, the method decreases hsCRP level in the subject by 40% relative to baseline. In some aspects, the method decreases hsCRP level in the subject by 45% relative to baseline. In some aspects, the method decreases hsCRP level in the subject by 48% relative to baseline. In some aspects, the method decreases non-HDL-C level in the subject by 40% relative to baseline. In some aspects, the method decreases non-HDL-C level in the subject by 50% relative to baseline. In some aspects, the method decreases non-HDL-C level in the subject by 60% relative to baseline non-HDL-C level in the subject. In some aspects, the method decreases apoB level in the subject by 40% relative to baseline. In some aspects, the method decreases apoB level in the subject by 50% relative to baseline. In some aspects, the method decreases apoB level in the subject by 54% relative to baseline ApoB level in the subject. In some aspects, the method decreases total cholesterol level in the subject by 40% relative to baseline. In some aspects, the method decreases total cholesterol level in the subject by 47% relative to baseline TC level in the subject. In some aspects, the method decreases triglyceride level in the subject by 25% relative to baseline. In some aspects, the method decreases total cholesterol level in the subject by 27% relative to baseline. In some aspects, the method decreases triglyceride (TG) level in the subject by 27% relative to baseline TG level in the subject.

In an embodiment, the administration does not significantly change or alternatively does reduce the: level of low-density lipoprotein cholesterol (LDL-C), level of very low density lipoprotein (VLDL), number of VLDL particles, size of VLDL particles, level of apolipoprotein A-1 (ApoA1), level of apolipoprotein B (ApoB), ratio of apolipoprotein B (ApoB) to apolipoprotein A-1 (ApoA1), level of triglycerides (TG), level of total cholesterol (TC), level of high-sensitivity C-reactive protein (hs-CRP), level of non-HDL-C, or any combination thereof in the subject.

In an embodiment, an effective amount of each one of ETC-1002; Ezetimibe; and a statin is administered wherein the combination produces a change in the: level of low-density lipoprotein cholesterol (LDL-C), level of very low density lipoprotein (VLDL), number of VLDL particles, size of VLDL particles, level of apolipoprotein A-1 (ApoA1), level of apolipoprotein B (ApoB), ratio of apolipoprotein B (ApoB) to apolipoprotein A-1 (ApoA1), level of triglycerides (TG), level of total cholesterol (TC), level of high-sensitivity C-reactive protein (hs-CRP), level of non-HDL-C, or any combination

thereof in the subject.

In an embodiment, an effective amount of each one of ETC-1002; Ezetimibe; and a statin is administered wherein the combination produces a change in the: level of low-density lipoprotein cholesterol (LDL-C), level of very low density lipoprotein (VLDL), number of VLDL particles, size of VLDL particles, level of apolipoprotein A-1 (ApoA1), level of apolipoprotein B (ApoB), ratio of apolipoprotein B (ApoB) to apolipoprotein A-1 (ApoA1), level of triglycerides (TG), level of total cholesterol (TC), level of high-sensitivity C-reactive protein (hs-CRP), level of non-HDL-C, or any combination thereof in the subject when compared to that of a matched control subject receiving the same dose of the statin and/or receiving the same dose of the Ezetimibe, but lacking ETC-1002 .

In an embodiment, an effective amount of each one of ETC-1002, Ezetimibe, and a statin is administered wherein the combination produces a change in the: level of low-density lipoprotein cholesterol (LDL-C), level of very low density lipoprotein (VLDL), number of VLDL particles, size of VLDL particles, level of apolipoprotein A-1 (ApoA1), level of apolipoprotein B (ApoB), ratio of apolipoprotein B (ApoB) to apolipoprotein A-1 (ApoA1), level of triglycerides (TG), level of total cholesterol (TC), level of high-sensitivity C-reactive protein (hs-CRP), level of non-HDL-C, or any combination thereof in the subject when compared to that of a matched control subject receiving placebo.

In an embodiment, an effective amount of each one of ETC-1002, Ezetimibe, and a statin is administered wherein the combination produces a change in the: level of low-density lipoprotein cholesterol (LDL-C), level of very low density lipoprotein (VLDL), number of VLDL particles, size of VLDL particles, level of apolipoprotein A-1 (ApoA1), level of apolipoprotein B (ApoB), ratio of apolipoprotein B (ApoB) to apolipoprotein A-1 (ApoA1), level of triglycerides (TG), level of total cholesterol (TC), level of high-sensitivity C-reactive protein (hs-CRP), level of non-HDL-C, or any combination thereof in the subject when compared to that of a matched control subject receiving the same dose of the Ezetimibe and the same dose of the ETC-1002.

In some aspects, the methods disclosed herein do not change the level of: level of low-density lipoprotein cholesterol (LDL-C), level of very low density lipoprotein (VLDL), number of VLDL particles, size of VLDL particles, level of apolipoprotein A-1 (ApoA1), level of apolipoprotein B (ApoB), ratio of apolipoprotein B (ApoB) to apolipoprotein A-1 (ApoA1), level of triglycerides (TG), level of total cholesterol (TC), level of high-sensitivity C-reactive protein (hs-CRP), level of non-HDL-C, and/or any combination thereof in the subject.

In an embodiment, the method provides for any combination of an increase, decrease, or unchanged value in: level of low-density lipoprotein cholesterol (LDL-C), level of very low density lipoprotein (VLDL), number of VLDL particles, size of VLDL particles, level of apolipoprotein A-1 (ApoA1), level of apolipoprotein B (ApoB), ratio of apolipoprotein B (ApoB) to apolipoprotein A-1 (ApoA1), level of triglycerides (TG), level of total cholesterol (TC), level of high-sensitivity C-reactive protein (hs-CRP), and level of non-HDL-C.

In another aspect, the method decreases the number of drug-related AEs by at least 25%, by 35%, 45% or by 50% or more. In another aspect, the method decreases the number of muscle-related AEs by at least 50%, by 65%, 75% or by 85% or more. In some aspects, the method decreases the number of drug-related AEs relative to therapy with ETC-1002, Ezetimibe, a statin, or any combination of two thereof. In some aspects, the method decreases the number of drug-related AEs relative to therapy with ETC-1002, Ezetimibe, a statin, or any combination of two thereof.

In another aspect, methods disclosed herein significantly reduce the risk of a cardiovascular event in a subject. In some aspects this risk is reduced by up to 35% or more.

In some aspects, the methods herein provide for treating cardiovascular disease and/or reducing the risk of cardiovascular disease in a subject comprising administering an amount of a compositions comprising: ETC-1002, Ezetimibe, and a statin which is/are rapidly absorbed having a $T_{max}$ at less than 4 hours.

In some aspects, the methods disclosed herein provide for treating cardiovascular disease and/or reducing the risk of cardiovascular disease in a subject comprising administering an amount of a compositions comprising: ETC-1002, Ezetimibe, and a statin which do not prolong QTc or QT/QTc (TQT study). In one aspect, the triplet combination does not affect subject heart rate and PR and QRS intervals. In one aspect, the triplet combination positively affects the subject’s heart rate and PR and QRS intervals in a clinically meaningful way.

In some aspects, the methods herein provide for treating cardiovascular disease and/or reducing the risk of cardiovascular disease in a subject comprising administering an effective amount of compositions comprising: ETC-1002, Ezetimibe, and a statin which systemic exposure, $AUC_{tau,ss}$, occurs with t½ approximately 15 to 27 hours.

In some aspects, the methods herein provide for treating cardiovascular disease and/or reducing the risk of cardiovascular disease in a subject comprising administering an amount of compositions comprising: ETC-1002, Ezetimibe, and a statin which provides exposure measures AUC and/or $C_{max}$ indicating that the 2 regimens have no appreciable drug interaction. In an embodiment, neither the statin nor ETC-1002 nor Ezetimibe exposure measures are outside safe values as established by confidence intervals.

In one aspect, the composition includes a statin as defined by the dosages of atorvastatin (10 mg or 20 mg), simvastatin (5 mg, 10 mg, or 20 mg), rosuvastatin (5 mg or 10 mg), and/or pravastatin (10 mg, 20 mg, or 40 mg). In another aspect, the method includes a statins as defined by the dosages of atorvastatin (10 mg or 20 mg), simvastatin (5 mg, 10 mg, or 20 mg), rosuvastatin (5 mg or 10 mg), and/or pravastatin (10 mg, 20 mg, or 40 mg).

In one aspect, the composition includes a statin as defined by the fixed dosages of Table 1 below:

Table 1.

| High Intensity Statins | Moderate Intensity Statins | Low Intensity Statins |
|---|---|---|
| • Atorvastatin 40-80 mg | • Atorvastatin 10-20 mg | • Simvastatin 10 mg |
| • Rosuvastatin 20-40 mg | • Rosuvastatin 5-10 mg | • Pravastatin 10-20 mg |
| • Simvastatin 80 mg‡ | • Simvastatin 20-40 mg | • Lovastatin 20 mg |
| | • Pravastatin 40-80 mg | • Fluvastatin 20-40 mg |
| | • Lovastatin 40 mg | • Pitavastatin 1 mg |
| | • Fluvastatin XL 80 mg | |
| | • Fluvastatin 40 mg twice daily | |
| | • Pitavastatin 2-4 mg | |

*Components*

In an embodiment, ETC-1002 is administered at a dose between 0.001 mg to 2000 mg.

In an embodiment, ETC-1002 is administered at a dose of 120 mg. In an embodiment, ETC-1002 is administered at a dose of 180 mg. In an embodiment, ETC-1002 is administered at a dose between 40 mg to 90 mg. ETC-1002 is administered at a dose of 40 mg. ETC-1002 is administered at a dose of 60 mg. ETC-1002 is administered at a dose of 90 mg.

In an embodiment, Ezetimibe is administered at a dose between 0.001 mg to 100 mg.

In an embodiment, Ezetimibe is administered at a dose of 10 mg.

In some aspects, ETC-1002 is administered at a dose of 120 mg or at a dose of 180 mg and Ezetimibe is administered at a dose of 10 mg.

In some aspects, ETC-1002 is administered at a dose of 120 mg or at a dose of 180 mg, the statin is administered at a dose of between 1 mg to 80 mg, and Ezetimibe is administered at a dose of 10 mg.

In some aspects, the subject has hypercholesterolemia. In some aspects, the method further comprises treating hypercholesterolemia.

In some aspects, the subject has a baseline LDL-C level of 130-220 mg/dL. In some aspects, the subject has a baseline triglycerides level of less than or equal to 400 mg/dL.

In some aspects, Ezetimibe; statin; and ETC-1002 are administered simultaneously. In some aspects, any two or more of: Ezetimibe; statin; and ETC-1002 are administered sequentially. In some aspects, all three of: Ezetimibe; statin; and ETC-1002 are administered sequentially.

In some aspects, the statin, Ezetimibe and ETC-1002 are each administered at least once daily. In some aspects, the statin, Ezetimibe and ETC-1002 are each administered at least once daily for at least 1, 2, 3, 4, 5, 6, 7, 8, 9, 10, 11, or 12 week(s). In some aspects, the Ezetimibe is administered at least once every other day. In some aspects, the Ezetimibe is administered at least once every other day for at least 1, 2, 3, 4, 5, 6, 7, 8, 9, 10, 11, or 12 week(s). In some aspects, the ETC-1002 is administered at least once every other day. In some aspects, the ETC-1002 is administered at least once every other day for at least 1, 2, 3, 4, 5, 6, 7, 8, 9, 10, 11, or 12 week(s). In some aspects, the statin is administered at least once every other day. In some aspects, the statin is administered at least once every other day for at least 1, 2, 3, 4, 5, 6, 7, 8, 9, 10, 11, or 12 week(s).

In some aspects, the statin, Ezetimibe and ETC-1002 are each administered simultaneously in one composition. In some aspects, the statin, Ezetimibe and ETC-1002 are separately administered simultaneously in two compositions. In some aspects, the statin, Ezetimibe and ETC-1002 are separately administered simultaneously in three compositions.

In some aspects, the statin, Ezetimibe and ETC-1002 are administered each separately and sequentially over a period of twelve (12) hours. In some aspects, two of the: statin, Ezetimibe and ETC-1002 are administered in one compositions followed sequentially over a period of twelve (12) hours by one other composition containing the last member of the statin, Ezetimibe and ETC-1002 combination.

*Patients*

In some aspects, the subject has dyslipidemia. In some aspects, the subject has hypercholesterolemia. In some aspects, the subject is obese, optionally wherein the BMI of the subject is 18-45 $kg/m^2$.

In one aspect, the subject is statin tolerant.

In some aspects, the subject is statin intolerant. In some aspects, the subject is unable to tolerate at least two statins including one statin at the lowest FDA approved dose due to muscle-related symptoms such as pain, aches, weakness, or cramping that began or increased during statin therapy and resolved when statin therapy was discontinued.

In some aspects, the subject suffers from muscle-related adverse effects associated with statin use.

In some aspects, the subject has mixed dyslipidemia.

In one aspect, the subject has hypercholesterolemia.

In some aspects, the subject atherosclerotic cardiovascular disease.

In some aspects, the subject is a mammal. In some aspects, the subject is human.

In some aspects, the subject is obese, optionally wherein the BMI of the subject is 18-45 kg/$m^2$.

In some aspects, the subject experiences an adverse event when using statin therapy at the lowest FDA approved dose, said adverse events being selected form the group consisting of muscle-related pain, aches, weakness, and cramping. The inventors have observed that such muscle-related adverse events that began or increased during statin therapy could be significantly lowered or even resolved when treatment with the triplet combination was employed.

In some aspects, the subject has a baseline LDL-C level of 115-220 mg/dL. In some aspects, the subject has a baseline triglycerides level of less than or equal to 400 mg/dL.

*Compounds*

Compositions or formulations of Ezetimibe, a statin and ETC-1002 are described herein.

Statins and ETC-1002 are described herein. In one aspect, the statin is a natural product isolated from a natural source such as *Penecillium* and *Aspergillus* fungi is provided for. In another aspect, the statins are synthetic, meaning they are made by advancing petrochemical starting material via organic synthesis to the desired statin compound.

Formula I below shows the structure of ETC-1002 derivatives that can be used in place of ETC-1002 according to the present disclosure:

Formula I

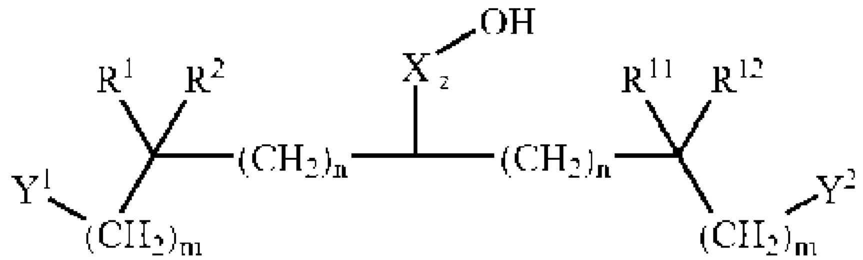

wherein each occurrence of m is independently an integer ranging from 0 to 5; (b) each occurrence of n is independently an integer ranging from 3 to 7; (c) X is ($CH_2$), or Ph, wherein z is an integer from 0 to 4 and Ph is a 1,2-, 1,3-, or 1,4 substituted phenyl group; (d) each occurrence of $R^1$, $R^2$, $R^{11}$, and $R^{12}$ is independently H, ($C_1$-$C_6$)alkyl, ($C_2$-$C_6$)alkenyl, ($C_2$-$C_6$)alkynyl, phenyl, or benzyl, wherein $R^1$, $R^2$, $R^{11}$, and $R^{12}$ are not each simultaneously H; and (e) each occurrence of $Y^1$ and $Y^2$ is independently ($C_1$-$C_6$)alkyl, OH, COOH, $COOR^3$, $SO_3H$,

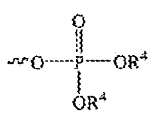 , 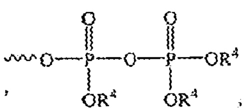 , 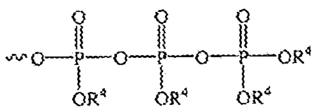 ,

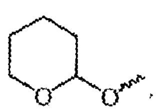 , 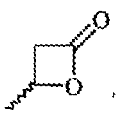 , 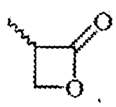 .

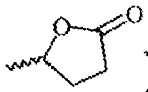, 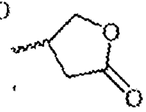, 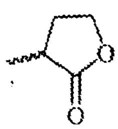, 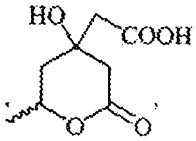,

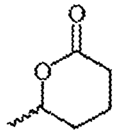, 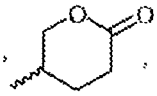, 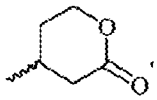, or 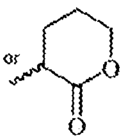;

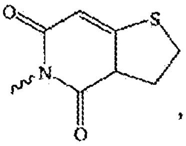, 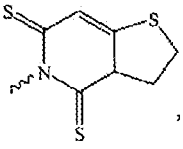, 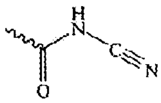, 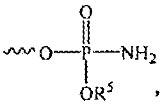,

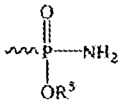, 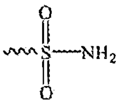, 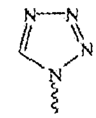, 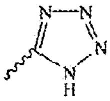,

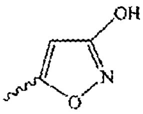, 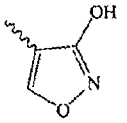, 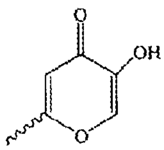, 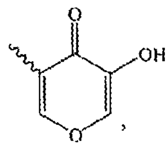,

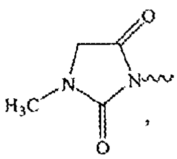, 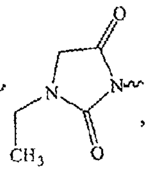, 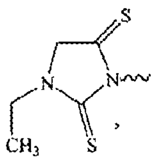, 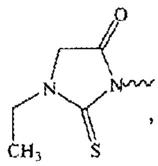, or 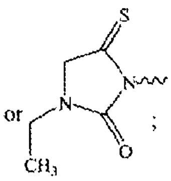;

wherein: (i) $Y^1$ and $Y^2$ are not each simultaneously ($C_1$-$C_6$)alkyl; (ii) $R^3$ is ($C_1$-$C_6$)alkyl, ($C_2$-$C_6$)alkenyl, ($C_2$-$C_6$)alkynyl, phenyl, or benzyl and is unsubstituted or substituted with one or more halo, OH, ($C_1$-$C_6$)alkoxy, or phenyl groups, (iii) each occurrence of $R^4$ is independently H, ($C_1$-$C_6$)alkyl, ($C_2$-$C_6$)alkenyl, or ($C_2$-$C_6$)alkynyl and is unsubstituted or substituted with one or two halo, OH, $C_1$-$C_6$, alkoxy, or phenyl groups; and (iv) each occurrence of $R^5$ is independently H, ($C_1$-$C_6$)alkyl, ($C_2$-$C_6$)alkenyl, or ($C_2$-$C_6$)alkynyl.

Below is the chemical structure of ETC-1002 (Bempedoic acid):

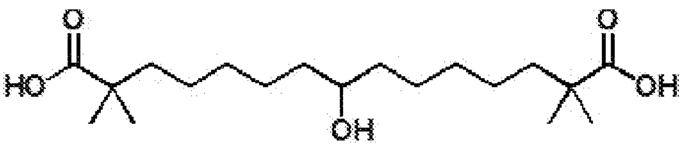.

ETC-1002 can be referred to as Bempedoic acid or 8-hydroxy-2,2,14,14 tetramethylpentadecanedioic acid.

Formula (II) below shows the structure of Ezetimibe derivatives that can be used in place of Ezetimibe according to the present disclosure:

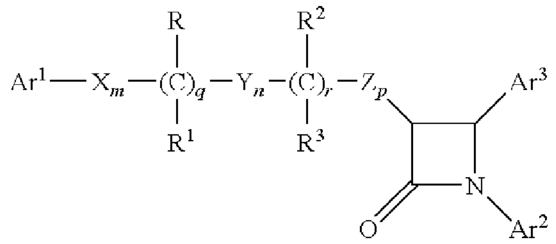

wherein in Formula (II) above or a salt thereof, wherein: $Ar^1$ and $Ar^2$ are independently selected from the group consisting of aryl and $R^4$-substituted aryl; $Ar^3$ is aryl or $R^5$-substituted aryl; X, Y and Z are independently selected from the group consisting of $-CH_2-$, -CH(lower alkyl)- and -C(dilower alkyl)-; R and $R^2$ are independently selected from the group consisting of $-OR^6$, $-O(CO)R^6$, $-O(CO)OR^9$ and $-O(CO)NR^6 R^7$ ; $R^1$ and $R^3$ are independently selected from the group consisting of hydrogen, lower alkyl and aryl; q is 0 or 1; r is 0 or 1; m, n and p are independently selected from 0, 1, 2, 3 or 4; provided that at least one of q and r is 1, and the sum of m, n, p, q and r is 1, 2, 3, 4, 5 or 6; and provided that when p is 0 and r is 1, the sum of m, q and n is 1, 2, 3, 4 or 5; $R^4$ is 1-5 substituents independently selected from the group consisting of lower alkyl, $-OR^6$, $- O(CO)R^6$, $-O(CO)OR^9$, $-O(CH_2)_{1-5} OR^6$, $-O(CO)NR^6 R^7$, $-NR^6R^7$, $-NR^6 (CO)R^7$, $- -NR^6 (CO)OR^9$, $-NR^6 (CO)NR^7 R^8$, $-NR^6 SO_2 R^9$, $-COOR^6$, $- CONR^6 R^7$, $-COR^6$, $-SO_2 -NR^6 R^7$, $S(O)_{0-2} R^9$, $-O(CH_2)_{1-10}-COOR^6$, $- O(CH_2)_{1-10} CONR^6 R^7$, -(lower alkylene)$COOR^6$, $-CH=CH-COOR^6$, $-CF_3$, - CN, $-NO_2$ and halogen; $R^5$ is 1-5 substituents independently selected from the group consisting of $-OR^6$, $-O(CO)R^6$, $-O(CO)OR^9$, $-O(CH_2)_{1-5} OR^6$, $- O(CO)NR^6 R^7$, $-NR^6 R^7$, $-NR^6 (CO)R^7$, $-NR^6 (CO)OR^9$, $-NR^6 (CO)NR^7 R^8$, $-NR^6 SO_2 R^9$, $-COOR^6$, $-CONR^6 R^7$, $-COR^6$, $-SO_2 -NR^6 R^7$, $S(O)_{0-2} R^9$, $- O(CH_2)_{1-10}-C-COOR^6$, $-O(CH_2)_{1-10} CONR^6 R^7$, -(lower alkylene)$COOR^6$ and $-CH=CH-COOR^6$ ; $R^6$, $R^7$ and $R^8$ are independently selected from the group consisting of hydrogen, lower alkyl, aryl and aryl-substituted lower alkyl; and $R^9$ is lower alkyl, aryl or aryl-substituted lower alkyl.

Statin compounds inhibit HMGR enzymatic activity in the liver. In terms of structure, all statin compounds possess a dihydroxyheptanoic acid group or lactone thereof and a substituted ring system (shown below).

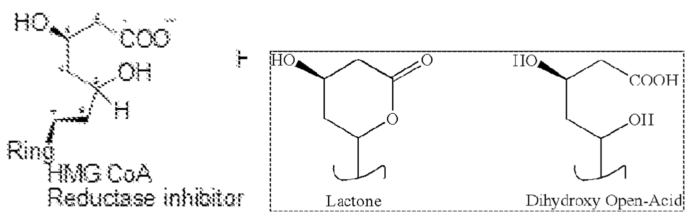
HMG CoA Reductase inhibitor
Lactone
Dihydroxy Open-Acid

However, statins do differ with respect to the substituted ring structure. Some statins have a substituted decalin-ring structure while others have substituted aryl and heteroaryl ring systems. The structure of exemplary statin compounds is shown below, however, this list is in no way limiting.

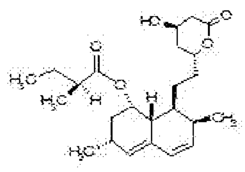
LOVASTATIN

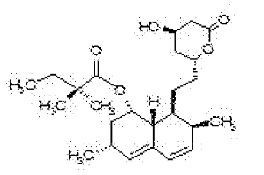
SIMVASTATIN

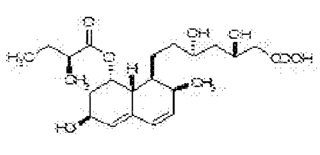
PRAVASTATIN

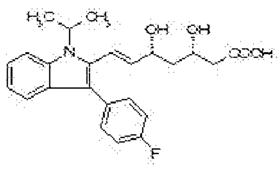
FLUVASTATIN

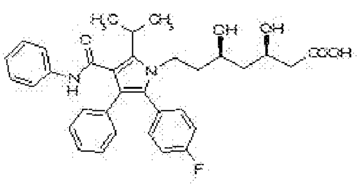
ATORVASTATIN

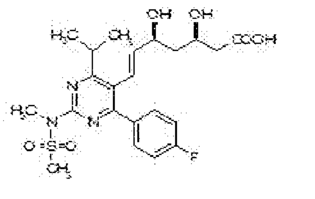
ROSUVASTATIN

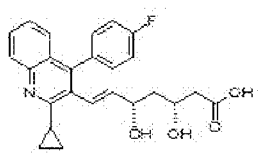
PITAVASTATIN

It is acknowledged that any and all analogs or derivatives of Ezetimibe and any and all analogs or derivatives ETC-1002 according to Formula I can be used in any of the methods and/or compositions or formulations disclosed herein. It is further acknowledged that any and all analogs of statins according to the description above can be used in any of the methods and/or compositions or formulations disclosed herein.

Ezetimibe is referred to as 1-(4-fluorophenyl)-3(R)-[3(S)-(4-fluorophenyl)-3-hydroxypropyl)]-4(S)-[4-(phenylmethoxy)phenyl]-2-azetidinone; or (3*R*,4*S*)-1-(4-fluorophenyl)-3-[(3*S*)-3-(4-fluorophenyl)-3-hydroxypropyl]-4-(4-hydroxyphenyl)azetidin-2-one.

The structure of Ezetimibe is:

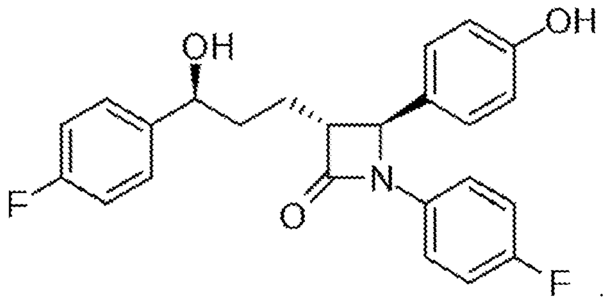

*Synthesis of ETC-1002, Ezetimibe, and statins*

ETC-1002 and the process of synthesizing ETC-1002 is disclosed in the issued U.S. Patent No. 7,335,799. The details of this process can be found in the published U.S. patent publication No. US2005-0043278 A1, in paragraphs [0247] - [0343] of the specification, each of which is herein incorporated by reference.

Ezetimibe and the process of synthesizing Ezetimibe is disclosed in the issued U.S. Patent No. 5,631,365. The details of this process can be found in the specification, beginning on page 4 right column, line 43 through page 11 right column, line 65, each of which is herein incorporated by reference.

The synthesis of statins is known in the art. In a strategic and general disclosure, the synthesis of statins is disclosed in WO2005047276A2 which is herein incorporated by reference. Any other synthetic modifications for statins (or analogs of ETC-1002 for that matter), which may include unique or alternative ring systems, are within the purview of the skilled artisan. For example, the skilled artisan will be able to use synthetic reference texts to incorporate unique or desired substituted-aryl, heteroaryl, and decalin ring systems into the final statin compound. Such references include, but are not limited to: as Fieser and Fieser's Reagents for Organic Synthesis, Volumes 1-15 (John Wiley, and Sons, 1991), Rodd's Chemistry of Carbon Compounds, Volumes 1-5, and Supplementals (Elsevier Science Publishers, 1989), Organic Reactions, Volumes 1-40 (John Wiley, and Sons, 1991), March's Advanced Organic Chemistry, (John Wiley, and Sons, 5th Edition, 2001), and Larock's Comprehensive Organic Transformations (VCH Publishers Inc., 1989), T. W. Greene and P.G.M. Wuts, Protecting Groups in Organic Synthesis, Third Edition, Wiley, New York, 1999.

*Pharmaceutical compositions*

Also disclosed herein is a pharmaceutical composition or pharmaceutical formulation comprising ETC-1002; Ezetimibe; a statin; and one or more pharmaceutical excipients or carriers.

The triplet combination of Ezetimibe, a statin, and ETC-1002 can be formulated in two or more separate pharmaceutical compositions or formulated in a single pharmaceutical composition.

In some aspects, the composition(s) includes one or more pharmaceutically acceptable carriers or vehicles.

In some aspects, the composition is formulated for oral delivery. In some aspects, the composition is formulated for administration once daily.

Each of these compositions can comprise one or more pharmaceutically acceptable: excipient, carrier, buffer, stabiliser or other materials well known to those skilled in the art. Such materials should be non-toxic and should not interfere with the efficacy of the active ingredient. The precise nature of the carrier or other material can depend on the route of administration, *e.g.* oral, intravenous, cutaneous or subcutaneous, nasal, intramuscular, intraperitoneal routes.

Pharmaceutical compositions for oral administration can be in tablet, capsule, powder or liquid form. A tablet can include a solid carrier such as gelatin or an adjuvant. Liquid pharmaceutical compositions generally include a liquid carrier such as water, petroleum, animal or vegetable oils, mineral oil or synthetic oil. Physiological saline solution, dextrose or other saccharide solution or glycols such as ethylene glycol, propylene glycol or polyethylene glycol can be included.

For intravenous, cutaneous or subcutaneous injection, or injection at the site of affliction, the active ingredient

will be in the form of a parenterally acceptable aqueous solution which is pyrogen-free and has suitable pH, isotonicity and stability. Those of relevant skill in the art are well able to prepare suitable solutions using, for example, isotonic vehicles such as Sodium Chloride Injection, Ringer's Injection, Lactated Ringer's Injection. Preservatives, stabilisers, buffers, antioxidants and/or other additives can be included, as required.

Whether it is a small molecule or other pharmaceutically useful compound according to the present disclosure that is to be given to an individual, administration is preferably in a "therapeutically effective amount" or "prophylactically effective amount"(as the case can be, although prophylaxis can be considered therapy), this being sufficient to show benefit to the individual. The actual amount administered, and rate and time-course of administration, will depend on the nature and severity of protein aggregation disease being treated. Prescription of treatment, e.g. decisions on dosage etc, is within the responsibility of general practitioners and other medical doctors, and typically takes account of the disorder to be treated, the condition of the individual patient, the site of delivery, the method of administration and other factors known to practitioners. Examples of the techniques and protocols mentioned above can be found in Remington's Pharmaceutical Sciences, 16th edition, Osol, A. (ed.), 1980.

*Kits*

Also provided for is a kit comprising one or more compositions disclosed herein.

In some aspects, the kit comprises two or more compositions, each of which comprises one or more of: ETC-1002; Ezetimibe; a statin; and one or more pharmaceutical excipients or carriers.

In some aspects, the kit comprises one composition comprising ETC-1002; Ezetimibe; a statin; and one or more pharmaceutical excipients or carriers.

**EXAMPLES**

Below are examples of specific embodiments for carrying out the present disclosure. The examples are offered for illustrative purposes only, and are not intended to limit the scope of the present disclosure in any way. Efforts have been made to ensure accuracy with respect to numbers used (*e.g.*, amounts, temperatures, etc.), but some experimental error and deviation should, of course, be allowed for.

The practice of the present disclosure will employ, unless otherwise indicated, conventional methods of protein chemistry, biochemistry, recombinant DNA techniques and pharmacology, within the skill of the art. Such techniques are explained fully in the literature. See, *e.g.,* T.E. Creighton, Proteins: Structures and Molecular Properties (W.H. Freeman and Company, 1993); A.L. Lehninger, Biochemistry (Worth Publishers, Inc., current addition); Sambrook, et al., Molecular Cloning: A Laboratory Manual (2nd Edition, 1989); Methods In Enzymology (S. Colowick and N. Kaplan eds., Academic Press, Inc.); Remington's Pharmaceutical Sciences, 18th Edition (Easton, Pennsylvania: Mack Publishing Company, 1990); Carey and Sundberg Advanced Organic Chemistry 3rd Ed. (Plenum Press) Vols A and B(1992).

Any terms not directly defined herein shall be understood to have the meanings commonly associated with them as understood within the art of the disclosure. Certain terms are discussed herein to provide additional guidance to the practitioner in describing the compositions, devices, methods and the like of aspects of the disclosure, and how to make or use them. It will be appreciated that the same thing may be said in more than one way. Consequently, alternative language and synonyms may be used for any one or more of the terms discussed herein. No significance is to be placed upon whether or not a term is elaborated or discussed herein. Some synonyms or substitutable methods, materials and the like are provided. Recital of one or a few synonyms or equivalents does not exclude use of other synonyms or equivalents, unless it is explicitly stated. Use of examples, including examples of terms, is for illustrative purposes only and does not limit the scope and meaning of the aspects of the disclosure herein.

It must be noted that, as used in the specification and the appended claims, the singular forms "a," "an" and "the" include plural referents unless the context clearly dictates otherwise.

**EXAMPLE 1: CLINICAL PROTOCOL**

**Brief description of clinical study and results.**

A RANDOMIZED, DOUBLE-BLIND, PLACEBO-CONTROLLED, PARALLEL GROUP STUDY TO EVALUATE THE EFFICACY AND SAFETY OF TRIPLET THERAPY WITH BEMPEDOIC ACID (ETC-1002) 180 MG, EZETIMIBE 10 MG, AND ATORVASTATIN 20 MG IN PATIENTS WITH ELEVATED LDL-C

**Synopsis**

| |
|---|
| **Name of Sponsor:** Esperion Therapeutics, Inc. |
| **Name of Investigational Products:** Bempedoic acid (ETC-1002) film-coated tablets<br>Ezetimibe overencapsulated tablets<br>Atorvastatin overencapsulated tablets |
| **Name of Active Ingredients:** Bempedoic acid (ETC-1002) Ezetimibe, Atorvastatin |
| **Title of Study:**<br>A Randomized, Double-Blind, Placebo-Controlled, Parallel Group Study to Evaluate the Efficacy and Safety of Triplet Therapy with Bempedoic Acid (ETC-1002) 180 mg, Ezetimibe 10 mg, and Atorvastatin 20 mg in Patients with Elevated LDL-C |
| **Study Number:** 1002-038 |
| **Phase of Development:** 2 |
| **Clinical Sites:** Approximately 20 sites located in United States (US) |
| **Primary Objective:**<br>• To assess the low-density lipoprotein cholesterol (LDL-C) lowering effect of triplet therapy with Bempedoic acid (ETC-1002) 180 mg, ezetimibe 10 mg, and atorvastatin 20 mg versus placebo administered daily for 6 weeks in patients with elevated LDL-C<br>**Secondary Objectives:**<br>• To assess the effect of triplet therapy versus placebo on non-high-density lipoprotein cholesterol (non-HDL-C), total cholesterol (TC), apolipoprotein B (ApoB), high-sensitivity C-reactive protein (hs-CRP), triglycerides (TG), and high-density lipoprotein cholesterol (HDL-C)<br>• To assess the effect of triplet therapy versus placebo on percent of patients achieving LDL-C level <70 mg/dL<br>• To assess the effect of triplet therapy versus placebo on percent of patients achieving LDL-C reduction ≥50%<br>• To assess the safety and tolerability of triplet therapy versus placebo |
| |
| **Endpoints:** |
| The following endpoints will be used to evaluate the objectives of the study.<br>Primary endpoint:<br>• Percent change from baseline to Week 6 in LDL-C |
| Secondary endpoints:<br>Percent change from baseline to Week 6 in non-HDL-C, TC, ApoB, hs-CRP, TG, and HDL-C<br>Percent of patients with LDL-C <70 mg/dL at Week 6<br>Percent of patients with LDL-C reduction ≥50% from baseline to Week 6<br>Safety Endpoints:<br>Subject incidence of adverse events (AEs)<br>Clinical safety laboratory (including hematology, blood chemistry, and urinalysis) results<br>Vital signs and physical examination (PE) findings |
| **Study Design:** |

(continued)

| |
|---|
| This is a Phase 2, multicenter, randomized, double-blind, placebo-controlled, parallel group study. Patients have initially undergone screening at Week -6 (Visit S1). Eligible patients have begun washout of all LDL-C lowering drugs and nutritional supplements at least 5 weeks prior to randomization. Patients have returned at Week -1 (Visit S2) for lipid and/or other assessments. Patients who are deemed not eligible for randomization at any point during screening were notified by clinical site personnel and considered screen failures. At Week 0 (Visit T1), approximately 60 patients were randomized in a ratio of 2:1 to receive either triplet therapy (bempedoic acid 180 mg + ezetimibe 10 mg + atorvastatin 20 mg) or placebo once daily for 6 weeks. Randomized patients were returned for clinic visits at Week 3 (Visit T2), and Week 6 (Visit T3). |
| **Number of Patients (Planned):** Approximately 60 adult male and female patients. |
| **Duration of Treatment:** Total treatment duration was 12 weeks (6 weeks screening and 6 weeks treatment) with the option to extend screening by 1 additional week. |
| **Inclusion and Exclusion Criteria:**<br>**Inclusion Criteria:**<br>1. Provision of written informed consent prior to any study-specific procedure;<br>2. Age ≥18 years or legal age of majority based on regional law, whichever is greater, at Week -6 (Visit S1); |
| 3. Fasting calculated LDL-C between 130-189 mg/dL at Week -1 (Visit S2) following washout of all LDL-C-lowering drugs and nutritional supplements;<br>Note: LDL-C may be repeated 1 time with the screening period extended up to 1 week.<br>For those patients who have a repeat LDL-C, the mean of the first value and the repeat value will be used to determine eligibility.<br>4. Patient is sufficiently stable and suitable to undergo washout of all LDL-C-lowering drugs and nutritional supplements for 12 weeks (with potential for 1 week extension) based on Investigator assessment;<br>5. Men and nonpregnant, nonlactating women. Women must be one of the following:<br>a. Naturally postmenopausal defined as ≥1 year without menses and:<br>i. ≥55 years, or<br>ii. <55 years with follicle-stimulating hormone (FSH) ≥40.0 IU/L, or<br>b. Surgically sterile including hysterectomy, bilateral oophorectomy, and/or tubal ligation, or<br>c. Women of childbearing potential willing to use one acceptable method of birth control during the study and for 30 days after the end of treatment including:<br>i. birth control medications,<br>ii. placement of an intrauterine device with or without hormones,<br>iii. barrier methods including condom or occlusive cap with spermicidal foam or spermicidal jelly,<br>iv. vasectomized male partner who is the sole partner for this patient,<br>v. true abstinence (not including periodic abstinence such as calendar, ovulation, symptothermal, postovulation methods, or withdrawal);<br>There are no protocol-specific birth control requirements for men with partners who are able to become pregnant. |
| **Exclusion Criteria:**<br>1. Body mass index (BMI) >50 kg/$m^2$;<br>2. History of documented clinically significant cardiovascular disease including, but not limited to: |

(continued)

| | | |
|---|---|---|
| | | a. Myocardial infarction, severe or unstable angina pectoris, coronary angioplasty, coronary artery bypass graft, stroke, transient ischemic attack, cerebrovascular event, symptomatic carotid artery disease, or symptomatic peripheral arterial disease, |
| | | b. Uncontrolled hypertension, defined as sitting mean systolic blood pressure (SBP) ≥160 mm Hg and/or diastolic blood pressure (DBP) ≥100 mm Hg after sitting quietly for 5 minutes. |
| | | Note: At the discretion of the investigator, a single repeat sitting mean SBP and DBP may be completed at a separate visit. For those patients who have repeat sitting mean SBP and DBP assessment, the repeat values will be used to determine eligibility. |
| | | c. An arrhythmia requiring medical intervention, |
| | | d. Abdominal aortic aneurysm, |
| | | e. New York Heart Association (NYHA) Class III and IV heart failure; |
| | 3. Fasting TG >400 mg/dL at Week -1 (S2); | |
| | | Note: TG may be repeated 1 time with the screening period extended up to 1 week. For those patients who have a repeat TG, the repeat value will be used to determine eligibility. |
| | 4. History of type 1 or type 2 diabetes or fasting glucose >125 mg/dL at Week -6 (Visit S1); | |
| | 5. Uncontrolled hypothyroidism, including thyroid-stimulating hormone (TSH) >1.5 × the upper limit of normal (ULN) at Week -6 (Visit S1); | |
| | 6. Liver disease or dysfunction, including: | |
| | | a. Positive serology for hepatitis B surface antigen (HBsAg) and/or hepatitis C antibodies (HCV-ABVivi) at Week -1 (Visit S2), or |
| | | b. Alanine aminotransferase (ALT), aspartate aminotransferase (AST) ≥2 × ULN, and/or total bilirubin (TB) ≥2 × ULN at Week -6 (Visit S1). If TB ≥1.2 × ULN, a reflex indirect (unconjugated) bilirubin will be obtained and if consistent with Gilbert's disease or if the patient has a history of Gilbert's Disease, the patient may be enrolled in the study. |
| | | Note: At the discretion of the investigator, a single repeat of ALT, AST, and/or TB may be completed. For those patients who have a repeat ALT, AST, and/or TB, the repeat value will be used to determine eligibility. Also, if test for hepatitis C antibody is positive, but optional reflexive test for hepatitis C ribonucleic acid (RNA) is negative, the patient can be enrolled. |
| | 7. Renal dysfunction or glomerulonephritis, including estimated glomerular filtration rate (eGFR; using central laboratory determined Modification of Diet in Renal Disease [MDRD] formula) <30 mL/min at Week -6 (Visit S1); | |

(continued)

| |
|---|
| Note: At the discretion of the investigator, a single repeat of eGFR may be completed.<br>For those patients who have a repeat eGFR, the repeat value will be used to determine eligibility;<br>8. Gastrointestinal conditions or procedures (including weight loss surgery; *e.g.*, Lap-Band® or gastric bypass) that may affect drug absorption;<br>9. Hematologic or coagulation disorders or a hemoglobin (Hgb) level <10.0 g/dL at Week - 6 (Visit S1);<br>10. Active malignancy, including those requiring surgery, chemotherapy, and/or radiation in the past 5 years. Nonmetastatic basal or squamous cell carcinoma of the skin and cervical carcinoma in situ are allowed;<br>11. Unexplained creatine kinase (CK) >3 × ULN at any time prior to randomization (*i.e.*, not associated with recent trauma or physically strenuous activity). Patients with an explained CK elevation must have single repeat CK ≤3 × ULN prior to randomization;<br>12. History of drug or alcohol abuse within the last 2 years or reported current consumption of >14 alcoholic drinks/week, or any illicit drug use, history of amphetamine and derivatives abuse or cocaine abuse. Subjects with amphetamine derivatives prescribed by and under the care of a health care practitioner can be enrolled after evaluation by the investigator;<br>13. Blood donation, participation in a multiple blood draws, clinical study, major trauma, blood transfusion or surgery with or without blood loss within 30 days prior to randomization;<br>14. Use of any experimental or investigational drugs within 30 days prior to screening;<br>15. Previous enrollment in a bempedoic acid clinical study;<br>16. Use of these prohibited drugs and/or nutritional supplements prior to randomization or planned use during the study:<br>a. LDL-C lowering drugs and/or nutritional supplements (within 5 weeks prior to randomization),<br>b. Probenecid or cyclosporine (within 2 weeks prior to randomization),<br>c. Potent CYP3A4 inhibitors including amiodarone, azoles (fluconazole, itraconazole, ketoconazole, posaconazole, voriconazole), bosentan, clarithromycin, cobicistat, |
| conivaptan, danazol, daptomycin, diltiazem, domperidone, erlotinib, erythromycin, fusidic acid, mibefradil, nefazodone, piperaquine, protease inhibitors (atazanavir, boceprevir, darunavir, delavirdine, fosamprenavir, indinavir, lopinavir, nelfinavir, ritonavir, saquinavir, telaprevir, tipranavir), quinupristin/ dalfopristin, telithromycin, verapamil (within 2 weeks prior to randomization),<br>d. Systemic corticosteroids (topical corticosteroids are allowed; within 5 weeks prior to randomization);<br>17. Planned initiation or dosing changes of these allowed drugs prior to or during the study:<br>a. Hormone replacement (within 5 weeks prior to randomization),<br>b. Thyroid replacement (within 5 weeks prior to randomization),<br>c. Obesity medication (within 3 months prior to randomization);<br>18. A medical or situational (*i.e.,* geographical) finding that in the investigator's opinion may compromise the patient's safety or ability to complete the study;<br>19. An employee or contractor of the facility conducting the study, or a family member of the principal investigator, co-investigator, or any Sponsor personnel. |
| **Investigational Medicinal Product, Dosage and Mode of Administration:**<br>• Bempedoic acid 180-mg tablets and matching placebo<br>• Ezetimibe 10-mg overencapsulated tablets and similarly matching placebo capsules |

(continued)

| |
|---|
| • Atorvastatin 20-mg overencapsulated tablets and similarly matching placebo capsules<br>Investigational medicinal product (IMP) will be ingested once daily with or without food. |
| **Non-Investigational Medicinal Product**<br>All other background drugs will be administered as prescribed by a physician |
| **Statistical Methods:**<br>Sample Size<br>The sample size of 40 randomized patients in the triplet therapy group and 20 randomized patients in the placebo group is expected to provide 90% power to detect a difference of 25% in the percent change from baseline to Week 6 in calculated LDL-C between the triplet therapy group and the placebo group. This calculation is based on a 2-sided t-test at the 5% level of significance, a common standard deviation of 26% and a dropout rate of 10%. The conservative but clinical meaningful treatment difference of 25% is chosen in order to have a reasonable sample size to assess other efficacy and safety information. |
| Analysis Populations<br>The modified Intent-to-Treat (mITT) population, used for all of the efficacy analyses, is defined as all randomized patients who received at least 1 dose of study drug and have a baseline assessment and at least 1 post baseline assessment, excluding any assessment taken more than 2 days after a dose of study drug.<br>The Safety Population (SP), used for all of the safety summaries, is defined as all randomized patients who received at least 1 dose of study medication. Patients in the SP will be included in the treatment group that they actually received, regardless of their randomized treatment. |
| Disposition and Baseline Characteristics<br>Disposition, including reason for withdrawal from the IMP and study, will be summarized by treatment group. Demographic information and patient characteristics including, but not limited to, gender, race, age, and baseline vital signs will also be summarized by treatment group.<br>Primary and Secondary Efficacy Analysis<br>The primary efficacy endpoint is the percent change from baseline to Week 6 in LDL-C. Baseline is defined as the mean of the values from Week -1 (Visit S2) and predose Day 1/Week 0 (Visit T1). An analysis of covariance (ANCOVA) with treatment group as factor and baseline LDL-C as covariate will be performed to compare triplet therapy versus placebo for the primary endpoint using the mITT population. Missing values at Week 6 will be imputed using the last observation carried forward (LOCF) procedure (only post baseline values will be carried forward). The least squares mean (LSM) and standard error (SE) will be provided for both treatment groups, along with the placebo-corrected LSM, its 95% confidence interval (CI), and associated p-value.<br>Secondary efficacy endpoints, which include the percent change from baseline to Week 6 in additional lipid and cardiometabolic biomarkers, will be analyzed in a similar manner as the primary efficacy endpoint. Baseline for non-HDL-C, HDL-C, TC, and TG is defined as the mean of the values from Week -1 (Visit S2) and predose Day 1/Week 0 (Visit T1), while baseline for ApoB and hs-CRP is defined as the predose Day 1/Week 0 (Visit T1) value.<br>Statistical testing of primary and secondary efficacy endpoints will be 2-sided and conducted at the 5% level of significance with no adjustment for multiple comparisons. |
| Safety Analyses<br>Descriptive summary will be provided for safety endpoints.<br>The summarization of AEs will include only treatment-emergent AEs (TEAEs). TEAEs and serious adverse events (SAEs) will be summarized by system organ class (SOC), severity, and relationship to IMP for each treatment group. Deaths and withdrawal from the IMP or study due to AEs will each be summarized by treatment group.<br>Clinical safety laboratories, including hematology, blood chemistry, glucose, and urinalysis; PE findings; vital signs; and weight will be summarized by the value and by change from baseline in the value (where appropriate) at each post baseline time point. |

The following abbreviations and specialist terms are used in this study protocol.

**Table 1: Abbreviations and Specialist Terms**

| Abbreviation or Specialist | Explanation |
|---|---|
| ACL | Adenosine triphosphate-citrate lyase |
| ACSVL1 | Very long-chain acyl-CoA synthetase 1 |
| ADR | Adverse drug reaction |
| AE | Adverse event |
| AESI | Adverse events of special interest |
| Alb | Albumin |
| ALK-P | Alkaline phosphatase |
| ALT | Alanine aminotransferase |
| ANCOVA | Analysis of covariance |
| ApoB | Apolipoprotein B |
| AST | Aspartate aminotransferase |
| $AUC_{0-24}$ | Area under the curve during 24 hours |
| BMI | Body mass index |
| BP | Blood pressure |
| BUN | Blood urea nitrogen |
| Ca | Calcium |
| CFR | Code of Federal Regulations |
| CHD | Coronary heart disease |
| CI | Confidence interval |
| CK | Creatine kinase |
| Cl | Chloride |
| CNS | Central nervous system |
| CoA | Acetyl-coenzyme A |
| CO2 | Carbon dioxide |
| CRF | Case report form |
| CRO | Contract research organization |
| CV | Cardiovascular |
| CVD | Cardiovascular disease |
| CYP | Cytochrome P450 |
| DBP | Diastolic blood pressure |
| ECG | Electrocardiogram |
| eCRF | Electronic case report form |
| EDC | Electronic data capture |
| eGFR | Estimated glomerular filtration rate |
| FDA | US Food and Drug Administration |
| FSH | Follicle-stimulating hormone |
| GCP | Good Clinical Practice |
| HBsAg | Hepatitis B surface antigen |

(continued)

| Abbreviation or Specialist | Explanation |
|---|---|
| Hct | Hematocrit |
| HCV | Hepatitis C virus |
| HCV-ABVivi | Hepatitis C antibodies |
| HDL-C | High-density lipoprotein cholesterol |
| Hgb | Hemoglobin |
| HMG-CoA | 3-hydroxy-3-methylglutaryl coenzyme A |
| hs-CRP | High-sensitivity C-reactive protein |
| IB | Investigator's Brochure |
| ICD | Informed Consent Document |
| ICH | International Conference on Harmonisation |
| IMP | Investigational medicinal product |
| IND | Investigational New Drug Application |
| INR | International normalized ratio |
| IRB | Institutional Review Board |
| IWRS | Interactive web response system |
| K | Potassium |
| LDH | Lactate dehydrogenase |
| LDL-C | Low-density lipoprotein cholesterol |
| LFT | Liver function test |
| LOCF | Last observation carried forward |
| LSM | Least squares mean |
| MCH | Mean corpuscular hemoglobin |
| MCHC | Mean corpuscular hemoglobin concentration |
| MCV | Mean corpuscular volume |
| MDRD | Modification of diet in renal disease |
| MED ID | Medication identification |
| MedDRA | Medical Dictionary for Regulatory Activities |
| mITT | Modified Intent-to-Treat |
| MRI | Magnetic resonance imaging |
| Na | Sodium |
| NOAEL | No-observed-adverse-effect level |
| non-HDL-C | Non-high-density lipoprotein cholesterol |
| NY14A | New York Heart Association |
| PCSK9 | Proprotein convertase subtilisin kexin type 9 |
| PE | Physical exam |
| PK | Pharmacokinetic(s) |
| PT | Prothrombin time |
| RBC | Red blood cell |

(continued)

| Abbreviation or Specialist | Explanation |
|---|---|
| RNA | Ribonucleic acid |
| SAE | Serious adverse event |
| SAP | Statistical Analysis Plan |
| SBP | Systolic blood pressure |
| SE | Standard error |
| SGOT | Serum glutamic oxaloacetic transaminase |
| SGPT | Serum glutamic pyruvic transaminase |
| SOC | System organ class |
| SOP | Standard operating procedures |
| SP | Safety population |
| SUSAR | Suspected and unexpected serious adverse |
| T2DM | Type 2 diabetes mellitus |
| TB | Total bilirubin |
| TC | Total cholesterol |
| TEAE | Treatment-emergent adverse event |
| TG | Triglycerides |
| TSH | Thyroid-stimulating hormone |
| ULN | Upper limit of normal |
| US | United States |
| WBC | White blood cell |
| WHO | World Health Organization |

*Dose Selection*

Doses of Bempedoic acid ranging from 40 to 240 mg/day have been evaluated in the Phase 2 program. Based on data from the integrated analysis of safety and efficacy, observed values and percent change from baseline in the primary efficacy endpoint, LDL-C, together with a positive safety profile, support the choice of the 180-mg dose for the Phase 3 studies. An integrated analysis of six Phase 2 studies resulted in placebo-adjusted LS means for percent change from baseline of approximately 32% with Bempedoic acid 180 mg monotherapy, 50% with Bempedoic acid 180 mg + Ezetimibe 10 mg, and 22% for 180 mg on top of stable statin therapy. The 180-mg dose was noted to have an excellent safety profile. Overall, balancing efficacy and safety, the 180-mg dose was chosen for the Phase 3 program.

Background on Ezetimibe

Please see the ezetimibe label.

Background on Atorvastatin

Please see the atorvastatin label.

Risk Benefit Summary

To date, the nonclinical and clinical data indicate that bempedoic acid 180 mg has a favorable risk-benefit profile. The ability of Bempedoic acid to achieve clinically meaningful LDL-C lowering while demonstrating a favorable tolerability profile in a variety of patient populations supports continued development of Bempedoic acid in Phase 3 studies.

Several Phase 2 clinical studies have assessed the safety and efficacy of Bempedoic acid as add-on to statins or in combination with ezetimibe. These studies showed significant LDL-C lowering and a favorable safety profile. The LDL-C lowering efficacy and safety of triplet therapy with Bempedoic acid 180 mg, Ezetimibe 10 mg, and Atorvastatin 20 mg has not previously been assessed. That is the purpose of this study.

**TRIAL OBJECTIVES AND PURPOSE**

Objectives

*Primary Objective*

The primary objective was to assess the LDL-C lowering efficacy of triplet therapy with bempedoic acid 180 mg, ezetimibe 10 mg, and atorvastatin 20 mg versus placebo administered daily for 6 weeks in patients with elevated LDL-C.

*Secondary Objectives*

- To assess the effect of triplet therapy versus placebo on non-high-density lipoprotein cholesterol (non-HDL-C), total cholesterol (TC), apolipoprotein B (ApoB), high-sensitivity C-reactive protein (hs-CRP), TG, and HDL-C
- To assess the effect of triplet therapy versus placebo on percent of patients achieving LDL-C level <70 mg/dL
- To assess the effect of triplet therapy versus placebo on percent of patients achieving LDL-C reduction ≥50%
- To assess the safety and tolerability of triplet therapy versus placebo

Study Endpoints

The following endpoints were used to evaluate the objectives of the study.

*Primary Endpoint*

- Percent change from baseline to Week 6 in LDL-C

*Secondary Endpoints*

- Percent change from baseline to Week 6 in non-HDL-C, TC, ApoB, hs-CRP, TG, and HDL-C
- Percent of patients with LDL-C <70 mg/dL at Week 6
- Percent of patients with LDL-C reduction ≥50% from baseline to Week 6

*Safety Endpoints*

- Subject incidence of adverse events
- Clinical safety laboratory (including hematology, blood chemistry, and urinalysis) results
- Vital signs and physical examination (PE) findings

**INVESTIGATIONAL PLAN**

Overall Study Design

This was a Phase 2, multicenter, randomized, double-blind, placebo-controlled, parallel group study. Patients initially undergo screening at Week -6 (Visit S1). Eligible patients have begun washout of all LDL-C-lowering drugs and

nutritional supplements at least 5 weeks prior to randomization. Patients have returned at Week -1 (S2) for lipid and/or other assessments. Patients who are deemed not eligible for randomization at any point during screening were notified by clinical site personnel and considered screen failures. At Week 0 (Visit T1), approximately 60 patients were randomized in a ratio of 2:1 to receive either triplet therapy (bempedoic acid 180 mg + ezetimibe 10 mg + atorvastatin 20 mg) or placebo once daily for 6 weeks. Randomized patients return for clinic visits at Week 3 (Visit T2) and Week 6 (Visit T3).

For details of study assessments, see the Schedule of Events in Appendix 1.

Study Hypothesis

The clinical hypothesis for this study is that triplet therapy with Bempedoic acid 180 mg, Ezetimibe 10 mg, and Atorvastatin 20 mg significantly reduce LDL-C in patients treated daily for 6 weeks versus placebo.

Estimated Study Duration and Period

Total treatment duration will be 12 weeks (6 weeks screening and 6 weeks treatment) with the option to extend screening by 1 additional week.

Number of Centers

Up to approximately 20 centers in the US have participated in this study. Additional sites may be invited to participate to ensure study timelines are met.

Number of Patients

The study has enrolled approximately 60 adult male and female patients.

**SELECTION OF PATIENTS**

Subject Inclusion Criteria

Each patient met the following criteria to be eligible for this study.

1. Provision of written informed consent prior to any study-specific procedure;

2. Age ≥18 years or legal age of majority based on regional law, whichever is greater, at Week -6 (Visit S1);

3. Fasting calculated LDL-C between 130-189 mg/dL at Week -1 (Visit S2) following washout of all LDL-C-lowering drugs and nutritional supplements;
Note: LDL-C may be repeated 1 time with the screening period extended up to 1 week. For those patients who have a repeat LDL-C, the mean of the first value and the repeat value was used to determine eligibility.

4. Patient was sufficiently stable and suitable to undergo washout of all LDL-C-lowering drugs and nutritional supplements for 12 weeks (with potential for 1 week extension) based on Investigator assessment;

5. Men and nonpregnant, nonlactating women. Women must be one of the following:

   a. Naturally postmenopausal defined as ≥1 year without menses and:

      i. ≥55 years, or

      ii. <55 years with follicle-stimulating hormone (FSH) ≥40.0 IU/L, or

   b. Surgically sterile including hysterectomy, bilateral oophorectomy, and/or tubal ligation, or

   c. Women of childbearing potential willing to use one acceptable method of birth control during the study and for 30 days after the end of treatment including:

      i. birth control medications,

ii. placement of an intrauterine device with or without hormones,

iii. barrier methods including condom or occlusive cap with spermicidal foam or spermicidal jelly,

iv. vasectomized male partner who is the sole partner for this patient,

v. true abstinence (not including periodic abstinence such as calendar, ovulation, symptothermal, postovulation methods, or withdrawal);

There were no protocol-specific birth control requirements for men with partners who are able to become pregnant.

Subject Exclusion Criteria

Patients who meet any of the following criteria were not be eligible to participate:

1. Body mass index (BMI) >50 kg/$m^2$;

2. History of documented clinically significant cardiovascular disease including, but not limited to:

a. Myocardial infarction, severe or unstable angina pectoris, coronary angioplasty, coronary artery bypass graft, stroke, transient ischemic attack, cerebrovascular event, symptomatic carotid artery disease, or symptomatic peripheral arterial disease,

b. Uncontrolled hypertension, defined as sitting mean systolic blood pressure (SBP) ≥160 mm Hg and/or diastolic blood pressure (DBP) ≥100 mm Hg after sitting quietly for 5 minutes.
Note: At the discretion of the investigator, a single repeat sitting mean SBP and DBP may be completed at a separate visit. For those patients who have repeat sitting mean SBP and DBP assessment, the repeat values will be used to determine eligibility.

c. An arrhythmia requiring medical intervention,

d. Abdominal aortic aneurysm,

e. New York Heart Association (NYHA) Class III and IV heart failure;

3. Fasting TG >400 mg/dL at Week -1 (S2);
Note: TG may be repeated 1 time with the screening period extended up to 1 week. For those patients who have a repeat TG, the repeat value was used to determine eligibility.

4. History of type 1 or type 2 diabetes or fasting glucose >125 mg/dL at Week -6 (Visit S1);

5. Uncontrolled hypothyroidism, including thyroid-stimulating hormone (TSH) >1.5 × the upper limit of normal (ULN) at Week -6 (Visit S1);

6. Liver disease or dysfunction, including:

a. Positive serology for hepatitis B surface antigen (HBsAg) and/or hepatitis C antibodies (HCV-ABVivi) at Week -1 (Visit S2), or

b. Alanine aminotransferase (ALT), aspartate aminotransferase (AST) ≥2 × ULN, and/or total bilirubin (TB) ≥2 × ULN at Week -6 (Visit S1). If TB ≥1.2 × ULN, a reflex indirect (unconjugated) bilirubin will be obtained and if consistent with Gilbert's disease or if the patient has a history of Gilbert's Disease, the patient may be enrolled in the study.
Note: At the discretion of the investigator, a single repeat of ALT, AST, and/or TB may be completed. For those patients who have a repeat ALT, AST, and/or TB, the repeat value was used to determine eligibility. Also, if test for hepatitis C antibody is positive, but optional reflexive test for hepatitis C ribonucleic acid (RNA) is negative, the patient can be enrolled.

7. Renal dysfunction or glomerulonephritis, including estimated glomerular filtration rate (eGFR; using central laboratory determined Modification of Diet in Renal Disease [MDRD] formula) <30 mL/min at Week -6 (Visit S1); Note: At the discretion of the investigator, a single repeat of eGFR may be completed. For those patients who have a repeat eGFR, the repeat value was used to determine eligibility;

8. Gastrointestinal conditions or procedures (including weight loss surgery; e.g., Lap-Band® or gastric bypass) that may affect drug absorption;

9. Hematologic or coagulation disorders or a hemoglobin (Hgb) level <10.0 g/dL at Week -6 (Visit S1);

10. Active malignancy, including those requiring surgery, chemotherapy, and/or radiation in the past 5 years. Nonmetastatic basal or squamous cell carcinoma of the skin and cervical carcinoma in situ are allowed;

11. Unexplained creatine kinase (CK) >3 × ULN at any time prior to randomization (ie, not associated with recent trauma or physically strenuous activity). Patients with an explained CK elevation must have single repeat CK ≤3 × ULN prior to randomization;

12. History of drug or alcohol abuse within the last 2 years or reported current consumption of >14 alcoholic drinks/week, or any illicit drug use, history of amphetamine and derivatives abuse or cocaine abuse. Subjects with amphetamine derivatives prescribed by and under the care of a health care practitioner can be enrolled after evaluation by the investigator;

13. Blood donation, participation in a multiple blood draws, clinical study, major trauma, blood transfusion or surgery with or without blood loss within 30 days prior to randomization;

14. Use of any experimental or investigational drugs within 30 days prior to screening;

15. Previous enrollment in a bempedoic acid clinical study;

16. Use of these prohibited drugs and/or nutritional supplements prior to randomization or planned use during the study:

a. LDL-C lowering drugs and/or nutritional supplements (within 5 weeks prior to randomization),

b. Probenecid or cyclosporine (within 2 weeks prior to randomization),

c. Potent CYP3A4 inhibitors including amiodarone, azoles (fluconazole, itraconazole, ketoconazole, posaconazole, voriconazole), bosentan, clarithromycin, cobicistat, conivaptan, danazol, daptomycin, diltiazem, domperidone, erlotinib, erythromycin, fusidic acid, mibefradil, nefazodone, piperaquine, protease inhibitors (atazanavir, boceprevir, darunavir, delavirdine, fosamprenavir, indinavir, lopinavir, nelfinavir, ritonavir, saquinavir, telaprevir, tipranavir), quinupristin/dalfopristin, telithromycin, verapamil (within 2 weeks prior to randomization),

d. Systemic corticosteroids (topical corticosteroids are allowed; within 5 weeks prior to randomization);

17. Planned initiation or dosing changes of these allowed drugs prior to or during the study:

a. Hormone replacement (within 5 weeks prior to randomization),
b. Thyroid replacement (within 5 weeks prior to randomization),
c. Obesity medication (within 3 months prior to randomization);

18. A medical or situational (ie, geographical) finding that in the investigator's opinion may compromise the patient's safety or ability to complete the study;

19. An employee or contractor of the facility conducting the study, or a family member of the principal investigator, co-investigator, or any Sponsor personnel.

Patient Lifestyle and Dietary Guidelines

Patients have fasted for a minimum of 10 hours prior to collection of all laboratory samples (water and concomitant medications, but not IMP are permitted).
Beginning at screening, patients were counseled to follow a heart healthy diet as per local or regional guidelines and should be encouraged (as able) to participate in a stable, regular exercise program throughout the study.

## TREATMENT OF PATIENTS

Administration of Investigational Medicinal Product

During the Treatment Period, patients were randomized to receive IMP of either Bempedoic acid 180 mg + Ezetimibe 10 mg + atorvastatin 20 mg or placebo once daily. Each daily allotment of IMP was comprised of one bempedoic acid tablet, one overencapsulated Ezetimibe tablet and one overencapsulated atorvastatin tablet provided in a blister package. The placebo blister pack contained the corresponding matching placebo of the three drugs. Patients were instructed to ingest IMP orally once daily with or without food. On clinic visit days, patients were instructed to delay ingestion of IMP until all study procedures were completed.
If the patient forgets to take IMP on nonclinic visit days, it may be taken up to 12 hours later the same day. After that time, the patient did not take IMP that day and has resumed ingestion of IMP the following morning. Details describing the reasons for missed doses have been documented in the patient's medical records and electronic case report form (eCRF). Extra IMP was provided and can be used, if needed, prior to the next visit or to replace an allotment of IMP that cannot be used because it is lost or damaged.
Other details regarding IMP, including description, supply and control, accountability, handling, and disposal were provided in Section 9.

Prior and Concomitant Medications

Patients were questioned about their concomitant medication use at each clinic visit. All concomitant medication taken chronically or intermittently during the study were recorded with indication, total daily dose, and start and stop dates of administration.
The Prior/Concomitant case report form (CRF) was used to record medications, herbal remedies, vitamins, other nutritional supplements, and over-the-counter medications taken within 6 weeks prior to screening and during the study.

*Prohibited Medications and Dietary Supplement*

Patients were required to stop taking all prescription or nonprescription drugs or nutritional supplements taken for the purpose of lipid regulation at least 5 weeks prior to randomization (approximately Study Day -35). Use of any drug(s) listed below either in mono or combination therapy was prohibited during the study:

Statins (within 5 weeks prior to randomization):

- Atorvastatin (LIPITOR®) (other than sponsor provided)
- Fluvastatin (LESCOL®)
- Lovastatin (MEVACOR®, ALTOPREV™)
- Pravastatin (PRAVACHOL®)
- Rosuvastatin Calcium (CRESTOR®)
- Simvastatin (ZOCOR®)
- Pitavastatin (LIVALO®)

Selective cholesterol and/or bile acid absorption inhibitors (within 5 weeks prior to randomization):

- Ezetimibe (ZETIA®, EZETROL®)
- Cholestyramine (QUESTRAN®, QUESTRAN® LIGHT, PREVALITE®, LOCHOLEST®, LOCHOLEST® LIGHT)
- Colestipol (COLESTID®)
- Colesevelam HCl (WELCHOL®, CHOLESTAGEL®)

Fibrates (within 5 weeks prior to randomization):

- Gemfibrozil (LOPID®)
- Fenofibrate (ANTARA®, LOFIBRA®, TRICOR®, AND TRIGLIDE™, LIPANTIL®, SUPRALIP®)
- Clofibrate (ATROMID-S®)
- Ciprofibrate (MODALIM®)
- Bezafibrate (BEZALIP®)

PCSK9 inhibitors (within 5 weeks prior to randomization):

- Evolocumab (REPATHA®)
- Alirocumab (PRALUENT®)

Other Lipid Regulating Drugs (within 5 weeks prior to randomization):

- Niacin (NIASPAN® Rx and OTC)
- Omega-3-acid Ethyl Esters (LOVAZA® and OTC fish oil)
- Statin fixed dose combinations (e.g., ATOZET®, VYTORIN®, INEGY®)

Lipid Altering Nutritional Supplements (within 5 weeks prior to randomization):

- Berberine
- Psyllium (Metamucil®)
- Green tea extract
- Niacin (crystalline >500 mg/day or slow release or timed release at any dose)
- Sitostanol (found in oral nutritional supplements and some margarines, such as Benecol)
- Beta-sitosterol (found in oral nutritional supplements and some margarines, such as Promise Activ)
- Cholestin (red yeast rice, also known as monascus purpureus extract)
- Pantothine
- Policosanol

Other Drugs:

- Probenecid or cyclosporine (within 2 weeks prior to randomization)
- Potent CYP3A4 inhibitors including amiodarone, azoles (fluconazole, itraconazole, ketoconazole, posaconazole, voriconazole), bosentan, clarithromycin, cobicistat, conivaptan, danazol, daptomycin, diltiazem, domperidone, erlotinib, erythromycin, fusidic acid, mibefradil, nefazodone, piperaquine, protease inhibitors (atazanavir, boceprevir, darunavir, delavirdine, fosamprenavir, indinavir, lopinavir, nelfinavir, ritonavir, saquinavir, telaprevir, tipranavir), quinupristin/dalfopristin, telithromycin, verapamil (within 2 weeks prior to randomization)
- Systemic corticosteroids (topical corticosteroids were allowed (within 5 weeks prior to randomization)

*Permitted Medications*

Permitted medications were stable for at least 2 weeks prior to Visit S 1 (Week -6) with the exception of the following:

- Hormone replacement (within 5 weeks prior to randomization)

• Thyroid replacement (within 5 weeks prior to randomization)

• Obesity medication (within 3 months prior to randomization)

Treatment Assignment, Randomization, and Blinding

During the Treatment Period, patients received double-blind IMP. At Day 1 (Visit T1), patients were randomized to receive either bempedoic acid 180 mg/day + ezetimibe 10 mg + atorvastatin 20 mg or placebo. The investigator or designee utilized Interactive web response system (IWRS) during the visit to obtain a randomization number and the appropriate IMP container via medication identification numbers (MED ID). A patient was considered to be randomized when they had been assigned a randomization number by IWRS.

The randomization number was determined by a computer-generated random code and corresponds to a treatment group according to patient's sequential entrance into the study. The randomization schedule for blinding of treatment assignment was generated by the contract research organization (CRO), provided to IWRS, and released only after the study is complete and the database is locked.

During the Treatment Period, Sponsor, site personnel, CRO, and patient were/are all be unaware of patient's treatment assignment.

Blinding of treatment was maintained for all patients unless, in the opinion of the investigator, the safety of the patient may be at risk. Only under the rarest of circumstances should the investigator consider breaking the blind and only when medical/supportive care cannot be provided without determining if the patient is receiving active drug treatment. In the event that the blind needs to be broken prior to completion of the study, the investigator should contact the appropriate Medical Monitor by telephone. If the blind must be broken prior to consultation with the Medical Monitor, contact must be made within 24 hours of breaking the blind.

At the initiation of the study, the clinical site was instructed on procedures for breaking the blind via the IWRS. In all cases of breaking the blind, the investigator documents in the patient's medical record the date, time, and reason for breaking the blind, and the names of personnel involved.

Post-randomization values for individual laboratory measures for LDL-C, non-HDL-C, TC, TG and HDL-C that may inadvertently suggest treatment assignment will not be available to personnel from the clinical site, the patient, the Sponsor, or the CRO. While knowledge of these values does not truly 'unblind,' the collection of these lab assessments by the investigator, all collaborating physicians, or the patients locally (outside the study visits) were strongly discouraged. Investigators have not performed testing of these analytes at the local lab during the conduct of the study.

**INVESTIGATIONAL MEDICINAL PRODUCT**

Description of Investigational Medicinal Product

**Table 2: Investigational Medicinal Products**

| **Product Name:** | **Investigational Medicinal Product** | |
|---|---|---|
| | **Bempedoic acid** | **Placebo to Match Bempedoic** |
| Dosage Form: | Film-coated tablets | Film-coated tablets |
| Unit Dose: | 180 mg | Not applicable |
| Container/ Closure: | 9 count blister card with child resistant closures | 9 count blister card with child resistant closures |
| Route of Administration: | Oral, daily at approximately the same time, with or without food | Oral, daily at approximately the same time, with or without food |
| Physical Description: | Oval, white to off-while film-coated tablet debossed with "ABC" on one face and debossed with "000" on the opposite face | Oval, white to off-while film-coated tablet debossed with "ABC" on one face and debossed with "000" on the opposite face |
| Man ufacturer (Fill/ Finish): | Patheon 2100 Syntex Court Mississauga, Ontario L5N 7K9 | Patheon 2100 Syntex Court Mississauga, Ontario L5N 7K9 |

(continued)

| Product Name: | Investigational Medicinal Product | |
|---|---|---|
| | **Ezetimibe** | **Placebo to Match Ezetimibe** |
| Dosage Form: | Overencapsulated tablets | Capsules |
| Unit Dose: | 10 mg | Not applicable |
| Container/ Closure: | 9-count blister card with child-resistant closures | 9-count blister card with child-resistant closures |
| Route of Administration: | Oral, daily at approximately the same time, with or without food | Oral, daily at approximately the same time, with or without food |
| Physical | TBD | TBD |
| Man ufacturer (Fill/ Finish): | TBD | Sharp Clinical Services Inc. 300 Kimberton Road |
| **Product Name:** | **Investigational Medicinal Product** | |
| | **Atorvastatin** | **Placebo to Match Atorvastatin** |
| Dosage Form: | Overencapsulated tablets | Capsules |
| Unit Dose: | 20 mg | Not applicable |
| Container/ Closure: | 9-count blister card with child-resistant closures | 9-count blister card with child-resistant closures |
| Route of Administration: | Oral, daily at approximately the same time, with or without food | Oral, daily at approximately the same time, with or without food |
| Physical | TBD | TBD |
| Man ufacturer (Fill/ Finish): | TBD | Sharp Clinical Services Inc. 300 Kimberton Road |

Please see Pharmacy Manual for detailed storage requirements and instructions.

Investigational Medicinal Product Supply and Control

The Sponsor has supplied the IMP for this study as described above. IMP was distributed and released in accordance with regional and local requirements during the conduct of the study.

The MED ID number (an identifier on the IMP packaging) was obtained via IWRS and used to select double-blind IMP from available clinical supplies at the clinical site.

IMP was dispensed by the investigator or other qualified site personnel only to appropriate patients who had provided written informed consent.

Packaging and Labeling

Double-blind IMP was packaged in blister pack. Each blister pack contained a 9-day supply and 3 blister packs were provided in drug kit.

The IMP labels included protocol number, MED ID number, patient identification number, lot number, site number and investigator name in addition to standard language regarding warnings and regulations, administration and storage of the product.

Investigational Medicinal Product Adherence

At each clinic visit during the Treatment Period, designated clinical site staff assessed patient IMP intake adherence by counting the number of doses that are returned as unused and by querying the patient with regards to daily intake. If the patient has not taken multiple doses as instructed, the patient was queried for a reason, findings were documented, and the patient was counseled on the importance of carefully following all dosing instructions. Factors contributing to poor adherence were determined and, if possible, remedied. Patients demonstrating poor adherence

during the Treatment Period will continue to be counseled on the importance of carefully following all dosing instructions, but will not be removed from the study.

Investigational Medicinal Product Accountability

Patients were instructed to return all packaging and unused IMP at every visit for assessment of adherence and drug accountability.

Accurate records of the receipt of all IMP shipped by the Sponsor (or designee) and the disposition of that IMP were maintained.

IMP records or logs must comply with applicable regulations, local law, and guidelines, and should include:

- Amount received/placed in storage area
- Amount currently in storage area
- MED ID number for all IMP
- Dates and initials of person(s) responsible for IMP inventory (including entry/movement/disposition)
- Date and amount of IMP dispensed to each patient, including unique patient identifiers
- Date that IMP was returned by patient, assessment of adherence, and relevant documentation of discrepancies
- Nonstudy disposition (e.g., lost, broken, wasted)
- Amount returned to Sponsor (or designee)/destroyed or amount destroyed per local standard operating procedure (SOP) following accountability by site monitor.

Investigational Medicinal Product Handling, Storage, and Disposal

The Principal Investigator ensures that all IMP is stored in a secured area, under recommended storage conditions (at room temperature [15°C/59°F to 30°C/86°F] protected from any extreme conditions of temperature, light, or humidity) in accordance with applicable regulatory requirements for investigational drugs. Access to IMP was/is limited to those clinical site personnel authorized by the investigator. Upon completion or termination of the study, all IMP and used and unused IMP packaging must be returned to the Sponsor (or designee) for eventual destruction unless otherwise authorized by the Sponsor. All IMP returns must be accompanied by the appropriate documentation.

**STUDY PROCEDURES AND SCHEDULE OF ASSESSMENTS**

Informed Consent

The patient was adequately informed of the nature and risks of the study and understands the Informed Consent Document (ICD). It is the investigator's responsibility that no study-related procedure will be performed until the each patient has been completely informed of the study, has freely consented to take part in the study, and has signed and dated an ICD approved by the Sponsor (or designee) and the Institutional Review Board (IRB). The written ICD should be prepared in the local language(s) of the potential patient population.

Interactive Web Response System and eCRFs

Data was captured on eCRFs. Randomization, IMP (re)ordering, IMP distribution, and patient status tracking has occurred via IWRS. Instructions for these systems and additional contact time points for IWRS was provided separately.

Patient Identification Numbers

**[0187]** A unique patient identification number was assigned to each patient to identify each patient throughout the study. Patient identification numbers were assigned sequentially by IWRS at the time of informed consent during the screening module transaction and was comprised of protocol, site, and patient-specific numbers.

Rescreening

Patients who are screening failures due to stability requirements for a condition or concurrent medication or other reason may be considered for rescreening after consultation with the Sponsor (or designee). If rescreened, these patients must also be reconsented and screening procedures must be repeated. If a patient is a screen failure, or if a patient discontinues from the study, their patient ID number will not be assigned to another patient.

Procedures and Schedule of Assessments

The study is comprised of 2 distinct periods: screening and double-blind treatment.
The schedule of study events is provided in Appendix 1. However, a patient can be seen at any time for reasons of safety.

*Screening Week -6 (Visit S1; Day -42 to Day -37)*

The screening period has begun with a screening visit that occurs 6 weeks prior to randomization. Visit S1 has allowed the investigator to assess the patient's preliminary eligibility. After the patient provides written informed consent (see Section 10.1), patients have undergone the following assessments and procedures at Visit S1:

• Assess AEs and serious adverse events (SAEs) (starting from signing the informed consent document)

• Demographics

• Clinically relevant medical history

• Prior and concomitant medication review

• Review of all inclusion/exclusion criteria that can be assessed at this time

• Height (cm), and weight (kg)

• Vital signs

• Central clinical laboratory evaluations:

  - Thyroid-stimulating hormone (TSH)

  - Hematology, blood chemistry, and urinalysis

  - Basic fasting lipids (TC, calculated LDL-C, HDL-C, non-HDL-C, and TG)

  - Serum pregnancy test (in female patients of childbearing potential) or follicle-stimulating hormone (FSH) (on postmenopausal women <55 years of age)

• Dietary and lifestyle counselling

• Contact IWRS to register the patient

Patients who meet all enrollment criteria that can be assessed following review of the Visit S1 central clinical laboratory results (available several days after Visit S 1) were instructed to washout of all lipid-regulating drugs and nutritional supplements and to maintain consistent diet and exercise patterns throughout the study. Patients who fail to meet any entry criterion that can be assessed at Visit S1 are considered to be screen failures and were not required to return for additional visits (although a patient can be seen at any time for safety reasons).

*Screening Week -1 (Visit S2; Day -10 to -7)*

The patient has undergone the following assessments and procedures at Visit S2:

• Concomitant medication review (ongoing)

• Assess AEs and SAEs

• Central clinical laboratory evaluations:

- Basic fasting lipids (TC, calculated LDL-C, HDL-C, non-HDL-C, and TG)

- Serology (including hepatitis B surface antigen [HBsAg], hepatitis C virus [HCV] antibody)

• Dietary and lifestyle counselling

Note:

• The screening period can be extended by an additional week and an additional visit may be completed prior to Visit T1 if patient fails to meet LDL-C and/or TG entry criterion. If this optional visit is completed, the mean of values from Visit S2 and the additional visit will be used to determine eligibility.

• An additional visit and/or assessment between Visit S 1 and Visit T1 MAY be completed if the patient fails to meet diastolic blood pressure (DBP), systolic blood pressure (SBP), TSH, estimated glomerular filtration rate (eGFR), alanine aminotransferase (ALT), aspartate aminotransferase (AST), serology and/or creatine kinase (CK) entry criteria. Patients may qualify for randomization after any associated medications have been adjusted and stabilized and the repeat assessment meets entry criteria.

*Treatment Week 0 (Visit T1; Day 1)*

Prior to scheduling Visit T1, screening results have been reviewed to determine whether the patient continues to meet eligibility criteria. At Visit T1, a physical exam and urine pregnancy test also has been completed prior to randomization. Patients not meeting all entry criteria at any point prior to randomization will be screen failures.

If the patient has met all inclusion criteria and none of the exclusion criteria, the patient have been randomized into the double-blind Treatment Period.

Patients are considered randomized once all eligibility criteria are confirmed and a randomization number is obtained by the IWRS on the day of first dose.

The patient has undergone the following assessments and procedures at Day 1 (Visit T1):

• Concomitant medication review (ongoing)

• Assess AEs and SAEs

• PE

• Review inclusion/exclusion criteria to establish patient eligibility

• Weight

• Vital signs

• Central clinical laboratory evaluations:

- Hematology, blood chemistry, and urinalysis
- Basic fasting lipids (TC, calculated LDL-C, HDL-C, non-HDL-C, and TG)
- ApoB and hs-CRP

• Urine pregnancy test (in female patients of childbearing potential)

• IWRS contact to obtain the patient randomization number and MED ID number for double-blind IMP

• Dispense double-blind IMP and provide dosing and storage instructions

• Dietary and lifestyle counselling

*Treatment Week 3 (Visit T2; Day 22 ± 3 days)*

Patients have undergone the following assessments and procedures at Week 3 (Visit T2):

• Concomitant medication review (ongoing)

• Assess AEs and SAEs

• Weight

• Vital signs

• Central clinical laboratory evaluations:

- Hematology, blood chemistry, and urinalysis
- Basic fasting lipids (TC, calculated LDL C, HDL C, non-HDL C, and TG)

• Return of IMP; assessment and recording of IMP dosing adherence

• IWRS contact to obtain new MED ID number for double-blind IMP

• Dispense double-blind IMP and provide dosing and storage instruction

• Dietary and lifestyle counselling

*Treatment Week 6 (Visit T3; Day 43 ± 3 days)/End of Study or Early Termination*

Patients have undergone the following assessments and procedures at Week 6 and/or End of Study/Early Termination:

• Concomitant medication review (ongoing)

• Assess AEs and SAEs

• PE

• Weight

• Vital signs

• Central clinical laboratory evaluations:

- Hematology, blood chemistry, and urinalysis

- Basic fasting lipids (TC, calculated LDL-C, HDL-C, non-HDL-C, and TG)

- ApoB and hs-CRP

• Return of IMP; assessment and recording of IMP adherence

Patient Withdrawal From the Study

Patients have remained in the study until the last scheduled visit at Study Day 43 (Visit T3) and have been considered as having completed participation in the study.

The patient’s decision to participate in the clinical study is voluntary. Patients may refuse to continue in the study and/or withdraw from participation in this study at any time, for any reason, specified or unspecified, and without

penalty or loss of benefits to which the patient is otherwise entitled.

It is the right and duty of the Investigator to interrupt the treatment of any patient whose health or well-being may be threatened by continuation in this study. Such patients have been withdrawn from the study and have not been continued under a modified regimen.

Patients who are withdrawn from the study may not re-enter. The reasons for withdrawal from this study may include:

- AE
- Patient's withdrawal of consent
- Failure to comply with the protocol
- Lost to follow-up
- Illness, condition, or procedural complication affecting the patient's ability to participate or requiring prohibited medication
- The Sponsor or Investigator terminates the study
- In the Investigator's judgment, it is deemed in the best interest of the patient to discontinue his/her participation in the study
- Any other reason

If a patient is lost to follow-up, every reasonable effort must be made by the clinical site personnel to contact the patient and determine the reason for discontinuation/withdrawal. The measures taken to follow-up must be documented.

## ASSESSMENT OF EFFICACY

Assessments of Lipids and hs-CRP

Central clinical laboratory samples were collected and analyzed for the parameters detailed in Table 3. LDL-C were calculated or measured directly if TG are >400 mg/dL or LDL-C is <50 mg/dL.

Blood draws for lipids (not safety) must meet the criteria below. If these criteria have not been met, these blood samples will NOT be collected. If these criteria were/are met by rescheduling clinic visit to occur within 3 days, these blood samples were/are collected at the rescheduled clinic visit only.

- Blood samples were drawn after a minimum 10-hour fast (water and concomitant medications [but not IMP] is allowed)
- Blood samples were drawn only if patient ingested IMP the previous day

Patients were in a seated position during the blood collection. Collection schedule and instructions are provided in the Central Clinical Laboratory Manual. A description of the sample collection, storage, and shipping as well as monitoring and management of abnormal laboratories are described in Section 12.1.5.

When vital signs and laboratory samples were collected at the same time point, vital sign measurements have preceded laboratory sample collection.

**Table 3: Central Clinical Laboratory Parameters (Lipids and hs-CRP)**

| **Clinical Laboratory Test** | **Clinical Laboratory Test** |
|---|---|
| Basic Lipid Parameters<br>• Total cholesterol (TC)<br>• Calculated low-density lipoprotein cholesterol (LDL-C) and non-HDL-C | Other Parameters<br>• High-sensitivity C-reactive protein (hs-CRP)<br>• Apolipoprotein B (ApoB) |

(continued)

| Clinical Laboratory Test | Clinical Laboratory Test |
|---|---|
| • High-density lipoprotein cholesterol (HDL-C)<br>• Triglycerides (TG) | |

**ASSESSMENT OF SAFETY**

Safety Parameters

At all clinic visits, investigators have reviewed safety information including vital signs, AEs, SAEs, and concomitant medications and have ensured that the collected data are recorded into the appropriate eCRF. Additionally, central clinical laboratory samples were collected and sent for analysis and the investigator has reviewed the results to ensure continued patient safety while participating in the study.

Investigators are responsible for monitoring the safety of patients who have entered this study and for alerting the Sponsor or its designee if appropriate.

The investigator is responsible for the appropriate medical care of patients during the study.

The investigator was responsible for following AEs that are serious or that caused the patient to discontinue before completing the study until the event is resolved or stable. Frequency of follow-up evaluation is/was left to the discretion of the investigator.

*Demographic/Medical History*

Demographic data and a complete medical history was obtained from the patient. For medical history, conditions that are relevant and/or clinically significant were captured with at least a start date (month and year) and whether the condition is ongoing or resolved. All surgeries regardless of date were reported.

*Vital Signs*

Vital signs include DBP and SBP as well as heart rate.

Vitals were collected prior to blood collection. Blood pressure (BP) and heart rate were measured using a calibrated, fully automated machine with a cuff that is appropriate to the size of the upper arm. If a fully automated machine was not available, BP was measured manually. The same method (either automated or manual) and the same arm (right or left) was/is used throughout the study. The patient was in a seated position with feet touching the floor. Patients was seated quietly for at least 5 minutes in a chair with their backs supported, their feet flat on the ground, and their arms bared and supported at heart level. At each clinic visit, 2 BP measurements was collected at 1- to 2-minute intervals.

*Weight and Height*

Body weight was measured on a calibrated scale in the morning while fasted and after voiding.

Height was measured using standard clinic procedures.

Body mass index (BMI) was calculated systematically using the formula:

$$\text{BMI (kg/m}^2\text{) = weight in kg/(height in meters)}^2$$

*Physical Examination*

Physical examinations include an assessment of the following:

- General appearance
- Skin
- Eyes, ears, nose, and throat

- Head and neck
- Extremities
- Musculoskeletal examination
- Respiratory examination
- Cardiovascular assessment, including rhythm and presence of cardiac abnormalities
- Abdominal examination
- Neurologic examination including documentation of the presence of abnormalities in mental status and motor and sensory function
- Any additional assessments necessary to establish baseline status or evaluate symptoms or adverse experiences

Documentation of the PE findings were included in the source documentation at the clinical site. Significant findings prior to the start of IMP were recorded on the Medical History/Current Medical Conditions page of the eCRF. Only changes from baseline physical examination findings that meet the definition of an AE were recorded on the AE page of the eCRF.

*Central Clinical Laboratory Tests*

Central Clinical Laboratory Parameters (Safety)

Patients were in a seated position during the blood collection. Clinical laboratory parameters and tests include those listed in Table 4. Collection schedule, schedule of laboratory parameters by visit, and instructions are in the Clinical Laboratory Manual provided by Central Laboratory.

**Table 4: Central Clinical Laboratory Parameters (Safety)**

| **Clinical Laboratory Test** | **Clinical Laboratory Test** |
|---|---|
| • Hematology<br>• Hematocrit (Hct)<br>• Hemoglobin (Hgb)<br>• Mean corpuscular hemoglobin (MCH)<br>• Mean corpuscular hemoglobin concentration (MCHC)<br>• Mean corpuscular volume (MCV)<br>• Platelet count<br>• Red blood (RBC) cell count<br>• White blood (WBC) cell count with differential (absolute values only) | Blood Chemistry (serum, fasting)<br>• Albumin (Alb)<br>• Alkaline phosphatase (Alk-P)<br>• Alanine aminotransferase (ALT; SGPT)<br>• Aspartate aminotransferase (AST; SCOT)<br>• Blood urea nitrogen (BUN)<br>• Calcium (Ca)<br>• Carbon dioxide (COz)<br>• Chloride (Cl)<br>• Creatinine |
| Urinalysis (Dipstick)<br>• Clarity<br>• Bilirubin<br>• Color<br>• Glucose<br>• Ketones<br>• Leukocyte esterase<br>• Nitrate<br>• Occult blood<br>• pH | • Creatine kinase (CK)<br>• Glucose<br>• Lactate dehydrogenase (LDH)<br>• Phosphorus<br>• Potassium (K)<br>• Sodium (Na)<br>• Total and direct bilirubin (TB)<br>• Total protein<br>• Uric acid |

(continued)

| Clinical Laboratory Test | Clinical Laboratory Test |
|---|---|
| • Protein<br>• Specific gravity<br>• Urobilinogen | |
| Urinalysis (Microscopic) - only if urine dipstick abnormal<br>• Bacteria<br>• Casts<br>• Crystals<br>• Epithelial cells<br>• Red blood cells (RBC)<br>• White blood cells (WBC) | Coagulation - ONLY in patients receiving anticoagulant therapy measured only at Visit T1 and 3 to 5 days post Visit T1<br>• Prothrombin time (PT)<br>• International normalized ratio (INR) |
| Other Screening Labs<br>• Hepatitis B surface antigen (HBsAg), hepatitis C virus (HCV), optional reflexive Hepatitis C RNA only if HCV is positive<br>• Serum and urine pregnancy test (only for females of childbearing potential)<br>• Follicle-stimulating hormone (FSH; only for postmenopausal females <55 years old)<br>• Thyroid-stimulating hormone (TSH) | |

Sample Collection, Storage, and Shipping

Central clinical laboratory samples were collected by appropriate clinical site personnel and then shipped according to a separate laboratory manual provided by the Central Laboratory. Samples will be/ were processed by the Central Laboratory.

General Monitoring and Management of Abnormal Clinical Labs

It is the investigator's responsibility to review the results of all laboratory tests as they become available and to sign and date the review. For each laboratory test outside of the laboratory normal range, the investigator needs to ascertain if this is a clinically significant change from baseline for the individual patient, with baseline defined as the last value or observation before the first dose of double-blind IMP. The investigator may repeat the laboratory test or request additional tests to verify the results of the original laboratory test.

If a laboratory value was determined to be an abnormal and a clinically significant change from baseline for the patient, the investigator determined if it qualifies as an AE, and if yes, an appropriate eCRF will be completed. All clinically significant laboratory abnormalities occurring during the study that were not present at baseline were followed and evaluated with additional tests if necessary, until diagnosis of the underlying cause or resolution. Specific monitoring and management guidelines for laboratories of special interest are outlined in the sections below.

Monitoring and Management of Elevated Liver Function Tests

If at any time after randomization a patient experiences a new ALT and/or AST >3 $\times$ ULN, the patient has undergone repeat confirmatory liver function test (LFT) assessment as soon as was reasonably possible, preferably within 3 to 7 days of the laboratory result becoming available.

Repeat LFT assessment includes: 1) measurement of ALT, AST, alkaline phosphatase, total and direct bilirubin, prothrombin time (PT)/international normalized ratio (INR), eosinophil count, CK; 2) history of concomitant medication use; 3) history of exposure to environmental chemical agents, including ethanol; and 4) query for related symptoms. Additionally, further testing such as antihepatitis A virus (total), HBsAg (confirmation of screening measurement), HCV (confirmation of screening measurement), and anti-cytomegalovirus/immunoglobulin M. Epstein-Barr, liver ultrasound or magnetic resonance imaging (MRI) scanning may be warranted to rule out additional pathology depending on clinical presentation and should be discussed with the Sponsor personnel or the authorized Medical Monitor. Although samples

were collected, some repeat LFT parameters may not be measured until elevation is confirmed.

• If repeat LFT assessment confirms ALT and/or AST >3 × ULN but ≤5 × ULN, consideration should be given to administering no further doses of IMP. At the investigator's discretion, IMP may be interrupted and the patient rechallenged with IMP after LFTs have returned to baseline levels.

• If repeat LFT assessment confirms ALT and/or AST >5 × ULN and no alternative reason for elevation is identified, patient should discontinue IMP. At the investigator's discretion, IMP may be interrupted and the patient rechallenged with IMP after LFTs have returned to baseline levels.

• If repeat LFT assessment confirms ALT and/or AST >3 × ULN in addition to any of the following and no alternative reason for elevation is identified, patient should discontinued IMP and be given no further IMP treatment:

- TB >2 × ULN

- INR >1.5 × ULN (unless the patient is on stable dose of anticoagulation medication)

- Appearance or worsening of right upper abdominal discomfort, anorexia, fatigue, nausea, vomiting, fever, rash, or eosinophilia

Monitoring and Management of Elevated Creatine Kinase

If at any time after randomization a patient experiences a marked CK elevation >5 × ULN, the patient will undergo repeat confirmatory assessment as soon as is reasonably possible, preferably within 3 to 7 days of the laboratory result becoming available. If initial CK elevation is >10 × ULN, patients will be instructed to discontinue IMP immediately (instead of continuing IMP until repeat lab value is assessed). It is very important that repeat confirmatory assessment occur as soon as possible (within a day of stopping IMP).

Repeat CK assessment includes query for the nature, duration and intensity of any muscle symptoms; review possible predisposing factors, such as unaccustomed exercise, heavy alcohol intake, viral illness (consider performing serology), concomitant medications, and/or other conditions which can cause myopathy; physical examination for muscle tenderness, weakness, and rash; measure serum creatinine, dipstick urinalysis ± microscopy if indicated; and basic metabolic panel.

- If the repeat CK assessment confirms an unexplained (*i.e.*, not associated with recent trauma or physically strenuous activity) CK abnormality >5 × ULN, if asymptomatic the investigator with input from the Sponsor may consider continuing IMP with continued CK assessments every 1-2 weeks.

- If the repeat CK assessment confirms an unexplained (*i.e.*, not associated with recent trauma or physically strenuous activity) CK abnormality as listed below, the patient should discontinue IMP:

  - >5 × ULN that is associated with symptoms of muscle pain, muscle weakness, or dark urine; or

  - >10 × ULN, even in the absence of symptoms.

- At the investigator's discretion, IMP may be interrupted and the patient rechallenged with IMP after CK has returned to the baseline level.

Monitoring and Management of Elevated LDL-C

Post-randomization, LDL-C results were masked to investigators in order to maintain the blind. The central laboratory will notify the investigator if the patient's LDL-C level is ≥220 mg/dL. The patient returns to the clinic for a repeat fasting blood lipid sample to confirm that the LDL-C value meets the threshold criteria. The patients were counseled on healthy dietary guidelines and reminded to take IMP. If confirmed, the patient will discontinue IMP.

Monitoring and Management of Elevated TG

Post-randomization, TG results will be masked to investigators in order to maintain the blind. The central laboratory will notify the investigator if patient's TG level is >1000 mg/dL. The patients will be counseled on healthy

dietary guidelines and reminded to take IMP. The patient will return to the clinic for a repeat fasting blood lipid sample to confirm that the TG value meets the threshold criteria. If confirmed, the patient will discontinue IMP.

Monitoring and Management of Potential Hypoglycemia and Metabolic Acidosis

Patients will be educated on the signs and symptoms of hypoglycemia. If such signs and symptoms are experienced, patients will be advised to report them to the study site (see Section 13.3 for additional details).

Clinical laboratories will be assessed to determine any signs of anion gap metabolic acidosis. If laboratories are consistent with metabolic acidosis, immediate follow up with the patient for further medical evaluation of the acidosis will occur (see Section 13.3 for additional details). This event should be captured as an AE.

Total Blood Volume of Central Clinical Laboratory Samples

The total number of venipunctures and total volume of whole blood collected during the study will be limited to that needed for safety, efficacy, and biomarker assessment. Total whole blood volume collected over the study duration is not to exceed approximately 250 mL for each patient.

**ADVERSE AND SERIOUS ADVERSE EVENTS**

Adverse Events

*Definition of Adverse Events*

An AE is defined as any untoward medical occurrence in a clinical investigation patient administered a pharmaceutical product, including control, and which does not necessarily have a causal relationship with treatment. The investigator is responsible for ensuring that any AEs observed by the investigator or reported by the patient are recorded in the patient's medical record.

An AE can be:

- Any unfavorable and unintended sign/symptom including an abnormal laboratory finding or disease temporally associated with the use of a medicinal product, whether or not considered related to the medicinal product
- Any new disease or exacerbation of an existing disease
- Laboratory abnormality or diagnostic test abnormalities (e.g., electrocardiogram [ECG] or X-ray) should be reported as an AE if one of the following occurs:
    - Treatment required due to the abnormality
    - Discontinuation of IMP
    - Per Investigator judgment
- TEAEs are defined as AEs that begin or worsen after the first dose of IMP administration as defined in the statistical analysis plan (SAP)

*Adverse Drug Reaction*

All noxious and unintended responses to a medicinal product related to any dose should be considered an adverse drug reaction (ADR). "Responses" to a medicinal product means that a causal relationship between the medicinal product and an AE is at least a reasonable possibility (*i.e.,* the relationship cannot be ruled out).

An unexpected ADR is defined as an adverse reaction, the nature or severity of which is not consistent with the applicable product information (e.g., IB for an unapproved investigational product or package insert/summary of product characteristics for an approved product).

*Reporting for Adverse Events*

All AEs occurring during the course of the study (starting from signing informed consent through 30 days after study completion/discontinuation) will be collected on the AE eCRF. Patients should be instructed to report any AE that they experience to the investigator. Investigators should make an assessment for AEs at each visit and record the event

on the appropriate AE eCRF.

Wherever possible, a specific disease or syndrome rather than individual associated signs and symptoms should be identified by the investigator and recorded on the eCRF. However, if an observed or reported sign or symptom is not considered a component of a specific disease or syndrome by the investigator, it should be recorded as a separate AE on the eCRF. Additionally, the condition that led to a medical or surgical procedure should be recorded as an AE, not the procedure.

Any medical condition already present at screening or baseline should not be reported as an AE unless the medical condition or signs or symptoms present at baseline worsens in severity or seriousness at any time during the study. In this case, it should be reported as an AE.

It is the responsibility of the investigator to assess the clinical significance of all abnormal values as defined by the list of reference ranges from the local laboratory. In some cases, significant changes in lab values within the normal range will require similar judgment.

An abnormal laboratory value that is not already associated with an AE is to be recorded as an AE only if any one of the following criteria is met:

- an action on the study drug is made as a result of the abnormality
- intervention for management of the abnormality is required
- at the discretion of the investigator should the abnormality be deemed clinically significant

Significant abnormal laboratory values occurring during the clinical study will be followed until repeat tests return to normal, stabilize, or are no longer clinically significant. Any abnormal test that is determined to be an error does not require reporting as an AE.

Each AE is to be evaluated for duration, severity, seriousness, and causal relationship to the IMP. For each AE, the following information will be/ were recorded:

- Description of the event (e.g., headache)
- Date of onset
- Date of resolution (or that the event is continuing)
- Action taken as a result of the event
- Seriousness of the event
- Severity of the event
- Outcome of the event
- Investigator’s assessment of relationship to IMP.

A cluster of signs and symptoms that results from a single cause should be/ were reported as a single AE (e.g., fever, elevated white blood cells [WBC], cough, abnormal chest X-ray, etc, can all be reported as "pneumonia").

The investigator has/ will carefully evaluate the comments of the patient and the response to treatment in order that he/she may judge the true nature and severity of the AE. The question of the relationship of AEs to IMP administration should be determined by the investigator or study physician after thorough consideration of all facts that are available.

*Severity*

It is the investigator’s responsibility to assess the intensity (severity) of an AE.

The severity of the AE will be/was characterized as mild, moderate, or severe according to the following definitions:

- Mild: Events are usually transient and do not interfere with the patient’s daily activities
- Moderate: Events introduce a low level of inconvenience or concern to the patient and may interfere with daily activities

- Severe: Events interrupt the patient's usual daily activity, are incapacitating with inability to do usual activities, or significantly affect clinical status and warrant intervention and/or close follow-up

Note: A severe AE need not be serious and an SAE need not, by definition, be severe.

*Relationship*

It is the investigator's responsibility to assess the relationship between the IMP and the AE. The degree of "relatedness" of the AE to the IMP may be described using the following scale:

- Not Related: No temporal association and other etiologies are likely the cause
- Unlikely: While cannot be definitively ruled as not related to IMP, a causal association is remote, and other etiologies are more likely to be the cause. For reporting and summarization, events assessed as Unlikely to be related to IMP will be considered as Not Related to IMP.
- Possible: Temporal association, but other etiologies are likely the cause. However, involvement of the IMP cannot be excluded.
- Probable: Temporal association, other etiologies are possible but unlikely. The event may respond if the IMP is discontinued.
- Definite: Established temporal association with administration of the IMP with no other more probable cause. Typically, the event should resolve when the IMP is discontinued and recur on re-challenge.

*Monitoring and Follow-up of Adverse Events*

Patients having AEs will be/ were monitored with relevant clinical assessments and laboratory tests, as determined by the investigator. All follow-up results were/ are to be reported to the Sponsor personnel or the authorized Medical Monitor. Any actions taken and follow up results must be recorded either on the appropriate page of the eCRF or in appropriate follow-up written correspondence, as well as in the patient's source documentation. Follow-up laboratory results should be filed with the patient's source documentation.

For all AEs that require the patient to be discontinued from the study, relevant clinical assessments and laboratory tests must be repeated at appropriate intervals until final resolution or stabilization of the event(s).

Patients with AEs related to IMP that are ongoing at study discontinuation or completion must be followed until resolution or for 30 days after study completion, whichever comes first, with the exception of patients reporting SAEs (see Section 13.2.2).

*Treatment-Emergent Adverse Events*

TEAE are defined as AEs that begin or worsen after the first dose of IMP administration as defined in the SAP.

Serious Adverse Events

*Definition of Serious Adverse Event*

An SAE is defined as any AE occurring at any dose that results in any of the following outcomes:

- Results in death
- Is life-threatening
- Requires in-patient hospitalization or prolongation of existing hospitalization
- Results in persistent or significant disability/incapacity, or substantial disruption of the ability to conduct normal life functions
- Is a congenital anomaly/birth defect
- An important medical event

NOTE: An emergency room visit without hospital admission does not meet inpatient hospitalization criterion,

nor does hospitalization for an elective or outpatient procedure scheduled or planned before signing of informed consent. However, unexpected complications and/or prolongation of hospitalization that occur during elective or outpatient surgery should be recorded as AEs and assessed for seriousness. Admission to the hospital for social or situational reasons (e.g., no place to stay, live too far away to come for hospital visits) will not be considered inpatient hospitalizations.

Important medical events that may not result in death, be life threatening, or require hospitalization may be considered an SAE when, based upon appropriate medical judgment, they may jeopardize the patient and may require medical or surgical intervention to prevent one of the outcomes listed in this definition. Examples of such medical events include allergic bronchospasm requiring intensive treatment in an emergency room, blood dyscrasias, convulsions that do not result in inpatient hospitalization, or the development of drug dependency or drug abuse.

Adverse Events of Special Interest

Adverse events of special interest (AESI) are included in this protocol for reasons either associated with other lipid lowering therapies or where deemed important based on nonclinical data. AESI for this protocol include: metabolic acidosis (clinical laboratories), hypoglycemia, muscular (AE and CK evaluation), hepatic, and neurocognitive/neurologic events.

Hypoglycemia and metabolic acidosis: This has led to monkey deaths in preclinical studies. Exposures in these studies were ≥17 times higher than those associated with the bempedoic acid 180-mg dose in Phase 1 and 2 clinical studies; the 180-mg dose will be the dose studied in Phase 3. Subsequent monkey studies proved that hypoglycemia was reversible with discontinuation of dosing and oral glucose administration. No cases of either hypoglycemia or metabolic acidosis have been identified in Phase 1 or 2 clinical trials to date. All patients enrolled in bempedoic acid clinical trials will be monitored by standard clinical chemistries for any potential risk of metabolic acidosis with appropriate follow-up if detected. Patients in all clinical trials will be provided with information (in the ICD) regarding the signs and symptoms of hypoglycemia including lightheadedness, shakiness, shaking of the hands, sweating, tingling, blurred visions, nausea or vomiting, mental confusion, difficulty concentrating and drowsiness. The investigative site will be required to specifically capture the reporting of these symptoms.

Muscle: Muscle events have been associated with statins (Thompson 2003) and other lipid-lowering therapies and are mentioned in the product information for these therapies. Muscle symptoms through AE review, CK elevations, and symptoms of potential myopathy will be closely monitored.

Hepatic: Hepatic function will be monitored throughout with the clinical safety labs. More detailed investigation will occur if the safety clinical laboratory results are >3 X ULN.

Neurocognitive events: Theoretically, it is possible that lipid-lowering agents that disrupt cholesterol homeostasis in the brain could impact neurological function, and there have been reports of cognitive impairment (e.g., memory loss) associated with the use of statin drugs (FDA 2012). Summarization of events will occur using prespecified Medical Dictionary for Regulatory Activities (MedDRA) terms outlined in the SAP.

**STATISTICS**

General Considerations

The statistical analyses described in this section will be performed as further outlined in the SAP, which will be finalized prior to the end of the study. The SAP will supersede the protocol if there are any differences between the 2 documents in the plans for data analysis. The SAP will be included as an appendix in the clinical study report for this protocol.

In general, summary statistics for continuous variables will include the number of subjects, mean, median, standard deviation or standard error, first and third quartiles, minimum, and maximum. For categorical variables, the frequency and percentage will be given.

Determination of Sample Size

The sample size of 40 randomized patients in the triplet therapy group and 20 randomized patients in the placebo group is expected to provide 90% power to detect a difference of 25% in the percent change from baseline to Week 6 in calculated LDL-C between the triplet therapy group and the placebo group. This calculation is based on a 2-sided t-test at the 5% level of significance and a common standard deviation of 26% and a dropout rate of 10%. The conservative but clinical meaningful treatment difference of 25% is chosen in order to have a reasonable sample size to assess other efficacy and safety information.

## Analysis Populations

The modified Intent-to-Treat (mITT) population, used for all of the efficacy analyses, is defined as all randomized patients who received at least 1 dose of IMP and have a baseline assessment and at least 1 post baseline assessment, excluding any assessment taken more than 2 days after a dose of IMP.
The Safety Population (SP), used for all of the safety summaries, is defined as all randomized patients who received at least 1 dose of study medication. Patients in the SP will be included in the treatment group that they actually received, regardless of their randomized treatment.

## Disposition, Demographics, and Baseline Characteristics

Disposition, including reason for withdrawal from the IMP and study, will be summarized by treatment group. Demographic information and patient characteristics including, but not limited to, gender, race, age, and baseline vital signs will also be summarized by treatment group.

## Primary Endpoint Analysis

The primary efficacy endpoint is the percent change from baseline to Week 6 in LDL-C. Baseline is defined as the mean of the values from Week -1 (Visit S2) and predose Day 1/Week 0 (Visit T1). An analysis of covariance (ANCOVA) with treatment group as factor and baseline LDL-C as covariate will be performed to compare triplet therapy versus placebo for the primary endpoint using the mITT population (Section 14.3). Missing values at Week 6 will be imputed using the last observation carried forward (LOCF) procedure (only post baseline values will be carried forward). The least squares mean (LSM) and standard error (SE) will be provided for both treatment groups, along with the placebo-corrected LSM, its 95% confidence interval (CI), and associated p-value.

## Secondary Efficacy Endpoint Analyses

Secondary efficacy endpoints, which include the percent change from baseline to Week 6 in additional lipid and cardiometabolic biomarkers, will be analyzed in a similar manner as the primary efficacy endpoint. Baseline for non-HDL C, HDL-C, TC, and TG is defined as the mean of the values from Week -1 (Visit S2) and predose Day 1/Week 0 (Visit T1), while baseline for ApoB and hs-CRP is defined as the predose Day 1/Week 0 (Visit T1) value.
Statistical testing of primary and secondary efficacy endpoints will be 2-sided and conducted at the 5% level of significance with no adjustment for multiple comparisons.

## Safety Endpoints

Descriptive summary will be provided for safety endpoints.
The MedDRA will be used to code all AE to a system organ class (SOC) and a preferred term.
The summarization of AEs will include only TEAEs defined as AEs that begin or worsen after first dose of IMP administration as defined in the SAP. All TEAEs, SAEs, AEs leading to withdrawal of IMP, fatal AEs and AEs of special interest will be summarized by SOC and preferred term in descending order of frequency by treatment group.
Clinical safety laboratories, including hematology, blood chemistry, glucose, and urinalysis; PE findings; vital signs; and weight will be summarized by the value and by change or percent change from baseline in the value (where appropriate) at each post baseline time point.

## Ethical Conduct of the Study

The investigator agrees, when signing the protocol, to conduct the study in accordance with ethical principles that have their origin in the current revision of the Declaration of Helsinki and are consistent with GCP, applicable regulatory requirements, and policies and procedures as outlined by the ethical requirements for IRB review and ICDs.
The investigator agrees to allow monitoring and auditing of all essential clinical study documents by the Sponsor or its authorized representatives and inspection by the FDA. Monitoring and auditing visits by the Sponsor or authorized designee will be scheduled with the appropriate staff at mutually agreeable times periodically throughout the study.
The investigator will assure proper implementation and conduct of the study, including those study-related duties delegated to other appropriately qualified individuals. The investigator will assure that study staff cooperates with monitoring and audits, and will demonstrate due diligence in recruiting and screening study patients. The investigator must sign and return to the Sponsor the "Investigator's Signature" page (see Appendix 3) and provide a copy of current curriculum vitae. For this study and all studies conducted under an IND, the investigator must sign and return a completed

Form FDA 1572 "Statement of Investigator" to the Sponsor (or designee).

Written Informed Consent

The Principal Investigator(s) at each center will ensure that the patient is given full and adequate oral and written information about the nature, purpose, possible risk and benefit of the study. Patients must also clearly understand that they are free to discontinue from the study at any time. The patient should be given the opportunity to ask questions and allowed time to consider the information provided.
The patient's signed and dated informed consent must be obtained before conducting any study procedures on the Sponsor agreed ICD. Updates to the ICD during the conduct of the study will be communicated by written letter from the Sponsor to the investigator. The ICD should be provided in the appropriate language of the patient population.
The Principal Investigator(s) must maintain the original, signed ICD. A copy of the signed ICD must be given to the patient.

Patient Confidentiality

The investigator must ensure that the patient's confidentiality is maintained.
The names and identities of all research patients will be kept in strict confidence and will not appear on eCRFs or other records provided to or retained by the
Sponsor (or designee). If a patient's name appears on any document, it must be redacted and replaced with the patient identifier before a copy of the document is supplied to the Sponsor (or designee). The ICD must include appropriate statements explaining that patient data will be confidential and what actions will be taken to ensure patient confidentiality.
Any other confidentiality requirements specified by the site, IRB, or local regulations will be adhered to and detailed appropriately in the ICD.

**APPENDIX 1. SCHEDULE OF EVENTS**

| **Schedule of Events** | | | | | |
|---|---|---|---|---|---|
| **Visit** | **S1** | **S2[1]** | **T1** | **T2** | **T4** |
| **Week** | **-6** | **-1** | **0** | **3** | **6/EO** |
| **Procedure** | **Day -42** | **Day -10** | **D** | **Day 22 ± 3** | **Day 43 ± 3** |
| Informed Consent | X | | | | |
| Enrollment Criteria | X | X | X | | |
| Demographics | X | | | | |
| Medical History | X | | | | |
| Concomitant\Prohibited | X | X | X | X | X |
| Adverse Event Recording | | X | X | X | X |
| Physical Exam | | | X | | X |
| Weight[3] | X | | X | | X |
| Height/BMI | X | | | | |
| Vital Signs[4] | X | X | X | X | X |
| Serology[5] | | X | | | |
| Serum Pregnancy/FSH[6] | X | | | | |
| Urine Pregnancy[6] | | | X | | |
| TSH | X | | | | |

(continued)

| Schedule of Events | | | | | |
|---|---|---|---|---|---|
| **Visit** | **S1** | **S2**[1] | **T1** | **T2** | **T4** |
| **Week** | **-6** | **-1** | **0** | **3** | **6/EO** |
| **Procedure** | **Day -42** | **Day -10** | **D** | **Day 22 ± 3** | **Day 43 ± 3** |
| Clinical Safety Labs[7] | X | | X | X | X |
| Basic Fasting Lipids[8] | X | X | X | X | X |
| Special Fasting Lipids and Other Biomarkers[9] | | | X | | X |
| Dietary and Lifestyle | X | X | X | X | |
| Randomization | | | X | | |
| Double Blind IMP Dispensing | | | X | X | |
| IMP Return | | | | X | X |

BMI = body mass index; FSH = follicle-stimulating hormone, TSH = thyroid-stimulating hormone, EOS = End of Study, ET = Early Termination.

[1] An optional visit approximately 1 week later MAY be completed if patient fails to meet a lipid entry criterion. If this optional visit is completed, the mean of the LDL-C values, or TG values, will be used to determine eligibility.

[2] All procedures will be completed at end of study or early termination.

[3] Body weight will be measured in the morning while fasting, using consistent scales, after voiding, and without shoes and outerwear (*e.g.*, coats).

[4] Vital signs will include SBP, DBP, and heart rate, and will be collected prior to any blood sample collection. Patient will rest for 5 minutes prior to assessments.

[5] Serology for Hep B antigen, Hep C antibody.

[6] FSH completed in appropriate postmenopausal women only; pregnancy test completed in non-postmenopausal women only.

[7] Clinical safety labs include hematology, blood chemistry, and urinalysis. Coagulation panel ONLY in patients receiving anticoagulants measured only at T1 and repeat 3-5 days after starting IMP.

[8] Basic fasting lipids include total cholesterol, calculated LDL-C, HDL-C, non-HDL-C, and triglycerides.

[9] Includes ApoB and hs-CRP

**EXAMPLE 2: CLINICAL DATA**

**Brief description of clinical study and results.**

Potential Results Scenarios

| Scenario | LDL-C | hsCRP | % Patients LDL-C reduction ≥50% | Safety | Tolerability | Muscle Pain |
|---|---|---|---|---|---|---|
| Upside | Greater than 70% lowering | Greater than 40% lowering | Greater than 75% | No LFT 3xULN or CK elevations (repeated and confirmed) | Same as placebo | Same as placebo |
| Base Case | 60-70% lowering | 25-40% lowering | 50-75% | 1% LFT elevations | Slightly worse than placebo | Same as placebo |
| Downside | Less than 60% lowering | Less than 25% lowering. Any change not SS | Less than 50% | Greater than 2% LFT and CK elevations | Worse than placebo | Worse than placebo with discontinuations |

Mean Baseline LDL-C = 155 mg/dL (Range 122-194); Median Baseline hsCRP mg/dL = 1.78 (Range 0.12-29.16)

Demographics & Baseline Characteristics

| | PLACEBO N=20 | BA+EZE+ATORVA N=41 |
|---|---|---|
| *Demographics* | | |
| Age (years) | 61 ± 7.8 | 61 ± 12.5 |
| Gender (M/F, % Female) | 6M/14F, 70% | 16M/25F, 61% |
| White (n, %) | 16, 80% | 33, 81% |
| *Baseline Characteristics* | | |
| BMI ($kg/m^2$) | 28 ± 5.9 | 29 ± 6.4 |
| LDL-C (mg/dL) | 156 ± 14 | 154 ± 18 |
| Total Cholesterol (mg/dL) | 235 ± 21 | 236 ± 28 |
| HDL-C (mg/dL) | 52 ± 13 | 52 ± 13 |
| non-HDL-C (mg/dL) | 183 ± 18 | 184 ± 28 |
| apoB (mg/dL) | 119 ± 15 | 119 ± 20 |
| hsCRP (mg/L)[b] | 1.64 | 1.94 |
| Triglycerides (mg/dL) | 140 ± 62 | 153 ± 83 |

[a] Unless otherwise indicated

[b] Median Values

mITT Population

LDL-C Percent Change from Baseline after 6 weeks of dosing of bempedoic acid 180mg, ezetimibe 10 mg, and atorvastatin 20 mg

| Treatment Group | N | LDL-C mg/dL, Mean (SD) | | % Change from Baseline | | |
|---|---|---|---|---|---|---|
| | | Baseline | Week 6 | LS Mean (SE) | PBO Adjusted LS Mean (SE) | P-value |
| **PLACEBO** | 20 | 156 (14) | 152 (27) | **-3% (3.3)** | - | - |
| **BA+EZE+ATORVA** | 41 | 154 (18) | 56 (17) | **-64% (1.7)** | **-61% (3.7)** | **<0.001** |

MITT Population (LOCF)

Other Secondary Lipids: HDL-C and TG Change from Baseline after 6 weeks of dosing of bempedoic acid 180mg, ezetimibe 10 mg, and atorvastatin 20 mg

| Parameter / Treatment Group | N | Baseline Mean (SD) | Week 6 Endpoint Mean (SD) | Percent Change from Baseline | |
|---|---|---|---|---|---|
| | | | | LS Mean (SE) | P Value vs Placebo |
| *HDL-C (mg/dL)* | | | | | |
| PLACEBO | 20 | 52 (13) | 52 (13) | 0 (2.26) | |
| BA+EZE+ATORVA | 41 | 52 (13) | 51 (15) | -1 (1.47) | p=0.588 |
| *Triglycerides (mg/dL)* | | | | | |
| PLACEBO | 20 | 140 (62) | 151 (70) | 9 (5.95) | |
| BA+EZE+ATORVA | 41 | 153 (83) | 103 (48) | -27 (2.79) | p<0.001 |

Safety & Tolerability: Overview of Adverse Events

| Treatment Emergent Adverse Events (AEs) | Number (%) of Patients PLACEBO N=20 | BA+EZE+ATORVA N=43 |
|---|---|---|
| Overview of AEs in All Patients (patient incidence) | | |
| Any AE(s) | 7 (35%) | 15 (35%) |
| Serious AE(s) | 0 | 0 |
| Total Related AE(s) | 2 (10%) | 8 (18.6%) |
| Study Drug Discontinuation due to AE(s) | 1 (5%) | 3 (7%) |
| Common AEs (≥ 3% of patients/group) by MedDRA Preferred Term | | |
| Muscle spasms | 2 (10%) | 1 (2.3%) |
| Headache | 1 (5%) | 2 (4.7%) |
| Arthralgia | 1 (5%) | 1 (2.3%) |
| Back pain | 1 (5%) | 1 (2.3%) |
| Dizziness | 1 (5%) | 1 (2.3%) |
| Nausea | 1 (5%) | 1 (2.3%) |
| Arthropod bite | 1 (5%) | 0 |
| Depression | 1 (5%) | 0 |
| Ear pain | 1 (5%) | 0 |
| Fall | 1 (5%) | 0 |
| Folliculitis | 1 (5%) | 0 |
| Joint swelling | 1 (5%) | 0 |
| Melanocytic naevus | 1 (5%) | 0 |
| Musculoskeletal chest pain | 1 (5%) | 0 |
| Diarrhea | 0 | 2 (4.7%) |
| Fatigue | 0 | 2 (4.7%) |
| Hepatic enzyme increased | 0 | 2 (4.7%) |
| Osteoarthritis | 0 | 2 (4.7%) |
| Pain in extremity | 0 | 2 (4.7%) |
| Rash | 0 | 2 (4.7%) |

Overview of Adverse Events Leading to Discontinuations

| Treatment Emergent Adverse Events (AEs) | Number (%) of Patients PLACEBO N=20 | BA+EZE+ATORVA N=43 |
|---|---|---|
| Overview of AEs in All Patients (patient incidence) | | |
| Any AE(s) | 7 (35%) | 15 (35%) |
| Serious AE(s) | 0 | 0 |
| Total Related AE(s) | 2 (10%) | 8 (18.6%) |
| Study Drug Discontinuation due to AE(s) | 1 (5%) | 3 (7%) |
| AEs Leading to Discontinuation by MedDRA Preferred Term | | |
| Back Pain | 1 (5%) | 0 |
| Depression | 1 (5%) | 0 |

| | | |
|---|---|---|
| Headache | 1 (5%) | 0 |
| Rash | 0 | 2 (4.7%) |
| Abdominal pain | 0 | 1 (2.3%) |
| Arthralgia | 0 | 1 (2.3%) |
| Dizziness | 0 | 1 (2.3%) |
| Fatigue | 0 | 1 (2.3%) |
| Myalgia | 0 | 1 (2.3%) |
| Nausea | 0 | 1 (2.3%) |
| Sinusitis | 0 | 1 (2.3%) |
| Tension headache | 0 | 1 (2.3%) |
| Urticaria | 0 | 1 (2.3%) |

Overview of Related Adverse Events

| Treatment Emergent Adverse Events (AEs) | Number (%) of Patients | |
|---|---|---|
| | PLACEBO N=20 | BA+EZE+ATORVA N=43 |
| Overview of AEs in All Patients (patient incidence) | | |
| Any AE(s) | 7 (35%) | 15 (35%) |
| Serious AE(s) | 0 | 0 |
| Total Related AE(s) | 2 (10%) | 8 (18.6%) |
| Study Drug Discontinuation due to AE(s) | 1 (5%) | 3 (7%) |
| Related AEs by MedDRA Preferred Term | | |
| Headache | 1 (5%) | 1 (2.3%) |
| Back pain | 1 (5%) | 0 |
| Depression | 1 (5%) | 0 |
| Muscle Spasms | 1 (5%) | 0 |
| Hepatic enzyme increased | 0 | 2 (4.7%) |
| Rash | 0 | 2 (4.7%) |
| Abdominal pain | 0 | 1 (2.3%) |
| Arthralgia | 0 | 1 (2.3%) |
| Constipation | 0 | 1 (2.3%) |
| Diarrhoea | 0 | 1 (2.3%) |
| Dizziness | 0 | 1 (2.3%) |
| Dizziness postural | 0 | 1 (2.3%) |
| Dyspepsia | 0 | 1 (2.3%) |
| Fatigue | 0 | 1 (2.3%) |
| Hypoglycaemia | 0 | 1 (2.3%) |
| Liver function test increased | 0 | 1 (2.3%) |
| Myalgia | 0 | 1 (2.3%) |
| Nausea | 0 | 1 (2.3%) |
| Pain in extremity | 0 | 1 (2.3%) |
| Sinusitis | 0 | 1 (2.3%) |
| Tension Headache | 0 | 1 (2.3%) |
| Urticaria | 0 | 1 (2.3%) |

No LFT or CK Changes During Treatment

| Lab Abnormality (Repeated and Confirmed) | Number (%) of Patients | |
|---|---|---|
| | PLACEBO N=20 | BA+EZE+ATORVA N=43 |

| | | |
|---|---|---|
| ALT/AST > 3 x ULN | 0 | 0 |
| CK > 5 x ULN | 0 | 0 |

Muscle Pain Reported During Treatment

| Potential Muscle TEAEs | Number (%) of Patients | |
|---|---|---|
| | PLACEBO N=20 | BA+EZE+ATORVA N=43 |
| Overview of Potential Muscle TEAEs in All Patients (patient incidence) | | |
| Any Potential Muscle AE(s) | 6 (30%) | 7 (16.3%) |
| Related Potential Muscle AE(s) | 2 (10%) | 2 (4.7%) |
| Discontinuation due to Potential Muscle AE(s) | 1 (5%) | 1 (2.3%) |
| All Potential Muscle AEs by MedDRA Preferred Term | | |
| Muscle spasms | 2 (10%) | 1 (2.3%) |
| Arthralgia | 1 (5%) | 1 (2.3%) |
| Back pain | 1 (5%) | 1 (2.3%) |
| Joint swelling | 1 (5%) | 0 |
| Musculoskeletal chest pain | 1 (5%) | 0 |
| Osteoarthritis | 0 | 2 (4.7%) |
| Pain in extremity | 0 | 2 (4.7%) |
| Arthritis | 0 | 1 (2.3%) |
| Myalgia | 0 | 1 (2.3%) |

| Potential Muscle TEAEs | Number (%) of Patients | |
|---|---|---|
| | PLACEBO N=20 | BA+EZE+ATORVA N=43 |
| Overview of Potential Muscle TEAEs in All Patients (patient incidence) | | |
| Any Potential Muscle AE(s) | 6 (30%) | 7 (16.3%) |
| Related Potential Muscle AE(s) | 2 (10%) | 2 (4.7%) |
| Discontinuation due to Potential Muscle AE(s) | 1 (5%) | 1 (2.3%) |
| Potential Muscle AEs Leading to Discontinuation by MedDRA Preferred Term | | |
| Back Pain | 1 (5%) | 0 |
| Arthralgia | 0 | 1 (2.3%) |
| Myalgia | 0 | 1 (2.3%) |

SUMMARY

- Bempedoic acid 180mg, ezetimibe 10 mg, and atorvastatin 20 mg:

  · Significantly lowered LDL-C by 64% compared to baseline (placebo-adjusted 61%), p<0.001 · Significantly

reduced hsCRP by 48%, $p<0.001$

· Significantly lowered total cholesterol, non-HDL-C and apoB

· 95% of patients had an LDL-C reduction of > 50%

· Well tolerated with safety profile comparable to placebo

Potential Results Scenarios Summary

| Scenario | LDL-C | hsCRP | % Patients LDL-C reduction ≥50% | Safety | Tolerability | Muscle Pain |
|---|---|---|---|---|---|---|
| Upside | Greater than 70% lowering | Greater than 40% lowering | Greater than 75% | No LFT 3XULN or CK elevations (repeated and confirmed) | Same as placebo | Same as placebo |
| Base Case | 60-70% lowering | 25-40% lowering | 50-75% | 1% LFT elevations | Slightly worse than placebo | Same as placebo |
| Downside | Less than 60% lowering | Less than 25% lowering; Any change not SS | Less than 50% | Greater than 2% LFT and CK elevations | Worse than placebo | Worse than placebo with discontinuations |

Mean Baseline LDL-C = 155 mg/dL (Range 122-194); Median Baseline hsCRP mg/dL = 1.78 (Range 0.12-29.16)

CONCLUSIONS

· Bempedoic acid plus ezetimibe plus atorvastatin 20 mg significantly lowered both LDL-C and hsCRP. The effect on LDL-C was consistent across all patients treated.

· The data from Study 1002-038 demonstrate that the use of bempedoic acid plus ezetimibe plus atorvastatin 20 mg achieved optimal tolerability and safety.

· Bempedoic acid and ezetimibe complemented the LDL-C lowering efficacy of atorvastatin 20 mg, the most highly prescribed statin and dose.

· Oral combination therapies such as the regimen evaluated in this study can potentially meet the needs of the vast majority of patients with elevated LDL-C with oral, once-daily therapies, providing physicians with more oral options and flexibility and payers with pricing they desire in today's constrained healthcare environment.

While the disclosure has been particularly shown and described with reference to a preferred embodiment and various alternate embodiments, it will be understood by persons skilled in the relevant art that various changes in form and details can be made therein without departing from the spirit and scope of the disclosure.

All references, issued patents and patent applications cited within the body of the instant specification are hereby incorporated by reference in their entirety, for all purposes.

**REFERENCES CITED**

Baigent C, Blackwell L, Emberson J, et al. Cholesterol Treatment Trialists' (CTT) Collaboration. Efficacy and safety

of more intensive lowering of LDL cholesterol: a meta-analysis of data from 170 000 participants in 26 randomized trials. Lancet. 2010;376:1670-81.

Cohen JD, Brinton EA, Ito MK, Jacobson TA. Understanding statin use in America and gaps in patient education (USAGE): an internet-based survey of 10,138 current and former statin users. Clinical Lipidol. 2012;6:208-15.

Egan A and Colman E. Weighing the benefits of high-dose simvastatin against the risk of myopathy. N Engl J Med. 2011;365:285-7.

Food and Drug Administration (2012). FDA announces safety changes in labeling for some cholesterol-lowering drugs [Press release]. Retrieved from http://www.fda.gov/NewsEvents/Newsroom/PressAnnouncements/ucm293623.htm

Goldberg AC. Clinical trial experience with extended-release niacin (Niaspan): dose-escalation study. Am J Cardiol. 1998;82:35U-8U.

Grundy SM, Cleeman JI, Merz CN, Brewer HB, Clark LT, Hunninghake DB, et al. Implications of recent clinical trials for the National Cholesterol Education Program Adult Treatment Panel III Guidelines. Circulation. 2004;110:227-39.

Insull W, Toth P, Mullican W, Hunninghake D, Burke S, Donovan J, et al. Effectiveness of colesevelam hydrochloride in decreasing LDL cholesterol in patients with primary hypercholesterolemia: a 24-week randomized controlled trial. Mayo Clin Proc. 2001;76:971-82.

Kathiresan S, Melander O, Anevski D, Guiducci C, Burtt NP, Roos C, et al. Polymorphisms associated with cholesterol and risk of cardiovascular events. New Engl J Med. 2008:358;12:1240-9.

Knopp RH, Brown WV, Dujovne CA, Farquhar JW, Feldman EB, Goldberg AC, et al. Effects of fenofibrate on plasma lipoproteins in hypercholesterolemia and combined hyperlipidemia. Am J Med 1987;83:50-9.

Knopp RH, Dujovne CA, LeBeautA, Lipka LJ, Suresh R, Veltri EP, et al. Evaluation of the efficacy, safety and tolerability of ezetimibe in primary hypercholesterolemia: a pooled analysis from two controlled phase III clinical studies. Int J Clin Pract 2003;57:363-8.

Martin SS, Gosch K, Kulkarni KR, Spertus JA, Mathews R, Ho PM, et al. Modifiable factors associated with failure to attain low-density lipoprotein cholesterol goal at 6 months after acute myocardial infarction. Am Heart J. 2013;165:26-33.e3.

Mozfrarian D, Benjamin EJ, Go AS, Arnett DK, Blaha MJ, Cushman M, et al, for the writing group Members. Heart Disease and Stroke Statistics-2015 Update. A Report from the American Heart Association. Circ. 2015;131:e29-322.

Nichols M, Townsend N, Scarborough P, Rayner M. Cardiovascular disease in Europe 2014: epidemiological update. Eur Heart J. 2014; 35:2950-9.

Robinson JG, Smith B, Maheshwari N, Schrott H. Pleiotropic effects of statins: benefit beyond cholesterol reduction: A meta-regression analysis. J Am Coll Cardiol. 2005 Nov 15;46(10):1855-62. Epub 2005 Oct 24.

Sharrett AR, Ballantyne CM, Coady SA, Heiss G, Sorlie PD, Catellier D, et al. Atherosclerosis Risk in Communities Study Group. Coronary heart disease prediction from lipoprotein cholesterol levels, triglycerides, lipoprotein(a), apolipoproteins A-I and B, and HDL density subfractions. The Atherosclerosis Risk in Communities (ARIC) Study. Circulation. 2001;104:1108-13.

Silverman MG, Ference BA, Im K, Wiviott SD, Giugliano RP, Grundy SM, et al. Association between lowering LDL-C and cardiovascular risk reduction among different therapeutic interventions: a systematic review and meta-analysis. JAMA. 2016;316(12):1289-97.

Stamler J, Wentworth D, Neaton J. Is relationship between serum cholesterol and risk for premature death from coronary heart disease continuous and graded? JAMA. 1986;256(20):823-8.

Stone NJ, Robinson J, Lichtenstein AH, et al. for the Heart Association Task Force on Practice Guidelines Cardiovascular Risk in Adults. 2013 ACC/AHA Guideline on the Treatment of Blood Cholesterol to Reduce Atherosclerotic Cardiovascular Risk in Adults. Circ, DOI: 10.1161/01.cir.0000437738.63853.7a

Townsend N, Nichols M, Scarborough P, Rayner M. Cardiovascular disease in Europe - epidemiological update 2015. Eur Heart J. 2015;36(40):2696-705.

Thompson PD, Clarkson P, Karas RH. Statin-associated myopathy. JAMA. 2003:289:1681-90.

Virani SS, Woodard LD, Chitwood SS, Landrum CR, Urech TH, Wang D, et al. Frequency and correlates of treatment intensification for elevated cholesterol levels in patients with cardiovascular disease. Am Heart J. 2011;162:725-732.e1.

Waters DD. What the statin trials have taught us. Am J Cardiol. 2006;98: 129-34.

World Health Organization (WHO) Fact Sheet No 317 Updated January 2015.

-The following numbered paragraphs clauses contain further statements of various aspects of the present invention:

1. A method of treating or reducing the risk of cardiovascular disease in a subject in need thereof, the method comprising administering:

   ETC-1002;
   Ezetimibe;
   a statin; and
   one or more pharmaceutical excipients or carriers to the subject,
   wherein the administration treats or reduces the risk of cardiovascular disease in the subject.

2. The method of clause 1, wherein the administered Ezetimibe dose is constant for at least one (1) week.

3. The method of clause 1, wherein the administered ETC-1002 dose is constant for at least one (1) week.

4. The method of clause 1, wherein the administered statin dose is constant for at least one (1) week.

5. The method of clause 1, wherein the administered doses of statin; Ezetimibe; and ETC-1002 are each constant for at least one (1) week.

6. The method of clause 1, wherein the administered doses of statin; Ezetimibe; and ETC-1002 are each constant for at least three (3) weeks.

7. The method of any one of clauses 1-6, wherein the amount of ETC-1002 per administered dose is 180 mg or 120 mg.

8. The method of any one of clauses 1-6, wherein the amount of ETC-1002 per administered dose is between 40 mg to 90 mg.

9. The method of any one of clauses 1-8, wherein the amount of Ezetimibe per administered dose is 10 mg.

10. The method of any one of clauses 1-9, wherein the amount of the statin per administered dose is between 1 mg to 80 mg.

11. The method of any one of clauses 1-10, wherein the administration comprises administering a fixed-dose combination formulation of:

    ETC-1002;
    Ezetimibe;
    a statin; and
    one or more pharmaceutical excipients or carriers to the subject.

12. The method of any one of clauses 1-11, wherein the level of low-density lipoprotein cholesterol (LDL-C) in the subject is below that of a matched control subject receiving the same dose of the statin and the Ezetimibe but not receiving ETC-1002.

13. The method of clause 12, wherein the level of low-density lipoprotein cholesterol (LDL-C) in the subject is reduced 64% relative to baseline LDL-C level in the subject.

14. The method of any one of clauses 1-13, wherein the level of very low density lipoprotein (VLDL) in the subject is below that of a matched control subject receiving the same dose of the statin and the Ezetimibe but not receiving ETC-1002.

15. The method of any one of clauses 1-14, wherein the number of VLDL particles in the subject is below that of a matched control subject receiving the same dose of the statin and the Ezetimibe but not receiving ETC-1002.

16. The method of any one of clauses 1-15, wherein the size of VLDL particles in the subject is below that of a matched control subject receiving the same dose of the statin and the Ezetimibe but not receiving ETC-1002.

17. The method of any one of clauses 1-16, wherein the level of apolipoprotein A-1 (ApoA1) in the subject is greater than that of a matched control subject receiving the same dose of the statin and the Ezetimibe but not receiving ETC-1002.

18. The method of any one of clauses 1-17, wherein the ratio of apolipoprotein B (ApoB) to apolipoprotein A-1 (ApoA1) in the subject is below that of a matched control subject receiving the same dose of the statin and the Ezetimibe but not receiving ETC-1002.

19. The method of any one of clauses 1-17, wherein the ratio of apolipoprotein B (ApoB) to apolipoprotein A-1 (ApoA1) in the subject is not significantly changed from that of a matched control subject receiving the same dose of the statin and the Ezetimibe but not receiving ETC-1002.

20. The method of any one of clauses 1-16, wherein the ratio of apolipoprotein B (ApoB) to apolipoprotein A-1 (ApoA1) in the subject is increased from that of a matched control subject receiving the same dose of the statin and the Ezetimibe but not receiving ETC-1002.

21. The method of any one of clauses 1-16, wherein the level of apolipoprotein B (ApoB) in the subject is reduced 54% relative to baseline ApoB level in the subject.

22. The method of any one of clauses 1-21, wherein the level of triglycerides (TG) in the subject is below that of a matched control subject receiving the same dose of the statin and the Ezetimibe but not receiving ETC-1002.

23. The method of any one of clauses 1-22, wherein the level of triglycerides (TG) in the subject is reduced 27% relative to baseline TG level in the subject.

24. The method of any one of clauses 1-23, wherein the level of total cholesterol (TC) in the subject is below that of a matched control subject receiving the same dose of the statin and the Ezetimibe but not receiving ETC-1002.

25. The method of any one of clauses 1-24, wherein the level of total cholesterol (TC) in the subject is reduced 47% relative to baseline TC level in the subject.

26. The method of any one of clauses 1-25, wherein the level of high-sensitivity C-reactive protein (hs-CRP) in the subject is below that or not changed from that of a matched control subject receiving the same dose of the statin and the Ezetimibe but not receiving ETC-1002.

27. The method of any one of clauses 1-26, wherein the level of high-sensitivity C-reactive protein (hs-CRP) in the subject is reduced 54% relative to baseline.

28. The method of any one of clauses 1-27,wherein the level of non-HDL-C in the subject is below that of a matched control subject receiving the same dose of the statin and the Ezetimibe but not receiving ETC-1002.

29. The method of any one of clauses 1-28, wherein the level of non-HDL-C in the subject is reduced 60% relative to baseline non-HDL-C level in the subject.

30. The method of any one of clauses 1-29, wherein the subject is statin-intolerant.

31. The method of any one of clauses 1-30, wherein the subject suffers from muscle-related adverse effects associated with statin use.

32. The method of any one of clauses 1-31, wherein the subject has hypercholesterolemia.

33. The method of any one of clauses 1-32, wherein the subject has hyperlipidemia.

34. The method of any one of clauses 1-33, wherein the subject has dyslipidemia or mixed dyslipidemia.

35. The method of any one of clauses 1-34, wherein the subject has atherosclerotic cardiovascular disease.

36. The method of any one of clauses 1-35, wherein the subject is human.

37. The method of any one of clauses 1-36, wherein the ETC-1002, the Ezetimibe, and the statin are administered in a single pharmaceutical formulation.

38. The method of any one of clauses 1-37, wherein the statin is atorvastatin.

39. The method of any one of clauses 1-38, wherein the amount of ETC-1002 is 180 mg, the amount of Ezetimibe is 10 mg.

40. The method of any one of clauses 1-41, wherein the method lowers LDL-C level 50% compared to the baseline LDL-C level in the subject after six (6) weeks of administration.

41. A pharmaceutical formulation comprising:

ETC-1002;
Ezetimibe;
a statin; and
one or more pharmaceutical excipients or carriers.

42. The composition of clause 41, wherein the amount of ETC-1002 is 180 or 120 mg.

43. The composition of clause 41, wherein the amount of ETC-1002 is between 40 mg to 90 mg.

44. The composition of clause 41, wherein the amount of Ezetimibe is 10 mg.

45. The composition of clause 41, wherein the amount of the statin is between 1 mg to 80 mg.

46. The composition of any one of clauses 41-45, wherein the statin is selected from the group consisting of: atorvastatin, simvastatin, fluvastatin, resuvastatin, and pitavastatin.

47. The composition of any one of clauses 41-45, wherein the statin is atorvastatin.

48. The composition of clause 47, wherein the amount of atorvastatin is between 10 and 80 mg.

49. The composition of any one of clauses 41-45, wherein the statin is simvastatin.

50. The composition of clause 49, wherein the amount of simvastatin is between 10 and 40 mg.

51. The composition of any one of clauses 1-45, wherein the statin is fluvastatin.

52. The composition of clause 51, wherein the amount of fluvastatin between 20 and 80 mg.

53. The composition of any one of clauses 41-45, wherein the statin is resuvastatin.

54. The composition of clause 53, wherein the amount of rosuvastatin is between 5 and 40 mg.

55. The composition of any one of clauses 41-45, wherein the statin is pitavastatin.

56. The composition of clause 55, wherein the amount of pitavastatin is between 1 and 4 mg.

57. A method for treating or reducing the risk of cardiovascular disease in a subject in need thereof, the method comprising administering: ETC-1002; Ezetimibe; a statin; a PCSK9 inhibitor antibody therapy; and one or more pharmaceutical excipients or carriers to the subject, wherein the administration treats or reduces the risk of cardiovascular disease in the subject.

58. The method of clause 57, wherein the antibody therapy is administered once every two (2) weeks.

59. The method of clause 57, wherein the antibody therapy is administered once every four (4) weeks.

60. A method for treating or reducing the risk of cardiovascular disease in a subject in need thereof, the method comprising administering: ETC-1002; Ezetimibe; a statin; an approved lipid-modifying therapy other than a statin and/or Ezetimibe; and one or more pharmaceutical excipients or carriers to the subject, wherein the administration treats or reduces the risk of cardiovascular disease in the subject.

61. A kit comprising the composition of any one of clauses 41-56 and instructions for use.

## Claims

**1.** ETC-1002, ezetimibe, and a statin for use in a method of treating or reducing the risk of cardiovascular disease in a subject in need thereof, said method comprising administering the ETC-1002, ezetimibe, and statin to the subject, wherein the administering treats or reduces the risk of cardiovascular disease in the subject, and wherein the statin is selected from the group consisting of 10-80 mg atorvastatin, 5-40 mg rosuvastatin, 10-20 simvastatin, 10-40 mg pravastatin, 20 or 40 mg lovastatin, 20-80 mg fluvastatin, or 1-4 mg pitavastatin.

**2.** A pharmaceutical composition comprising:

ETC-1002;
Ezetimibe;
a statin; and
one or more pharmaceutical excipients or carriers,

wherein the statin is selected from the group consisting of 10-80 mg atorvastatin, 5-40 mg rosuvastatin, 10-20 simvastatin, 10-40 mg pravastatin, 20 or 40 mg lovastatin, 20-80 mg fluvastatin, or 1-4 mg pitavastatin.

**3.** ETC-1002, ezetimibe, and a statin for use according to claim 1, or the pharmaceutical composition according to claim 2, wherein the amount of ETC-1002 is 180 mg or 120 mg;
or wherein the amount of ETC-1002 is between 40 mg to 90 mg.

**4.** ETC-1002, ezetimibe, and a statin for use according to claim 1 or 3, or the pharmaceutical composition according to claim 2 or 3, wherein the amount of ezetimibe is 10 mg.

**5.** ETC-1002, ezetimibe, and a statin for use according to any one of claims 1, 3, or 4, wherein:

(i) the level of low-density lipoprotein cholesterol (LDL-C) in the subject is below that of a matched control subject receiving the same dose of the statin and the ezetimibe but not receiving ETC-1002;
optionally wherein the level of LDL-C in the subject is reduced 64% relative to baseline LDL-C level in the subject;
(ii) the level of very low density lipoprotein (VLDL) in the subject is below that of a matched control subject receiving the same dose of the statin and the ezetimibe but not receiving ETC-1002;
(iii) the number of VLDL particles in the subject is below that of a matched control subject receiving the same

dose of the statin and the ezetimibe but not receiving ETC-1002;
(iv) the size of VLDL particles in the subject is below that of a matched control subject receiving the same dose of the statin and the ezetimibe but not receiving ETC-1002;
(v) the level of apolipoprotein A-1 (ApoA1) in the subject is greater than that of a matched control subject receiving the same dose of the statin and the ezetimibe but not receiving ETC-1002;
(vi) the ratio of apolipoprotein B (ApoB) to ApoA1 in the subject is:

(a) below;
(b) not significantly changed from; or
(c) increased from;

that of a matched control subject receiving the same dose of the statin and the ezetimibe but not receiving ETC-1002;
(vii) the level of ApoB in the subject is reduced 54% relative to baseline ApoB level in the subject;
(viii) the level of triglycerides (TG) in the subject is below that of a matched control subject receiving the same dose of the statin and the ezetimibe but not receiving ETC-1002;
optionally wherein the level of TG in the subject is reduced 27% relative to baseline TG level in the subject;
(ix) the level of total cholesterol (TC) in the subject is below that of a matched control subject receiving the same dose of the statin and the ezetimibe but not receiving ETC-1002;
optionally wherein the level of TC in the subject is reduced 47% relative to baseline TC level in the subject;
(x) the level of high-sensitivity C-reactive protein (hs-CRP) in the subject is below that or not changed from that of a matched control subject receiving the same dose of the statin and the ezetimibe but not receiving ETC-1002;
optionally wherein the level of hs-CRP in the subject is reduced 54% relative to baseline;
(xi) the level of non-HDL-C in the subject is below that of a matched control subject receiving the same dose of the statin and the ezetimibe but not receiving ETC-1002;
optionally wherein the level of non-HDL-C in the subject is reduced 60% relative to baseline non-HDL-C level in the subject; and/or
(xii) the method lowers LDL-C level 50% compared to the baseline LDL-C level in the subject after six (6) weeks of administration.

**6.** ETC-1002, ezetimibe, and a statin for use according to any one of claims 1 or 3-5, wherein the subject has:

(i) hypercholesterolemia;
(ii) familial hypercholesterolemia;
(iii) hyperlipidemia;
(iv) dyslipidemia or mixed dyslipidemia; and/or
(v) atherosclerotic cardiovascular disease.

**7.** ETC-1002, ezetimibe, and a statin for use according to any one of claims 1 or 3-6, wherein the subject is human.

**8.** ETC-1002, ezetimibe, and a statin for use according to any one of claims 1 or 3-7, or the pharmaceutical composition according to any one of claims 2-4, wherein the statin is atorvastatin, and the amount of atorvastatin is 10-80 mg.

**9.** ETC-1002, ezetimibe, and a statin for use according to any one of claims 1 or 3-7, or the pharmaceutical composition according to any one of claims 2-4, wherein the statin is rosuvastatin, and the amount of rosuvastatin is 5-40 mg.

**10.** ETC-1002, ezetimibe, and a statin for use according to any one of claims 1 or 3-7, or the pharmaceutical composition according to any one of claims 2-4, wherein the statin is simvastatin, and the amount of simvastatin is 10-20 mg.

**11.** ETC-1002, ezetimibe, and a statin for use according to any one of claims 1 or 3-7, or the pharmaceutical composition according to any one of claims 2-4, wherein the statin is pravastatin, and the amount of pravastatin is 10-40 mg.

**12.** ETC-1002, ezetimibe, and a statin for use according to any one of claims 1 or 3-7, or the pharmaceutical composition according to any one of claims 2-4, wherein the statin is fluvastatin, and the amount of fluvastatin is 20-80 mg.

**13.** ETC-1002, ezetimibe, and a statin for use according to any one of claims 1 or 3-7, or the pharmaceutical composition according to any one of claims 2-4, wherein the statin is pitavastatin, and the amount of pitavastatin is 1-4 mg.

**14.** ETC-1002, ezetimibe, and a statin for use according to any one of claims 1 or 3-7, or the pharmaceutical composition according to any one of claims 2-4, wherein the statin is lovastatin, and the amount of lovastatin is 20 or 40 mg.

**15.** ETC-1002, ezetimibe, and a statin for use according to any one of claims 1 or 3-14, or the pharmaceutical composition according to any one of claims 2-4 or 8-14, wherein the amount of ETC-1002 is 180 mg, and the amount of Ezetimibe is 10 mg.

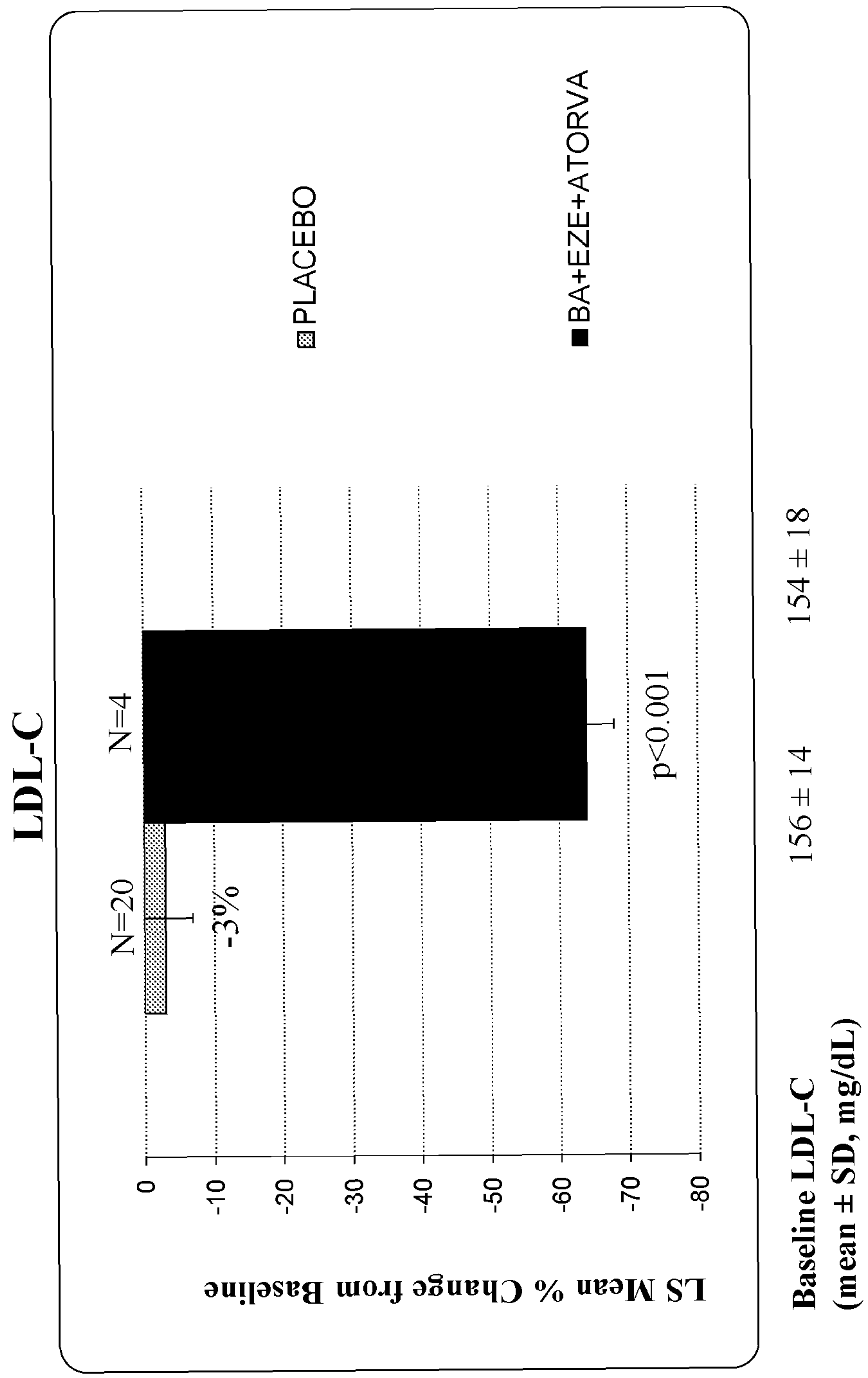
LDL-C
LS Mean % Change from Baseline
0
-10
-20
-30
-40
-50
-60
-70
-80
N=20
-3%
N=4
p<0.001
PLACEBO
BA+EZE+ATORVA
Baseline LDL-C
(mean ± SD, mg/dL)
156 ± 14
154 ± 18

FIG. 1

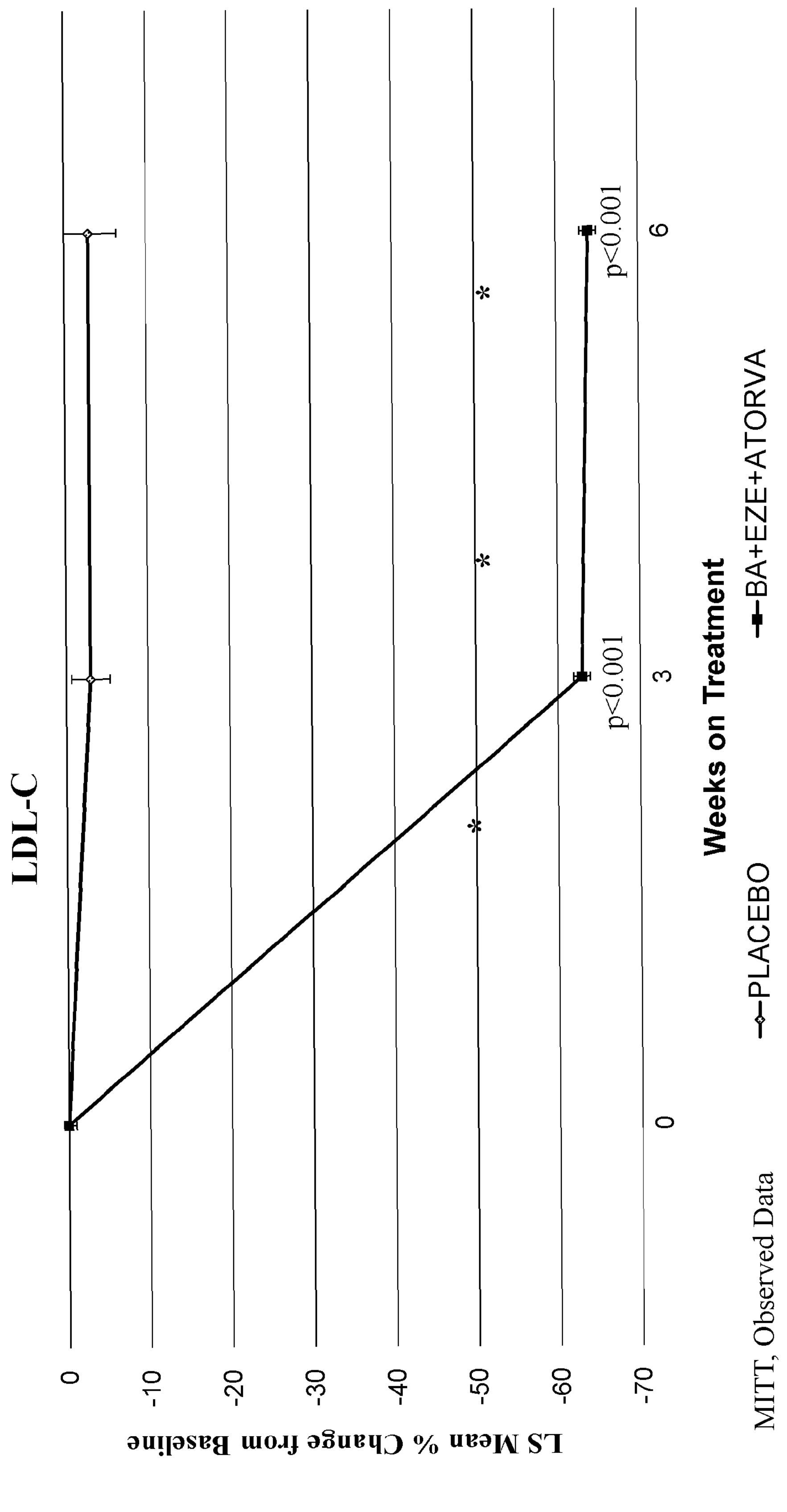
LDL-C
LS Mean % Change from Baseline
0
-10
-20
-30
-40
-50
-60
-70
0
3
6
Weeks on Treatment
p<0.001
p<0.001
PLACEBO
BA+EZE+ATORVA
MITT, Observed Data

FIG. 2

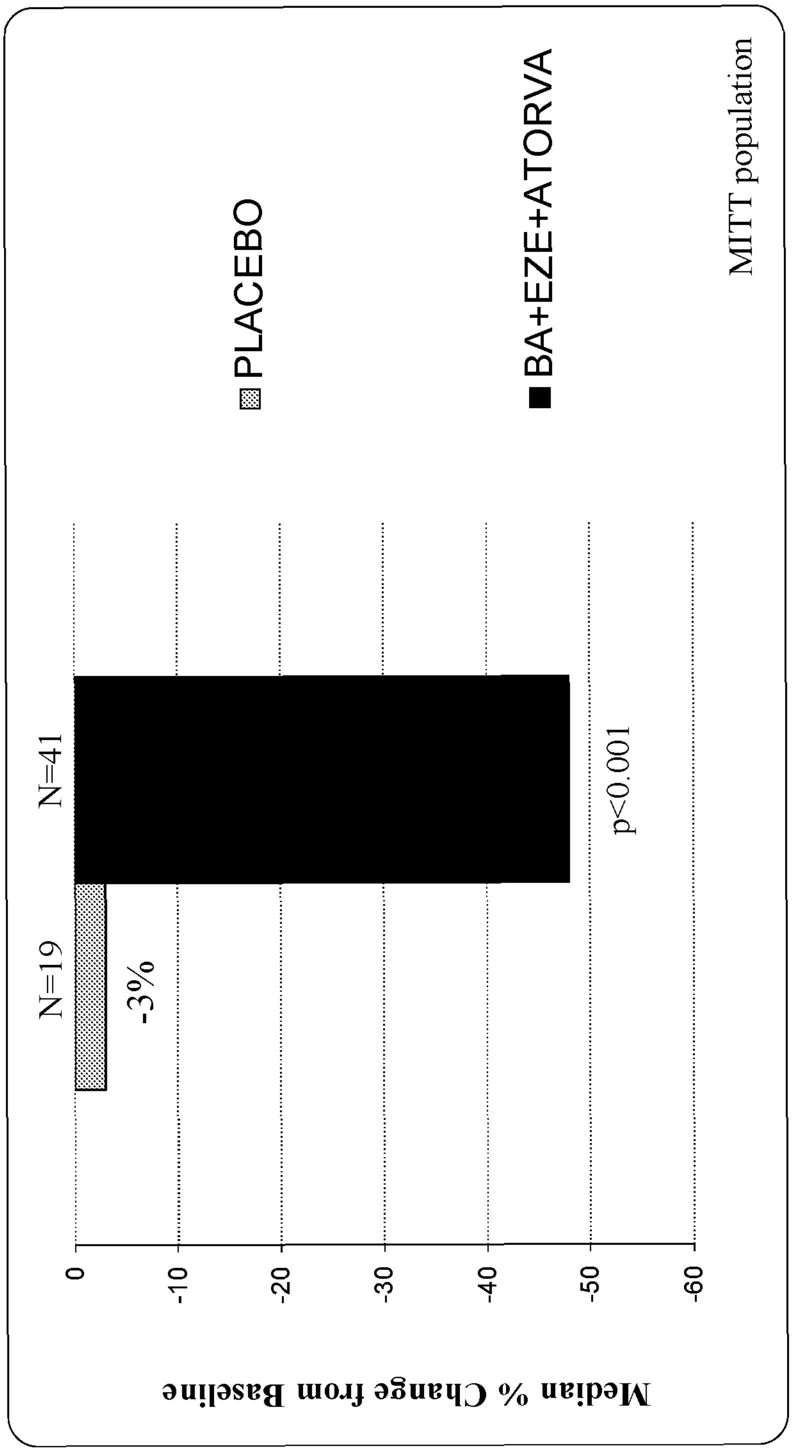
hsCRP
Median % Change from Baseline
0
-10
-20
-30
-40
-50
-60
N=19
-3%
N=41
p<0.001
Baseline hsCRP (median, mg/L)
1.64
1.94
PLACEBO
BA+EZE+ATORVA
MITT population

FIG. 3

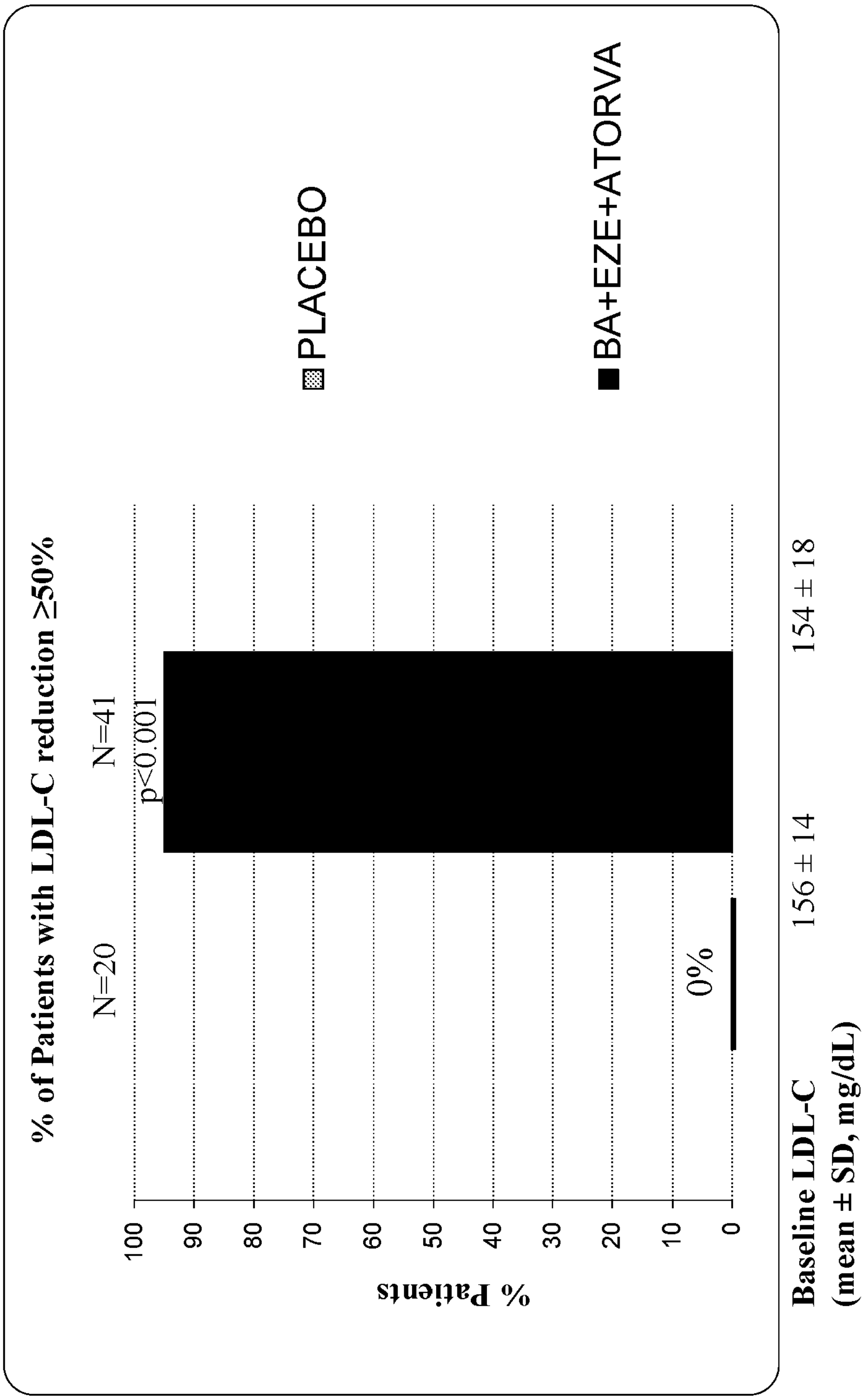
% of Patients with LDL-C reduction ≥50%
N=20
N=41
p<0.001
0%
% Patients
100
90
80
70
60
50
40
30
20
10
0
Baseline LDL-C
(mean ± SD, mg/dL)
156 ± 14
154 ± 18
PLACEBO
BA+EZE+ATORVA

FIG. 4

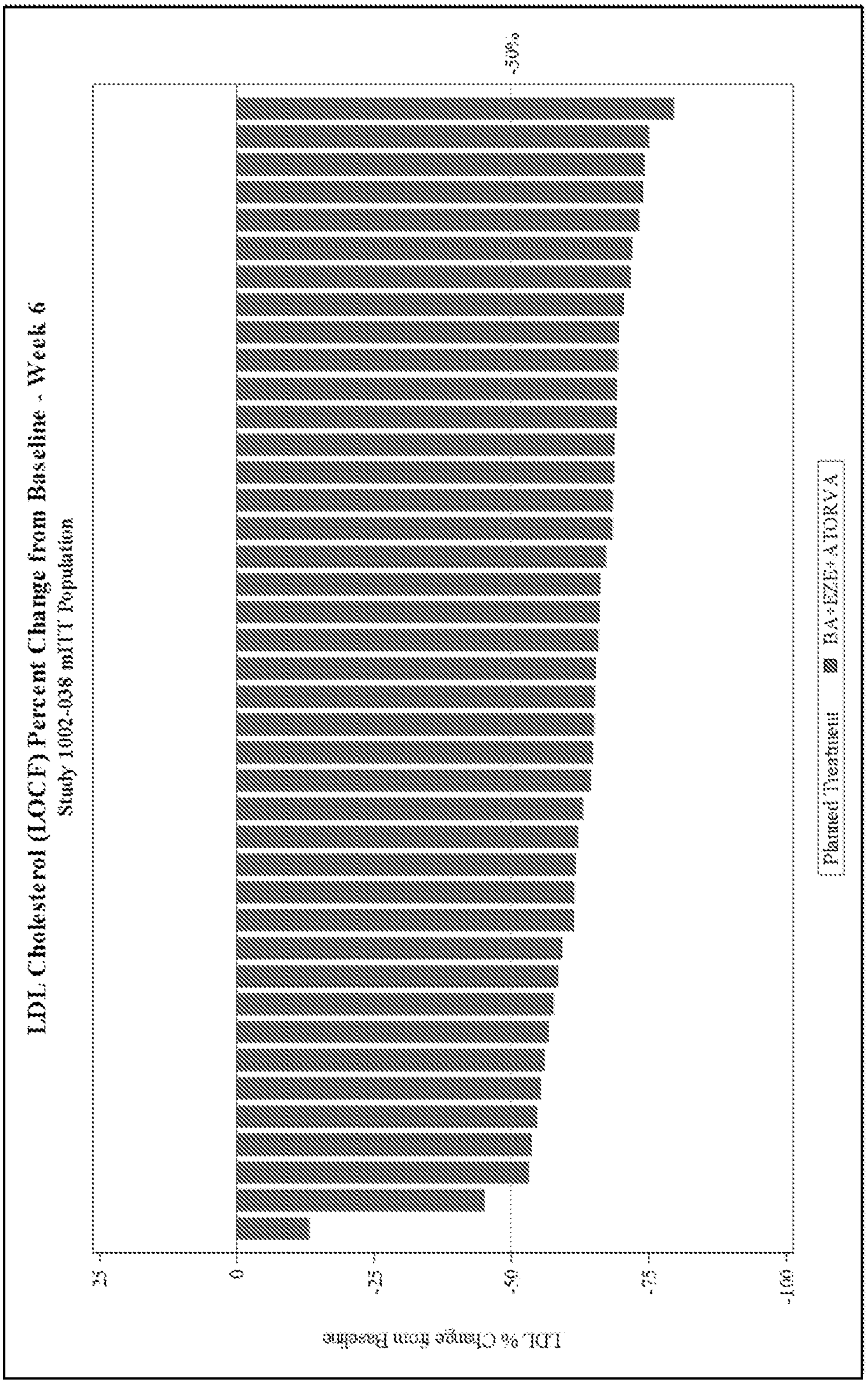
LDL Cholesterol (LOCF) Percent Change from Baseline - Week 6
Study 1002-038 mITT Population
-50%
LDL % Change from Baseline
25
0
-25
-50
-75
-100
Planned Treatment
BA+EZE+ATORVA

**FIG. 5**

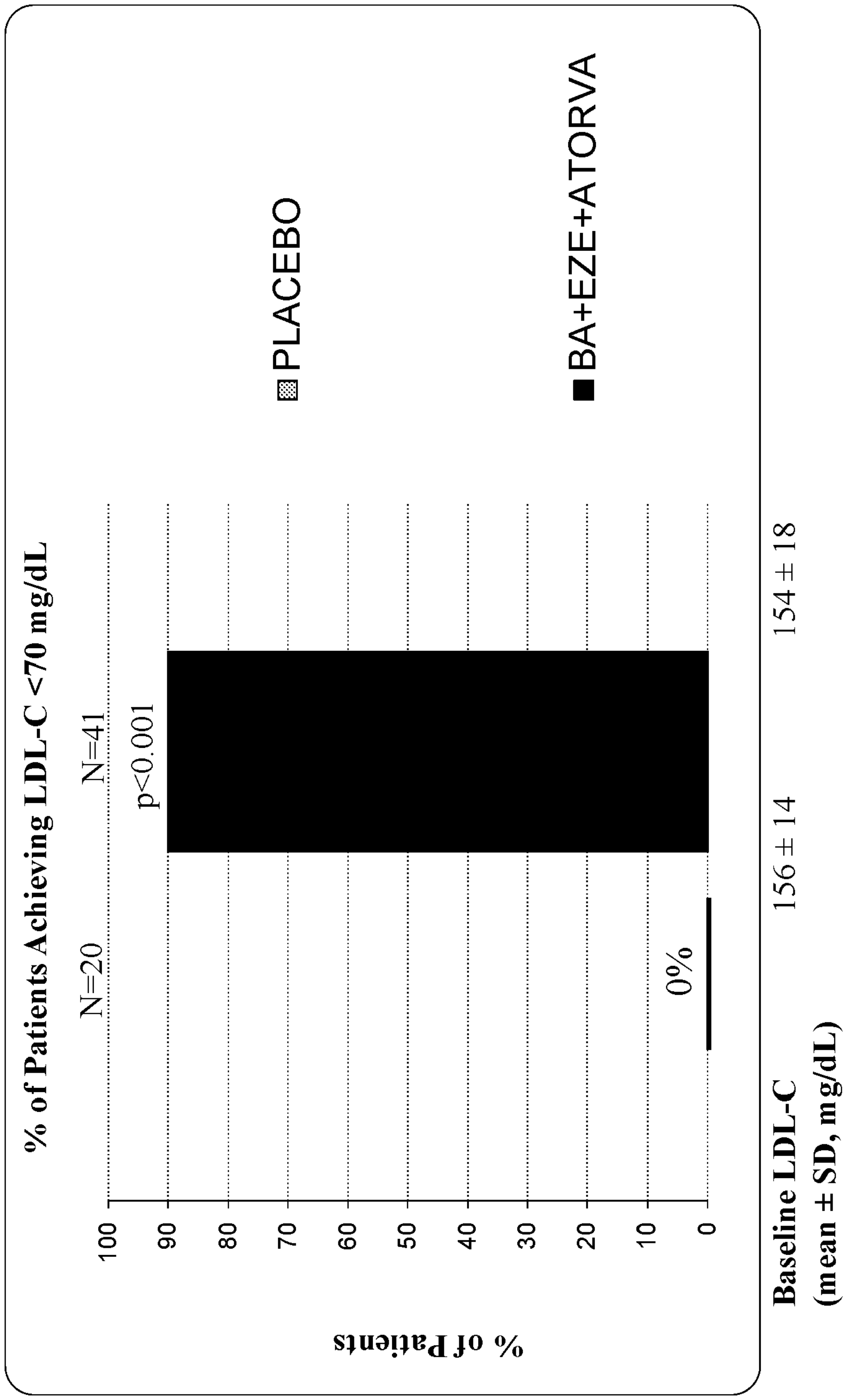

% of Patients Achieving LDL-C <70 mg/dL
% of Patients
100
90
80
70
60
50
40
30
20
10
0
N=20
N=41
p<0.001
0%
PLACEBO
BA+EZE+ATORVA
Baseline LDL-C
(mean ± SD, mg/dL)
156 ± 14
154 ± 18


FIG. 6

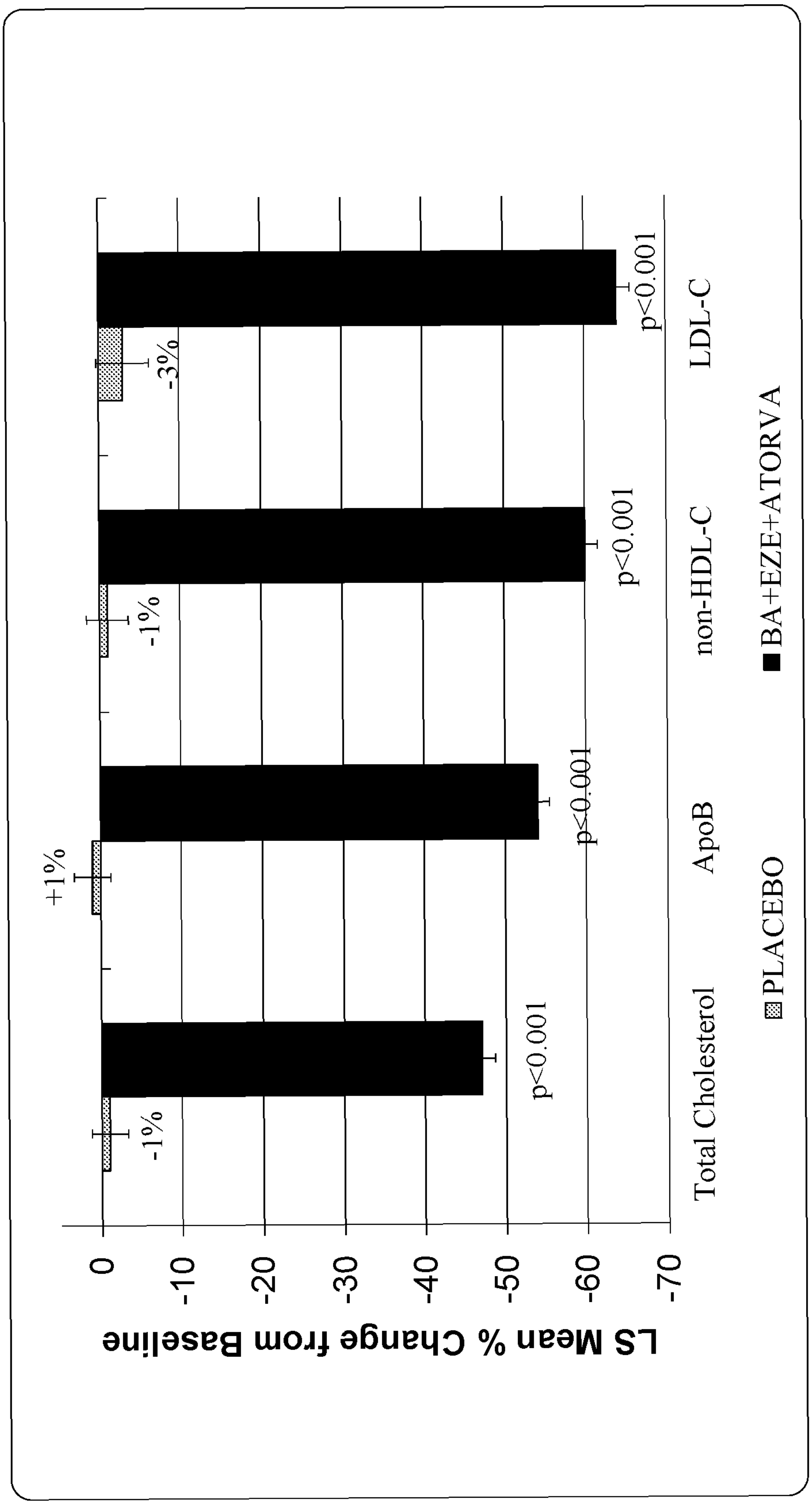
LS Mean % Change from Baseline
0
-10
-20
-30
-40
-50
-60
-70
Total Cholesterol
ApoB
non-HDL-C
LDL-C
-1%
p<0.001
+1%
p<0.001
-1%
p<0.001
-3%
p<0.001
PLACEBO
BA+EZE+ATORVA

**FIG. 7**

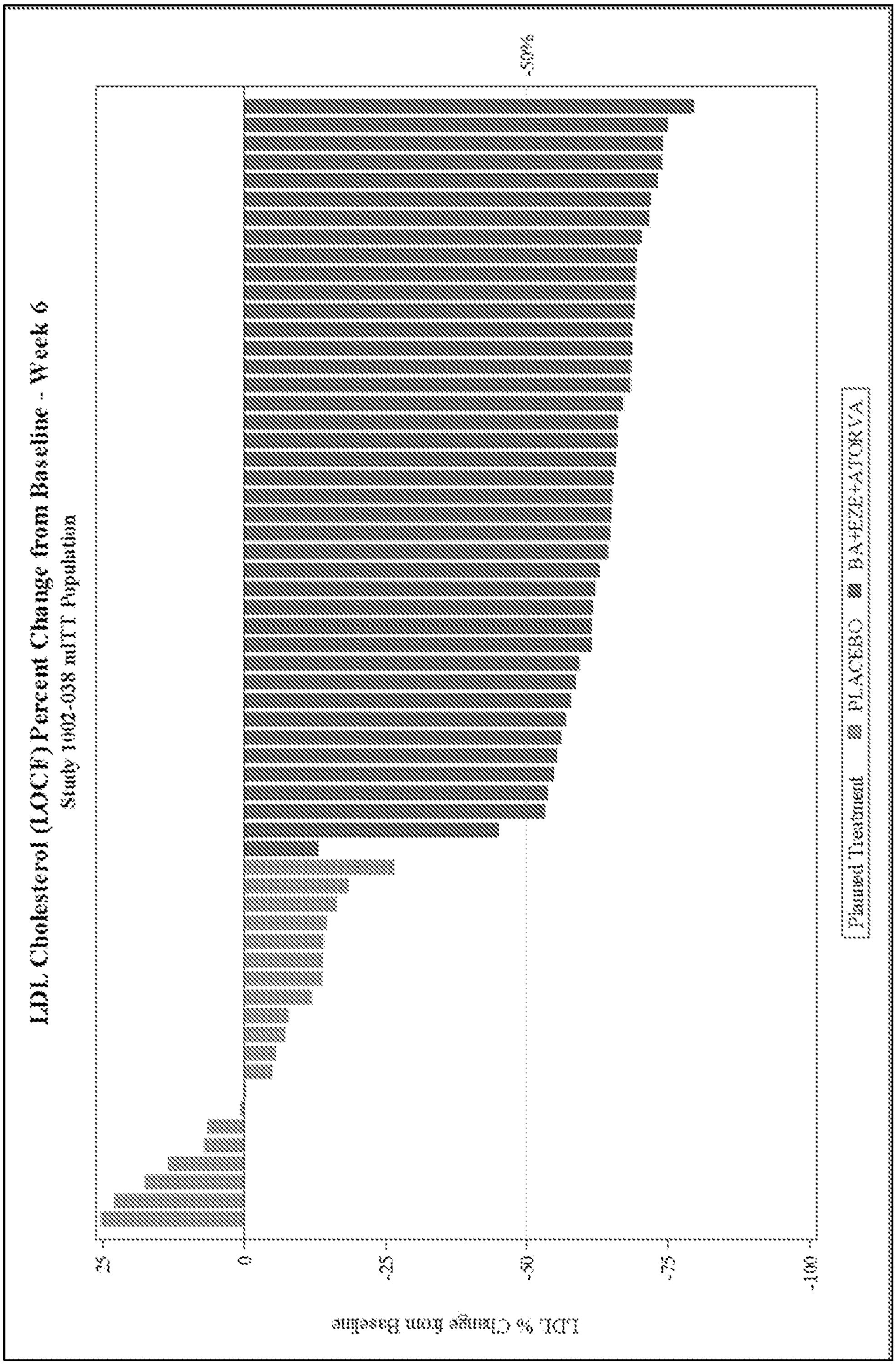
LDL Cholesterol (LOCF) Percent Change from Baseline - Week 6
Study 1002-038 mITT Population
-50%
25
0
-25
-50
-75
-100
LDL % Change from Baseline
Planned Treatment
PLACEBO
BA+EZE+ATORVA

**FIG. 8**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 7335799 B
- US 20050043278 A1
- US 5631365 A
- WO 2005047276 A2


### Non-patent literature cited in the description


- Fieser and Fieser's Reagents for Organic Synthesis. John Wiley, and Sons, 1991, vol. 1-15
- Rodd's Chemistry of Carbon Compounds. Elsevier Science Publishers, 1989, vol. 1-5
- Organic Reactions. John Wiley, and Sons, 1991, vol. 1-40
- March's Advanced Organic Chemistry. John Wiley, and Sons, 2001
- Larock's Comprehensive Organic Transformations. VCH Publishers Inc, 1989
- **T. W. GREENE ; P.G.M. WUTS.** Protecting Groups in Organic Synthesis. Wiley, 1999
- Remington's Pharmaceutical Sciences. 1980
- **T.E. CREIGHTON.** Proteins: Structures and Molecular Properties. W.H. Freeman and Company, 1993
- **A.L. LEHNINGER.** Biochemistry. Worth Publishers, Inc,
- **SAMBROOK et al.** Molecular Cloning: A Laboratory Manual. 1989
- Methods In Enzymology. Academic Press, Inc,
- Remington's Pharmaceutical Sciences. Mack Publishing Company, 1990
- **CAREY ; SUNDBERG.** Advanced Organic Chemistry 3rd Ed. Plenum Press, 1992, vol. A,B
- **BAIGENT C ; BLACKWELL L ; EMBERSON J et al.** Cholesterol Treatment Trialists' (CTT) Collaboration. Efficacy and safety of more intensive lowering of LDL cholesterol: a meta-analysis of data from 170 000 participants in 26 randomized trials. *Lancet,* 2010, vol. 376, 1670-81
- **COHEN JD ; BRINTON EA ; ITO MK ; JACOBSON TA.** Understanding statin use in America and gaps in patient education (USAGE): an internet-based survey of 10,138 current and former statin users. *Clinical Lipidol.,* 2012, vol. 6, 208-15
- **EGAN A ; COLMAN E.** Weighing the benefits of high-dose simvastatin against the risk of myopathy. *N Engl J Med.,* 2011, vol. 365, 285-7
- *FDA announces safety changes in labeling for some cholesterol-lowering drugs,* 2012, http://www.fda.gov/NewsEvents/Newsroom/PressAnnouncements/ucm293623.htm
- **GOLDBERG AC.** Clinical trial experience with extended-release niacin (Niaspan): dose-escalation study. *Am J Cardiol,* 1998, vol. 82, 35U-8U
- **GRUNDY SM ; CLEEMAN JI ; MERZ CN ; BREWER HB ; CLARK LT ; HUNNINGHAKE DB et al.** Implications of recent clinical trials for the National Cholesterol Education Program Adult Treatment Panel III Guidelines. *Circulation,* 2004, vol. 110, 227-39
- **INSULL W ; TOTH P ; MULLICAN W ; HUNNINGHAKE D ; BURKE S ; DONOVAN J et al.** Effectiveness of colesevelam hydrochloride in decreasing LDL cholesterol in patients with primary hypercholesterolemia: a 24-week randomized controlled trial. *Mayo Clin Proc.,* 2001, vol. 76, 971-82
- **KATHIRESAN S ; MELANDER O ; ANEVSKI D ; GUIDUCCI C ; BURTT NP ; ROOS C et al.** Polymorphisms associated with cholesterol and risk of cardiovascular events. *New Engl J Med.,* 2008, vol. 358 (12), 1240-9
- **KNOPP RH ; BROWN WV ; DUJOVNE CA ; FARQUHAR JW ; FELDMAN EB ; GOLDBERG AC et al.** Effects of fenofibrate on plasma lipoproteins in hypercholesterolemia and combined hyperlipidemia. *Am J Med,* 1987, vol. 83, 50-9
- **KNOPP RH ; DUJOVNE CA ; LEBEAUTA ; LIPKA LJ ; SURESH R ; VELTRI EP et al.** Evaluation of the efficacy, safety and tolerability of ezetimibe in primary hypercholesterolemia: a pooled analysis from two controlled phase III clinical studies. *Int J Clin Pract,* 2003, vol. 57, 363-8
- **MARTIN SS ; GOSCH K ; KULKARNI KR ; SPERTUS JA ; MATHEWS R ; HO PM et al.** Modifiable factors associated with failure to attain low-density lipoprotein cholesterol goal at 6 months after acute myocardial infarction. *Am Heart J.,* 2013, vol. 165, 26-33.e3

- **MOZFRARIAN D ; BENJAMIN EJ ; GO AS ; ARNETT DK ; BLAHA MJ ; CUSHMAN M et al.** Heart Disease and Stroke Statistics-2015 Update. American Heart Association, 2015, vol. 131, e29-322
- **NICHOLS M ; TOWNSEND N ; SCARBOROUGH P ; RAYNER M.** Cardiovascular disease in Europe 2014: epidemiological update. *Eur Heart J.,* 2014, vol. 35, 2950-9
- **ROBINSON JG ; SMITH B ; MAHESHWARI N ; SCHROTT H.** Pleiotropic effects of statins: benefit beyond cholesterol reduction: A meta-regression analysis. *J Am Coll Cardiol.,* 24 October 2005, vol. 46 (10), 1855-62
- **SHARRETT AR ; BALLANTYNE CM ; COADY SA ; HEISS G ; SORLIE PD ; CATELLIER D et al.** Atherosclerosis Risk in Communities Study Group. Coronary heart disease prediction from lipoprotein cholesterol levels, triglycerides, lipoprotein(a), apolipoproteins A-I and B, and HDL density subfractions. The Atherosclerosis Risk in Communities (ARIC) Study. *Circulation,* 2001, vol. 104, 1108-13
- **SILVERMAN MG ; FERENCE BA ; IM K ; WIVIOTT SD ; GIUGLIANO RP ; GRUNDY SM et al.** Association between lowering LDL-C and cardiovascular risk reduction among different therapeutic interventions: a systematic review and meta-analysis. *JAMA,* 2016, vol. 316 (12), 1289-97
- **STAMLER J ; WENTWORTH D ; NEATON J.** Is relationship between serum cholesterol and risk for premature death from coronary heart disease continuous and graded?. *JAMA,* 1986, vol. 256 (20), 823-8
- **STONE NJ ; ROBINSON J ; LICHTENSTEIN AH et al.** Practice Guidelines Cardiovascular Risk in Adults. *2013 ACC/AHA Guideline on the Treatment of Blood Cholesterol to Reduce Atherosclerotic Cardiovascular Risk in Adults. Circ*
- **TOWNSEND N ; NICHOLS M ; SCARBOROUGH P ; RAYNER M.** Cardiovascular disease in Europe - epidemiological update 2015. *Eur Heart J,* 2015, vol. 36 (40), 2696-705
- **THOMPSON PD ; CLARKSON P ; KARAS RH.** Statin-associated myopathy. *JAMA,* 2003, vol. 289, 1681-90
- **VIRANI SS ; WOODARD LD ; CHITWOOD SS ; LANDRUM CR ; URECH TH ; WANG D et al.** Frequency and correlates of treatment intensification for elevated cholesterol levels in patients with cardiovascular disease. *Am Heart J.,* 2011, vol. 162, 725-732.e1
- **WATERS DD.** What the statin trials have taught us. *Am J Cardiol.,* 2006, vol. 98, 129-34
- *Fact Sheet,* January 2015, (317